# EUROPEAN PATENT APPLICATION

(11) **EP 1 384 487 A1**
(43) Date of publication of application: **28.01.2004**
(21) Application number: 02713258.8
(22) Date of filing: 29.03.2002
(51) Int. Cl.: A61K 39/395, A61K 45/00, A61K 38/20, A61K 38/21, A61K 31/4164, A61P 35/00

(54) **DRUGS CONTAINING GENETICALLY MODIFIED ANTIBODY AGAINST GANGLIOSIDE GD3**

(30) Priority: 29.03.2001 JP 2001097483
(71) Applicant: KYOWA HAKKO KOGYO CO., LTD., Chiyoda-ku, Tokyo 100-8185 (JP)
(72) Inventor: SHITARA, Kenya, c/o Kyoto Hakko Kogyo Co. Ltd., Machida-shi, Tokyo 194-8533 (JP); NIWA, Rinpei, Kyowa Hakko Kogyo Co. Ltd., Machida-shi, Tokyo 194-8533 (JP); KANAZAWA, Junji, c/o Kyowa Hakko Kogyo Co. Ltd., Sunto-gun, Shizuoka 411-8731 (JP); ASADA, Masao, c/o Kyowa Hakko Kogyo Co. Ltd., Sunto-gun, SHizuoka 411-8731 (JP)
(74) Representative: VOSSIUS & PARTNER
(86) International application number: PCT/JP2002/003170
(87) International publication number: WO 2002/078739

(57) **Abstract**

In order to obtain high therapeutic effects in treating malignant tumors, particularly melanoma, a new therapeutic method having less side effects, or a new therapeutic method which can provide further high therapeutic effects at conventional doses of agents has been desired.

An object of the present invention is to provide a medicament which can provide higher therapeutic effects than any one of a gene recombinant antibody against ganglioside GD3 or the antibody fragment thereof alone, and a substance which activates an immunocompetent cell and a substance having an antitumor activity alone, by combining a gene recombinant antibody against ganglioside GD3 or the antibody fragment thereof with at least one of a substance which activates an immunocompetent cell and a substance having an antitumor activity. Also, the medicament is expected to relieve side effects which were problems in the case of administration of individual single agents.

SEQUENCE LISTING

<110> KYOWA HAKKO KOGYO CO., LTD.

<120> Agents for treatment of cancer using recombinant anti-GD3 antibodies
and the antibody fragments

<130> 11374W01

<140>
<141>

<150> JP2001-097483
<151> 2001-03-29

<160> 50

<170> PatentIn Ver. 2.0

<210> 1
<211> 138
<212> PRT
<213> Mus musculus
<400> 1
Met Glu Phe Gly Leu Ser Trp Leu Phe Leu Val Leu Val Phe Lys Gly
-19 -15 -10 -5
Val Gln Cys Glu Val Thr Leu Val Glu Ser Gly Gly Asp Phe Val Lys
-1 1 5 10
Pro Gly Gly Ser Leu Lys Val Ser Cys Ala Ala Ser Gly Phe Ala Phe
15 20 25
Ser His Tyr Ala Met Ser Trp Val Arg Gln Thr Pro Ala Lys Arg Leu
30 35 40 45
Glu Trp Val Ala Tyr Ile Ser Ser Gly Gly Ser Gly Thr Tyr Tyr Ser
50 55 60
Asp Ser Val Lys Gly Arg Phe Thr Ile Ser Arg Asp Asn Ala Lys Asn
65 70 75
Thr Leu Tyr Leu Gln Met Arg Ser Leu Arg Ser Glu Asp Ser Ala Met
80 85 90
Tyr Phe Cys Thr Arg Val Lys Leu Gly Thr Tyr Tyr Phe Asp Ser Trp
95 100 105
Gly Gln Gly Thr Thr Leu Thr Val Ser Ser
110 115

<210> 2
<211> 128
<212> PRT
<213> Mus musculus
<400> 2
Met Met Ser Ser Ala Gln Phe Leu Gly Leu Leu Leu Leu Cys Phe Gln
-20                 -15                 -10                 -5
Gly Thr Arg Cys Asp Ile Gln Met Thr Gln Thr Ala Ser Ser Leu Pro
            -1  1               5                   10
Ala Ser Leu Gly Asp Arg Val Thr Ile Ser Cys Ser Ala Ser Gln Asp
        15                  20                  25
Ile Ser Asn Tyr Leu Asn Trp Tyr Gln Gln Lys Pro Asp Gly Thr Val
    30                  35                  40
Lys Leu Leu Ile Phe Tyr Ser Ser Asn Leu His Ser Gly Val Pro Ser
45                  50                  55                  60
Arg Phe Ser Gly Gly Gly Ser Gly Thr Asp Tyr Ser Leu Thr Ile Ser
                65                  70                  75
Asn Leu Glu Pro Glu Asp Ile Ala Thr Tyr Phe Cys His Gln Tyr Ser
            80                  85                  90
Lys Leu Pro Trp Thr Phe Gly Gly Gly Thr Lys Leu Glu Ile Lys Arg
        95                  100                 105
<210> 3
<211> 5
<212> PRT
<213> Mus musculus
<400> 3
His Tyr Ala Met Ser
1               5
<210> 4
<211> 17
<212> PRT
<213> Mus musculus
<400> 4
Tyr Ile Ser Ser Gly Gly Ser Gly Thr Tyr Tyr Ser Asp Ser Val Lys Gly
1               5                   10                  15
<210> 5
<211> 10
<212> PRT
<213> Mus musculus
<400> 5
Val Lys Leu Gly Thr Tyr Tyr Phe Asp Ser
1               5                   10
<210> 6

<211> 11
<212> PRT
<213> Mus musculus
<400> 6
Ser Ala Ser Gln Asp Ile Ser Asn Tyr Leu Asn
1               5                   10
<210> 7
<211> 7
<212> PRT
<213> Mus musculus
<400> 7
Tyr Ser Ser Asn Leu His Ser
1               5
<210> 8
<211> 9
<212> PRT
<213> Mus musculus
<400> 8
His Gln Tyr Ser Lys Leu Pro Trp Thr
1               5
<210> 9
<211> 119
<212> PRT
<213> Artificial Sequence
<400> 9
Glu Val Gln Leu Val Glu Ser Gly Gly Asp Phe Val Gln Pro Gly Gly
1               5                   10                  15
Ser Leu Arg Val Ser Cys Ala Ala Ser Gly Phe Ala Phe Ser His Tyr
            20                  25                  30
Ala Met Ser Trp Val Arg Gln Ala Pro Gly Lys Gly Leu Glu Trp Val
        35                  40                  45
Ala Tyr Ile Ser Ser Gly Gly Ser Gly Thr Tyr Tyr Ser Asp Ser Val
    50                  55                  60
Lys Gly Arg Phe Thr Ile Ser Arg Asp Asn Ser Lys Asn Thr Leu Tyr
65                  70                  75                  80
Leu Gln Met Arg Ser Leu Arg Ala Glu Asp Ser Ala Val Tyr Phe Cys
                85                  90                  95
Thr Arg Val Lys Leu Gly Thr Tyr Tyr Phe Asp Ser Trp Gly Gln Gly
            100                 105                 110
Thr Leu Leu Thr Val Ser Ser
        115
<210> 10
<211> 108

<212> PRT
<213> Artificial Sequence
<400> 10
Asp Ile Gln Met Thr Gln Ser Pro Ser Ser Leu Ser Ala Ser Val Gly
1               5                   10                  15
Asp Arg Val Thr Ile Thr Cys Ser Ala Ser Gln Asp Ile Ser Asn Tyr
            20                  25                  30
Leu Asn Trp Tyr Gln Gln Lys Pro Asp Lys Ala Pro Lys Leu Leu Ile
        35                  40                  45
Phe Tyr Ser Ser Asn Leu His Ser Gly Val Pro Ser Arg Phe Ser Gly
    50                  55                  60
Gly Gly Ser Gly Thr Asp Tyr Thr Leu Thr Ile Ser Ser Leu Gln Pro
65                  70                  75                  80
Glu Asp Ile Ala Thr Tyr Phe Cys His Gln Tyr Ser Lys Leu Pro Trp
                85                  90                  95
Thr Phe Gly Gln Gly Thr Lys Val Glu Ile Lys Arg
            100                 105
<210> 11
<211> 95
<212> DNA
<213> Artificial Sequence
<220>
<223> Description of Artificial Sequence:synthetic DNA
<400> 11
caggaaacag ctatgacgcg gccgccacca tggagtttgg gctcagctgg ctttttcttg 60
tccttgtttt caaaggtgtt cagtgtgagg tgcag 95
<210> 12
<211> 97
<212> DNA
<213> Artificial Sequence
<220>
<223> Description of Artificial Sequence:synthetic DNA
<400> 12
aaagcgaatc cagaggctgc acaggagact ctcagagacc cccccggctg tacaaagtct 60
cccccagact ccaccagctg cacctcacac tgaacac 97
<210> 13
<211> 98
<212> DNA
<213> Artificial Sequence
<220>
<223> Description of Artificial Sequence:synthetic DNA
<400> 13
gcagcctctg gattcgcttt cagtcattat gccatgtctt gggtccgcca ggctccaggg 60

aaggggctgg agtgggtggc ttatattagt agtggtgg 98
<210> 14
<211> 97
<212> DNA
<213> Artificial Sequence
<220>
<223> Description of Artificial Sequence:synthetic DNA
<400> 14
gatacagcgt gttcttggag ttatctctgg agatggtgaa tctgcccttt acactgtctg 60
aatagtaggt gccactacca ccactactaa tataagc 97
<210> 15
<211> 98
<212> DNA
<213> Artificial Sequence
<220>
<223> Description of Artificial Sequence:synthetic DNA
<400> 15
ctccaagaac acgctgtatc tgcaaatgcg cagcctgaga gctgaggact cggctgtgta 60
tttctgtaca agagttaaac tgggaaccta ctactttg 98
<210> 16
<211> 92
<212> DNA
<213> Artificial Sequence
<220>
<223> Description of Artificial Sequence:synthetic DNA
<400> 16
gttttcccag tcacgacggg cccttggtgg aggctgagga gacggtgagc agggttccct 60
ggccccagga gtcaaagtag taggttccca gt 92
<210> 17
<211> 94
<212> DNA
<213> Artificial Sequence
<220>
<223> Description of Artificial Sequence:synthetic DNA
<400> 17
caggaaacag ctatgacgaa ttccaccatg atgtcctctg ctcagttcct tggtctcctg 60
ttgctctgtt ttcaaggtac cagatgtgac atcc 94
<210> 18
<211> 87
<212> DNA
<213> Artificial Sequence
<220>
<223> Description of Artificial Sequence:synthetic DNA

<400> 18
gcactacaag tgatggtgac tctgtctcct acagatgcag acagggagga tggagactgg 60
gtcatctgga tgtcacatct ggtacct 87
<210> 19
<211> 89
<212> DNA
<213> Artificial Sequence
<220>
<223> Description of Artificial Sequence:synthetic DNA
<400> 19
gtcaccatca cttgtagtgc aagtcaggac attagtaatt atttaaactg gtatcagcag 60
aaaccaggga aagcccctaa gctcctgat 89
<210> 20
<211> 89
<212> DNA
<213> Artificial Sequence
<220>
<223> Description of Artificial Sequence:synthetic DNA
<400> 20
taatctgtcc cagatccacc gccgctgaac cttgatggga cccccgagtg taaatttgat 60
gagtaaaaga tcaggagctt aggggcttt 89
<210> 21
<211> 92
<212> DNA
<213> Artificial Sequence
<220>
<223> Description of Artificial Sequence:synthetic DNA
<400> 21
ggtggatctg ggacagatta tactctcacc atcagcagcc tgcagcctga agattttgca 60
acttattact gtcatcagta tagtaagctt cc 92
<210> 22
<211> 80
<212> DNA
<213> Artificial Sequence
<220>
<223> Description of Artificial Sequence:synthetic DNA
<400> 22
gttttcccag tcacgaccgt acgtttaatc tctaccttgg tcccctggcc gaacgtccac 60
ggaagcttac tatactgatg 80
<210> 23
<211> 45
<212> DNA
<213> Artificial Sequence

<220>
<223> Description of Artificial Sequence:synthetic DNA
<400> 23
agcttccatg gacgttcggt ggaggcacca agctggaaat caaac 45
<210> 24
<211> 45
<212> DNA
<213> Artificial Sequence
<220>
<223> Description of Artificial Sequence:synthetic DNA
<400> 24
gtacgtttga tttccagctt ggtgcctcca ccgaacgtcc atgga 45
<210> 25
<211> 29
<212> DNA
<213> Artificial Sequence
<220>
<223> Description of Artificial Sequence:synthetic DNA
<400> 25
gatgcaggca gggaggatgc agtctgggt 29
<210> 26
<211> 29
<212> DNA
<213> Artificial Sequence
<220>
<223> Description of Artificial Sequence:synthetic DNA
<400> 26
acccagactg catcctccct gcctgcatc 29
<210> 27
<211> 384
<212> DNA
<213> Artificial Sequence
<220>
<223> Description of Artificial Sequence:synthetic DNA
<220>
<221> CDS
<222> (1)..(384)
<400> 27
atg atg tcc tct gct cag ttc ctt ggt ctc ctg ttg ctc tgt ttt caa 48
Met Met Ser Ser Ala Gln Phe Leu Gly Leu Leu Leu Leu Cys Phe Gln
-20 -15 -10 -5
ggt acc aga tgt gac atc cag atg acc cag act gca tcc tcc ctg cct 96
Gly Thr Arg Cys Asp Ile Gln Met Thr Gln Thr Ala Ser Ser Leu Pro

-1 1 5 10
gca tct gta gga gac aga gtc acc atc act tgt agt gca agt cag gac 144
Ala Ser Val Gly Asp Arg Val Thr Ile Thr Cys Ser Ala Ser Gln Asp
15 20 25
att agt aat tat tta aac tgg tat cag cag aaa cca ggg aaa gcc cct 192
Ile Ser Asn Tyr Leu Asn Trp Tyr Gln Gln Lys Pro Gly Lys Ala Pro
30 35 40
aag ctc ctg atc ttt tac tca tca aat tta cac tcg ggg gtc cca tca 240
Lys Leu Leu Ile Phe Tyr Ser Ser Asn Leu His Ser Gly Val Pro Ser
45 50 55 60
agg ttc agc ggc ggt gga tct ggg aca gat tat act ctc acc atc agc 288
Arg Phe Ser Gly Gly Gly Ser Gly Thr Asp Tyr Thr Leu Thr Ile Ser
65 70 75
agc ctg cag cct gaa gat ttt gca act tat tac tgt cat cag tat agt 336
Ser Leu Gln Pro Glu Asp Phe Ala Thr Tyr Tyr Cys His Gln Tyr Ser
80 85 90
aag ctt ccg tgg acg ttc ggc cag ggg acc aag gta gag att aaa cgt 384
Lys Leu Pro Trp Thr Phe Gly Gln Gly Thr Lys Val Glu Ile Lys Arg
95 100 105
<210> 28
<211> 26
<212> DNA
<213> Artificial Sequence
<220>
<223> Description of Artificial Sequence:synthetic DNA
<400> 28
actgatgaca gaaataagtt gcaaaa 26
<210> 29
<211> 26
<212> DNA
<213> Artificial Sequence
<220>
<223> Description of Artificial Sequence:synthetic DNA
<400> 29
ttttgcaact tatttctgtc atcagt 26
<210> 30
<211> 384
<212> DNA
<213> Artificial Sequence
<220>
<223> Description of Artificial Sequence:synthetic DNA
<220>
<221> CDS

<222> (1)..(384)
<400> 30
atg atg tcc tct gct cag ttc ctt ggt ctc ctg ttg ctc tgt ttt caa 48
Met Met Ser Ser Ala Gln Phe Leu Gly Leu Leu Leu Leu Cys Phe Gln
-20 -15 -10 -5
ggt acc aga tgt gac atc cag atg acc cag tct cca tcc tcc ctg tct 96
Gly Thr Arg Cys Asp Ile Gln Met Thr Gln Ser Pro Ser Ser Leu Ser
-1 1 5 10
gca tct gta gga gac aga gtc acc atc act tgt agt gca agt cag gac 144
Ala Ser Val Gly Asp Arg Val Thr Ile Thr Cys Ser Ala Ser Gln Asp
15 20 25
att agt aat tat tta aac tgg tat cag cag aaa cca ggg aaa gcc cct 192
Ile Ser Asn Tyr Leu Asn Trp Tyr Gln Gln Lys Pro Gly Lys Ala Pro
30 35 40
aag ctc ctg atc ttt tac tca tca aat tta cac tcg ggg gtc cca tca 240
Lys Leu Leu Ile Phe Tyr Ser Ser Asn Leu His Ser Gly Val Pro Ser
45 50 55 60
agg ttc agc ggc ggt gga tct ggg aca gat tat act ctc acc atc agc 288
Arg Phe Ser Gly Gly Gly Ser Gly Thr Asp Tyr Thr Leu Thr Ile Ser
65 70 75
agc ctg cag cct gaa gat ttt gca act tat ttc tgt cat cag tat agt 336
Ser Leu Gln Pro Glu Asp Phe Ala Thr Tyr Phe Cys His Gln Tyr Ser
80 85 90
aag ctt ccg tgg acg ttc ggc cag ggg acc aag gta gag att aaa cgt 384
Lys Leu Pro Trp Thr Phe Gly Gln Gly Thr Lys Val Glu Ile Lys Arg
95 100 105
<210> 31
<211> 28
<212> DNA
<213> Artificial Sequence
<220>
<223> Description of Artificial Sequence:synthetic DNA
<400> 31
ggagcttaac ggctttgtct ggtttctg 28
<210> 32
<211> 28
<212> DNA
<213> Artificial Sequence
<220>
<223> Description of Artificial Sequence:synthetic DNA
<400> 32
cagaaaccag acaaagccgt taagctcc 28
<210> 33

<211> 384
<212> DNA
<213> Artificial Sequence
<220>
<223> Description of Artificial Sequence:synthetic DNA
<220>
<221> CDS
<222> (1)..(384)
<400> 33
atg atg tcc tct gct cag ttc ctt ggt ctc ctg ttg ctc tgt ttt caa 48
Met Met Ser Ser Ala Gln Phe Leu Gly Leu Leu Leu Leu Cys Phe Gln
-20 -15 -10 -5
ggt acc aga tgt gac atc cag atg acc cag tct cca tcc tcc ctg tct 96
Gly Thr Arg Cys Asp Ile Gln Met Thr Gln Ser Pro Ser Ser Leu Ser
-1 1 5 10
gca tct gta gga gac aga gtc acc atc act tgt agt gca agt cag gac 144
Ala Ser Val Gly Asp Arg Val Thr Ile Thr Cys Ser Ala Ser Gln Asp
15 20 25
att agt aat tat tta aac tgg tat cag cag aaa cca gac aaa gcc gtt 192
Ile Ser Asn Tyr Leu Asn Trp Tyr Gln Gln Lys Pro Asp Lys Ala Val
30 35 40
aag ctc ctg atc ttt tac tca tca aat tta cac tcg ggg gtc cca tca 240
Lys Leu Leu Ile Phe Tyr Ser Ser Asn Leu His Ser Gly Val Pro Ser
45 50 55 60
agg ttc agc ggc ggt gga tct ggg aca gat tat act ctc acc atc agc 288
Arg Phe Ser Gly Gly Gly Ser Gly Thr Asp Tyr Thr Leu Thr Ile Ser
65 70 75
agc ctg cag cct gaa gat ttt gca act tat tac tgt cat cag tat agt 336
Ser Leu Gln Pro Glu Asp Phe Ala Thr Tyr Tyr Cys His Gln Tyr Ser
80 85 90
aag ctt ccg tgg acg ttc ggc cag ggg acc aag gta gag att aaa cgt 384
Lys Leu Pro Trp Thr Phe Gly Gln Gly Thr Lys Val Glu Ile Lys Arg
95 100 105
<210> 34
<211> 48
<212> DNA
<213> Artificial Sequence
<220>
<223> Description of Artificial Sequence:synthetic DNA
<400> 34
gttgcgatat cttcaggctg cagattgctg atggtgagac tataatct 48
<210> 35
<211> 48

<212> DNA
<213> Artificial Sequence
<220>
<223> Description of Artificial Sequence:synthetic DNA
<400> 35
agattatagt ctcaccatca gcaatctgca gcctgaagat atcgcaac 48
<210> 36
<211> 384
<212> DNA
<213> Artificial Sequence
<220>
<223> Description of Artificial Sequence:synthetic DNA
<220>
<221> CDS
<222> (1)..(384)
<400> 36
atg atg tcc tct gct cag ttc ctt ggt ctc ctg ttg ctc tgt ttt caa 48
Met Met Ser Ser Ala Gln Phe Leu Gly Leu Leu Leu Leu Cys Phe Gln
-20 -15 -10 -5
ggt acc aga tgt gac atc cag atg acc cag tct cca tcc tcc ctg tct 96
Gly Thr Arg Cys Asp Ile Gln Met Thr Gln Ser Pro Ser Ser Leu Ser
-1 1 5 10
gca tct gta gga gac aga gtc acc atc act tgt agt gca agt cag gac 144
Ala Ser Val Gly Asp Arg Val Thr Ile Thr Cys Ser Ala Ser Gln Asp
15 20 25
att agt aat tat tta aac tgg tat cag cag aaa cca ggg aaa gcc cct 192
Ile Ser Asn Tyr Leu Asn Trp Tyr Gln Gln Lys Pro Gly Lys Ala Pro
30 35 40
aag ctc ctg atc ttt tac tca tca aat tta cac tcg ggg gtc cca tca 240
Lys Leu Leu Ile Phe Tyr Ser Ser Asn Leu His Ser Gly Val Pro Ser
45 50 55 60
agg ttc agc ggc ggt gga tct ggg aca gat tat agt ctc acc atc agc 288
Arg Phe Ser Gly Gly Gly Ser Gly Thr Asp Tyr Ser Leu Thr Ile Ser
65 70 75
aat ctg cag cct gaa gat atc gca act tat tac tgt cat cag tat agt 336
Asn Leu Gln Pro Glu Asp Ile Ala Thr Tyr Tyr Cys His Gln Tyr Ser
80 85 90
aag ctt ccg tgg acg ttc ggc cag ggg acc aag gta gag att aaa cgt 384
Lys Leu Pro Trp Thr Phe Gly Gln Gly Thr Lys Val Glu Ile Lys Arg
95 100 105
<210> 37
<211> 25
<212> DNA

<213> Artificial Sequence
<220>
<223> Description of Artificial Sequence:synthetic DNA
<400> 37
ttcaggctgc agattgctga tggtg 25
<210> 38
<211> 25
<212> DNA
<213> Artificial Sequence
<220>
<223> Description of Artificial Sequence:synthetic DNA
<400> 38
caccatcagc aatctgcagc ctgaa 25
<210> 39
<211> 384
<212> DNA
<213> Artificial Sequence
<220>
<223> Description of Artificial Sequence:synthetic DNA
<220>
<221> CDS
<222> (1)..(384)
<400> 39
atg atg tcc tct gct cag ttc ctt ggt ctc ctg ttg ctc tgt ttt caa 48
Met Met Ser Ser Ala Gln Phe Leu Gly Leu Leu Leu Leu Cys Phe Gln
-20 -15 -10 -5
ggt acc aga tgt gac atc cag atg acc cag tct cca tcc tcc ctg tct 96
Gly Thr Arg Cys Asp Ile Gln Met Thr Gln Ser Pro Ser Ser Leu Ser
-1 1 5 10
gca tct gta gga gac aga gtc acc atc act tgt agt gca agt cag gac 144
Ala Ser Val Gly Asp Arg Val Thr Ile Thr Cys Ser Ala Ser Gln Asp
15 20 25
att agt aat tat tta aac tgg tat cag cag aaa cca ggg aaa gcc cct 192
Ile Ser Asn Tyr Leu Asn Trp Tyr Gln Gln Lys Pro Gly Lys Ala Pro
30 35 40
aag ctc ctg atc ttt tac tca tca aat tta cac tcg ggg gtc cca tca 240
Lys Leu Leu Ile Phe Tyr Ser Ser Asn Leu His Ser Gly Val Pro Ser
45 50 55 60
agg ttc agc ggc ggt gga tct ggg aca gat tat act ctc acc atc agc 288
Arg Phe Ser Gly Gly Gly Ser Gly Thr Asp Tyr Thr Leu Thr Ile Ser
65 70 75
aat ctg cag cct gaa gat ttt gca act tat tac tgt cat cag tat agt 336
Asn Leu Gln Pro Glu Asp Phe Ala Thr Tyr Tyr Cys His Gln Tyr Ser

80 85 90
aag ctt ccg tgg acg ttc ggc cag ggg acc aag gta gag att aaa cgt 384
Lys Leu Pro Trp Thr Phe Gly Gln Gly Thr Lys Val Glu Ile Lys Arg
95 100 105
<210> 40
<211> 30
<212> DNA
<213> Artificial Sequence
<220>
<223> Description of Artificial Sequence:synthetic DNA
<400> 40
gacagaaata agttgcgata tcttcaggct 30
<210> 41
<211> 30
<212> DNA
<213> Artificial Sequence
<220>
<223> Description of Artificial Sequence:synthetic DNA
<400> 41
agcctgaaga tatcgcaact tatttctgtc 30
<210> 42
<211> 384
<212> DNA
<213> Artificial Sequence
<220>
<223> Description of Artificial Sequence:synthetic DNA
<220>
<221> CDS
<222> (1)..(384)
<400> 42
atg atg tcc tct gct cag ttc ctt ggt ctc ctg ttg ctc tgt ttt caa 48
Met Met Ser Ser Ala Gln Phe Leu Gly Leu Leu Leu Leu Cys Phe Gln
-20 -15 -10 -5
ggt acc aga tgt gac atc cag atg acc cag tct cca tcc tcc ctg tct 96
Gly Thr Arg Cys Asp Ile Gln Met Thr Gln Ser Pro Ser Ser Leu Ser
-1 1 5 10
gca tct gta gga gac aga gtc acc atc act tgt agt gca agt cag gac 144
Ala Ser Val Gly Asp Arg Val Thr Ile Thr Cys Ser Ala Ser Gln Asp
15 20 25
att agt aat tat tta aac tgg tat cag cag aaa cca ggg aaa gcc cct 192
Ile Ser Asn Tyr Leu Asn Trp Tyr Gln Gln Lys Pro Gly Lys Ala Pro
30 35 40
aag ctc ctg atc ttt tac tca tca aat tta cac tcg ggg gtc cca tca 240

Lys Leu Leu Ile Phe Tyr Ser Ser Asn Leu His Ser Gly Val Pro Ser
45                  50                  55                  60
agg ttc agc ggc ggt gga tct ggg aca gat tat act ctc acc atc agc    288
Arg Phe Ser Gly Gly Gly Ser Gly Thr Asp Tyr Thr Leu Thr Ile Ser
                65                  70                  75
agc ctg cag cct gaa gat atc gca act tat ttc tgt cat cag tat agt    336
Ser Leu Gln Pro Glu Asp Ile Ala Thr Tyr Phe Cys His Gln Tyr Ser
            80                  85                  90
aag ctt ccg tgg acg ttc ggc cag ggg acc aag gta gag att aaa cgt    384
Lys Leu Pro Trp Thr Phe Gly Gln Gly Thr Lys Val Glu Ile Lys Arg
        95                  100                 105
<210> 43
<211> 87
<212> DNA
<213> Artificial Sequence
<220>
<223> Description of Artificial Sequence:synthetic DNA
<400> 43
gcactacaag tgatggtgac tctgtctcct acagatgcag gcagggagga tgcagactgg 60
gtcatctgga tgtcacatct ggtacct                                     87
<210> 44
<211> 89
<212> DNA
<213> Artificial Sequence
<220>
<223> Description of Artificial Sequence:synthetic DNA
<400> 44
gtcaccatca cttgtagtgc aagtcaggac attagtaatt atttaaactg gtatcagcag 60
aaaccaggga aagccgttaa gctcctgat                                   89
<210> 45
<211> 89
<212> DNA
<213> Artificial Sequence
<220>
<223> Description of Artificial Sequence:synthetic DNA
<400> 45
taatctgtcc cagatccacc gccgctgaac cttgatggga cccccgagtg taaatttgat 60
gagtaaaaga tcaggagctt aacggcttt                                   89
<210> 46
<211> 92
<212> DNA
<213> Artificial Sequence
<220>

<223> Description of Artificial Sequence:synthetic DNA
<400> 46
ggtggatctg ggacagatta tactctcacc atcagcagcc tgcagcctga agattttgca 60
acttatttct gtcatcagta tagtaagctt cc 92
<210> 47
<211> 384
<212> DNA
<213> Artificial Sequence
<220>
<223> Description of Artificial Sequence:synthetic DNA
<220>
<221> CDS
<222> (1)..(384)
<400> 47
atg atg tcc tct gct cag ttc ctt ggt ctc ctg ttg ctc tgt ttt caa 48
Met Met Ser Ser Ala Gln Phe Leu Gly Leu Leu Leu Leu Cys Phe Gln
-20 -15 -10 -5
ggt acc aga tgt gac atc cag atg acc cag tct gca tcc tcc ctg cct 96
Gly Thr Arg Cys Asp Ile Gln Met Thr Gln Ser Ala Ser Ser Leu Pro
-1 1 5 10
gca tct gta gga gac aga gtc acc atc act tgt agt gca agt cag gac 144
Ala Ser Val Gly Asp Arg Val Thr Ile Thr Cys Ser Ala Ser Gln Asp
15 20 25
att agt aat tat tta aac tgg tat cag cag aaa cca ggg aaa gcc gtt 192
Ile Ser Asn Tyr Leu Asn Trp Tyr Gln Gln Lys Pro Gly Lys Ala Val
30 35 40
aag ctc ctg atc ttt tac tca tca aat tta cac tcg ggg gtc cca tca 240
Lys Leu Leu Ile Phe Tyr Ser Ser Asn Leu His Ser Gly Val Pro Ser
45 50 55 60
agg ttc agc ggc ggt gga tct ggg aca gat tat act ctc acc atc agc 288
Arg Phe Ser Gly Gly Gly Ser Gly Thr Asp Tyr Thr Leu Thr Ile Ser
65 70 75
agc ctg cag cct gaa gat ttt gca act tat ttc tgt cat cag tat agt 336
Ser Leu Gln Pro Glu Asp Phe Ala Thr Tyr Phe Cys His Gln Tyr Ser
80 85 90
aag ctt ccg tgg acg ttc ggc cag ggg acc aag gta gag att aaa cgt 384
Lys Leu Pro Trp Thr Phe Gly Gln Gly Thr Lys Val Glu Ile Lys Arg
95 100 105
<210> 48
<211> 384
<212> DNA
<213> Artificial Sequence
<220>

<223> Description of Artificial Sequence:synthetic DNA
<220>
<221> CDS
<222> (1)..(384)
<400> 48
atg atg tcc tct gct cag ttc ctt ggt ctc ctg ttg ctc tgt ttt caa    48
Met Met Ser Ser Ala Gln Phe Leu Gly Leu Leu Leu Leu Cys Phe Gln
-20                 -15                 -10                 -5
ggt acc aga tgt gac atc cag atg acc cag tct cca tcc tcc ctg tct    96
Gly Thr Arg Cys Asp Ile Gln Met Thr Gln Ser Pro Ser Ser Leu Ser
            -1  1               5                   10
gca tct gta gga gac aga gtc acc atc act tgt agt gca agt cag gac    144
Ala Ser Val Gly Asp Arg Val Thr Ile Thr Cys Ser Ala Ser Gln Asp
        15                  20                  25
att agt aat tat tta aac tgg tat cag cag aaa cca gac aaa gcc gtt    192
Ile Ser Asn Tyr Leu Asn Trp Tyr Gln Gln Lys Pro Asp Lys Ala Val
    30                  35                  40
aag ctc ctg atc ttt tac tca tca aat tta cac tcg ggg gtc cca tca    240
Lys Leu Leu Ile Phe Tyr Ser Ser Asn Leu His Ser Gly Val Pro Ser
45                  50                  55                  60
agg ttc agc ggc ggt gga tct ggg aca gat tat act ctc acc atc agc    288
Arg Phe Ser Gly Gly Gly Ser Gly Thr Asp Tyr Thr Leu Thr Ile Ser
                65                  70                  75
agc ctg cag cct gaa gat atc gca act tat ttc tgt cat cag tat agt    336
Ser Leu Gln Pro Glu Asp Ile Ala Thr Tyr Phe Cys His Gln Tyr Ser
            80                  85                  90
aag ctt ccg tgg acg ttc ggc cag ggg acc aag gta gag att aaa cgt    384
Lys Leu Pro Trp Thr Phe Gly Gln Gly Thr Lys Val Glu Ile Lys Arg
        95                  100                 105
<210> 49
<211> 119
<212> PRT
<213> Mus musculus
<400> 49
Glu Val Thr Leu Val Glu Ser Gly Gly Asp Phe Val Lys Pro Gly Gly
1               5                   10                  15
Ser Leu Lys Val Ser Cys Ala Ala Ser Gly Phe Ala Phe Ser His Tyr
            20                  25                  30
Ala Met Ser Trp Val Arg Gln Thr Pro Ala Lys Arg Leu Glu Trp Val
        35                  40                  45
Ala Tyr Ile Ser Ser Gly Gly Ser Gly Thr Tyr Tyr Ser Asp Ser Val
    50                  55                  60
Lys Gly Arg Phe Thr Ile Ser Arg Asp Asn Ala Lys Asn Thr Leu Tyr

65                  70                  75                  80
Leu Gln Met Arg Ser Leu Arg Ser Glu Asp Ser Ala Met Tyr Phe Cys
                85                  90                  95
Thr Arg Val Lys Leu Gly Thr Tyr Tyr Phe Asp Ser Trp Gly Gln Gly
            100                 105                 110
Thr Thr Leu Thr Val Ser Ser
        115
<210> 50
<211> 108
<212> PRT
<213> Mus musculus
<400> 50
Asp Ile Gln Met Thr Gln Thr Ala Ser Ser Leu Pro Ala Ser Leu Gly
 1               5                  10                  15
Asp Arg Val Thr Ile Ser Cys Ser Ala Ser Gln Asp Ile Ser Asn Tyr
            20                  25                  30
Leu Asn Trp Tyr Gln Gln Lys Pro Asp Gly Thr Val Lys Leu Leu Ile
        35                  40                  45
Phe Tyr Ser Ser Asn Leu His Ser Gly Val Pro Ser Arg Phe Ser Gly
    50                  55                  60
Gly Gly Ser Gly Thr Asp Tyr Ser Leu Thr Ile Ser Asn Leu Glu Pro
65                  70                  75                  80
Glu Asp Ile Ala Thr Tyr Phe Cys His Gln Tyr Ser Lys Leu Pro Trp
                85                  90                  95
Thr Phe Gly Gly Gly Thr Lys Leu Glu Ile Lys Arg
            100                 105

## Description

### TECHNICAL FIELD

The present invention relates to a medicament which comprises a gene recombinant antibody against ganglioside GD3 or the antibody fragment thereof in combination with at least one of a substance which activates an immunocompetent cell and a substance having an antitumor activity.

### BACKGROUND OF THE INVENTION

Ganglioside which is one of glycolipids containing sialic acid is a constituent of animal cell membrane. Also, ganglioside has a sugar chain as a hydrophilic side chain and a sphingosine and fatty acid as hydrophobic side chains. It is known that kinds and expression levels of ganglioside vary depending on the cell types, organ species, animal species and the like. It is also known that the expression of ganglioside changes quantitatively and qualitatively in the process of malignant transformation of cells [*Cancer Res*., 45, 2405 (1985)].

Particularly, it is known that GD3 is present in an extremely small amount in normal cells but in a large amount in cancer cells, particularly melanoma, and is present together with ganglioside GD2 in cancer cells such as sarcoma, glioma and neuroblastoma [*Proc. Natl. Acad Sci. U.S.A*., 77, 6114 (1980); *J. Exp. Med*., 155, 1133 (1982); *Cancer Res*., 45, 4401 (1985); *Cancer*, 70, 633 (1992); *Acta Neuropathologica,* 82, 45 (1991)], and an antibody against GD3 (hereinafter referred to as "anti-GD3 antibody") is considered to be useful for the treatment of these cancers [*Melanoma Research*, 7, S115 (1997)].

Administration tests to melanoma patients have already been carried out using R24 (hereinafter referred to as "anti-GD3 mouse antibody R24") as an anti-GD3 mouse monoclonal antibody, and its clinical responses were observed in a part of the patients but not to an expected degree because of considerably rapid disappearance of the antibody from blood due to induction of a human antibody against the mouse antibody in the body of the patient (human anti mouse antibody: hereinafter referred to as "HAMA") [*Melanoma Research*, 7, S115 (1997)].

In order to prevent decrease of therapeutic effects derived from antigenicity such as generation of HAMA when a mouse antibody is administered to human, methods for the production of humanized antibodies for attenuating antigenicity for human have so far been known, such as a human chimeric antibody in which a variable region derived from an animal other than human and a constant region derived from human are bound [*Proc. Natl. Acad Sci. U.S.A*., 81, 6851 (1984)] and a human CDR-grafted antibody in which, among the variable regions, all of framework regions other than the ultra-variable region which directly relate to binding to the antigen are further changed to human-derived sequences [*Nature*, 321, 522 (1986)]. Regarding antibodies for melanoma, anti-GD3 human chimeric antibody KM871 (Japanese Published Unexamined Patent Application No. 304989/93) and anti-GD2 human chimeric antibody ch14.18 [*J. Immunol*., 144, 1382, (1990)] are known. The ch14.18 has already been used in clinical studies and its good blood retentivity has been observed [*Human Antibodies & Hybridomas*, 3, 19 (1992)].

Also, since increase in cytotoxic activity via a constant region can be found by the administration of humanized antibodies to the living body, it is considered that they are advantageous in view of therapeutic effects particularly for antibodies against cancers and the like. Since antibody-dependent cell-mediated cytotoxic activity (hereinafter referred to as "ADCC activity") using human peripheral blood monocyte as an effector and complement-dependent cytotoxicity (hereinafter referred to as "CDC activity") using human serum as the complement source are increased by the anti-GD3 human chimeric antibody KM871 in comparison with the original mouse-derived antibody [*Cancer Immunol. Immunother*., 36, 373 (1993)], the antibody is considered to be useful in treating GD3-positive melanoma.

In general chemotherapy, there is a case in which a good therapeutic result is obtained by its combined use with an antibody even when it is difficult to introduce remission due to appearance of a drug-resistance tumor cell and the like. Significant therapeutic results have been obtained from combined use of an anti-HER2/neu humanized antibody for breast cancer, rhuMAb HER2 (Herceptin/trastuzumab) with a taxane-series antitumor agent [*Clinical Therapeutics*, 21, 309 (1999)] and from combined use of an anti-CD20 human chimeric antibody for B cell lymphoma, IDEC-C2B8 (Rituxan, manufactured by Roche) with polypharmacy [*J. Clin. Oncol*., 17, 268 (1999)].

Regarding combined use of an antibody with a substance having an antitumor activity in melanoma treatment, it is known that CDC sensitivity of an anti-GD3 mouse antibody R24 against a complement non-sensitive melanoma cell line is increased by combined use of an anti-GD3 mouse antibody R24 with doxorubicin [*Proc. Natl. Acad Sci. U.S.A*., 83, 9144 (1986)]. However, no significant therapeutic effect was observed in the phase I clinical study of the combined use of the anti-GD3 mouse antibody R24 with dacarbazine, combined use of the anti-GD3 mouse antibody R24 with cisplatin or combined use of the anti-GD3 mouse antibody R24 with doxorubicin [*Melanoma Res*., 7, Supple 2, S155 (1997)].

Combined therapy using an antibody with cytokine is also expected as new immunotherapy. Cytokine is a general term for various humoral factors which relate to cytoclesis in immune response. ADCC activity as one of cytotoxic activities is induced by effector cells having Fc receptor on the cell surface such as monocyte, macrophage and NK cell [*J. Immunol*., 138, 1992 (1987)]. Since various species of cytokine activate these effector cells, administration in combination with an antibody has been tried in order to increase ADCC activity and the like of the antibody.

Regarding the anti-GD3 mouse antibody R24, a possibility of combined use effects with IL-2 has been suggested based on *in vitro* evaluation (U.S. Patent 5,104,652). Also, clinical studies have been carried out on the combined administration of the anti-GD3 mouse antibody R24 with IL-2, the anti-GD3 mouse antibody R24 with IFNα, the anti-GD3 mouse antibody R24 with M-CSF, the anti-GD3 mouse antibody R24 with GM-CSF, and the like [*Medical Oncology*, 15, 191 (1998), *Clinical Cancer Res*., 3, 17 (1997), *Cancer*, 75, 2251 (1995), *Journal Of Immunotherapy With Emphasis On Tumor Immunology*, 16, 132 (1994)]. However, effects to be expected were not found by these combined therapies due to antigenicity of the mouse antibody or side effects of cytokine. It is important to predict therapeutic effects when used in the clinical field, by *in vivo* evaluation using a tumor model animal having high extrapolation ability to human melanoma treatment. However, only combined use of the anti-GD3 mouse antibody R24 with M-CSF is known regarding examination of antitumor effects by combined administration of the anti-GD3 mouse antibody R24 with cytokine to animal tumor models [*Journal of Immunotherapy with Emphasis on Tumor Immunology*, 19, 317 (1996)].

Melanoma is a malignant tumor in chromocytes and is known as a tumor having high malignancy, which is apt to generate metastasis and has high resistance against chemotherapy and the like. Immunotherapy by IL-2 or interferon, chemotherapy mainly using dacarbazine and cisplatin, combined therapy thereof and the like has so far been carried out.

As an example of the immunotherapy, a combination of IL-2 with IFNα is known [*J. Clin. Oncol*., 7, 1863 (1989), *J. Clin. Oncol*., 11, 1969 (1993), *J. Immunother*., 13, 117 (1993)].

As examples of the chemotherapy, a combination of dacarbazine, cisplatin, carmustine and tamoxifen [*Cancer Treat. Rep*., 68, 1403 (1984), *Cancer*, 63, 1292 (1989)], a combination of dacarbazine, cisplatin and vinblastine [*Proc. ASCO*, 12, 1328 (1993)], a combination of dacarbazine, fotemustine and vindesine [*Proc. ASCO*, 33, 1336 (1992)] and the like are known.

As examples of the combined therapy of immunotherapy and chemotherapy, dacarbazine and IL-2 [*J. Natl. Cancer Inst*., 82, 1345 (1990)], dacarbazine and IFNα [*J. Clin. Oncol*., 9, 1403 (1991), *J. Clin. Oncol*., 12, 806 (1994)], cisplatin and IL-2 [*J. Clin. Oncol*., 9, 1821 (1991)], cisplatin and IFNα [*J. Clin. Oncol*., 10, 1574 (1992)], cisplatin, dacarbazine, IL-2 and IFNα [*Proc. ASCO*, 10, 1029 (1991)] and the like are known.

However, although a slight improvement is recognized by the chemotherapy regarding the efficacy, great improvement is not still found on the survival rate of 5 years or more [*Jpn. J. Cancer Chemotherapy*, 27, S238 (2000)]. Also, since IL-2 [*Immunology Today*, 9, 58 (1998)] and chemotherapy show strong side effects, a large dose cannot be administered to patients.

### DISCLOSURE OF THE INVENTION

In order to obtain high therapeutic effects in treating malignant tumors, particularly melanoma, a new therapeutic method having less side effects, or a new therapeutic method which can provide further high therapeutic effects at conventional doses of agents has been desired. An object of the present invention is to provide a medicament which can provide higher therapeutic effects than any one of a gene recombinant antibody against ganglioside GD3 or the antibody fragment thereof, and a substance which activates an immunocompetent cell and a substance having an antitumor activity as single agents, by combining the gene recombinant antibody against ganglioside GD3 or the antibody fragment thereof with at least one of the substance which activates an immunocompetent cell and the substance having an antitumor activity. Also, the medicament is expected to relieve side effects which were problems in the case of single administration.

The present invention relates to the following (1) to (12):
(1) A medicament which comprises a gene recombinant antibody against ganglioside GD3 or the antibody fragment thereof in combination with at least one of a substance which activates an immunocompetent cell and a substance having an antitumor activity.
(2) An antitumor agent which comprises a gene recombinant antibody against ganglioside GD3 or the antibody fragment thereof in combination with at least one of a substance which activates an immunocompetent cell and a substance having an antitumor activity.
(3) The antitumor agent according to (2), wherein the tumor is melanoma.
(4) The agent according to any one of (1) to (3), wherein the gene recombinant antibody against ganglioside GD3 is a humanized antibody against ganglioside GD3.
(5) The agent according to (4), wherein the humanized antibody is a human chimeric antibody or a human complementarity determining region (CDR)-grafted antibody.
(6) The agent according to (5), wherein the humanized antibody is a humanized antibody which comprises CDR1, CDR2 and CDR3 of a heavy chain (H chain) variable region (V region) comprising the amino acid sequences represented by SEQ ID NOs:3, 4 and 5, respectively, and CDR1, CDR2 and CDR3 of a light chain (L chain) variable region (V region) comprising the amino acid sequences represented by SEQ ID NOs:6, 7 and 8, respectively.
(7) The agent according to (5), wherein the human chimeric antibody is a human chimeric antibody which comprises an H chain V region and an L chain V region comprising the amino acid sequences represented by SEQ ID NOs:49 and 50, respectively.
(8) The agent according to (5), wherein the human CDR-grafted antibody is a human CDR-grafted antibody which comprises an H chain V region and an L chain V region of the human CDR-grafted antibody comprising the amino acid sequences represented by SEQ ID NOs:9 and 10, respectively.
(9) The agent according to any one of (1) to (8), wherein the substance which activates an immunocompetent cell is cytokine.
(10) The agent according to (9), wherein the cytokine is selected from the group consisting of IFNα, IFNβ, IFNγ, TNFα, TNFβ, IL-1α, IL-1β, IL-2, IL-3, IL-4, IL-6, IL-7, IL-9, IL-12, IL-15, IL-17, IL-18, GM-CSF, G-CSF, M-CSF and SCF.
(11) The agent according to any one of (1) to (8), wherein the substance having an antitumor activity is a hormonotherapeutant agent or a chemotherapy agent.
(12) The agent according to (11), wherein the hormonotherapeutant agent or the chemotherapy agent is an agent selected from the group consisting of dacarbazine, cisplatin, fotemustine, carmustine, nimustine, lomustine, semustine, pacritaxel, dosetaxel, vindesine, vinblastine, vincristine, carboplatin, bleomycin, doxorubicin and tamoxifen.

The present invention relates to a medicament which comprises a gene recombinant antibody against ganglioside GD3 or the antibody fragment thereof in combination with at least one of a substance which activates an immunocompetent cell and a substance having an antitumor activity. As the medicament of the present invention, mention may be made of the antibody in combination with one of a substance which activates a single immunocompetent cell and a substance having an antitumor activity; the antibody in combination with two or more of substances which activate a immunocompetent cell and substances having an antitumor activity; a medicament which comprises the antibody and at least one of a substance which activates a immunocompetent cell and a substance having an antitumor activity; or a medicament which comprises the antibody and at least one of a substance which activates a immunocompetent cell and a substance having an antitumor activity and which is administered to the living body simultaneously or separately.

The gene recombinant antibody includes a humanized antibody, a human antibody and the like.

The humanized antibody includes a human chimeric antibody, a human CDR-grafted antibody and the like.

A human chimeric antibody is an antibody which comprises an H chain V region (hereinafter referred to as "HV" or "VH") and an L chain V region (hereinafter referred to as "LV" or "VL") of an antibody derived from a non-human animal, an H chain C region (hereinafter referred to as "CH") of a human antibody and an L chain C region (hereinafter referred to as "CL") of a human antibody. As the non-human animal, any animal such as a mouse, a rat, a hamster or a rabbit can be used, so long as a hybridoma can be prepared therefrom.

The human chimeric antibody of the present invention can be produced by obtaining cDNAs encoding VH and VL from a hybridoma capable of producing a monoclonal antibody which specifically reacts with GD3, constructing a human chimeric antibody expression vector by inserting them into an expression vector for animal cells having genes encoding human antibody CH and human antibody CL, and expressing the expression vector by introducing it into an animal cell.

As the CH of a human chimeric antibody, it may be any region which belongs to human immunoglobulin (hereinafter referred to as "hIg"). Those of hIgG class are preferred and any one of subclasses belonging to hIgG class such as hIgG 1, hIgG2, hIgG3 and hIgG4 can also be used. Also, as the CL of a human chimeric antibody, it may be any region which belongs to hIg, and those of κ class or λ class can be used.

The anti-GD3 human chimeric antibody includes a human chimeric antibody which comprises CDR1, CDR2 and CDR3 of VH comprising the amino acid sequences represented by SEQ ID NOs:3, 4 and 5, respectively, and CDR1, CDR2 and CDR3 of VL comprising the amino acid sequences represented by SEQ ID NOs:6, 7 and 8, respectively; specifically, a human chimeric antibody which comprises VH and VL comprising the amino acid sequences represented by SEQ ID NOs:49 and 50, respectively; and more specifically, a human chimeric antibody which comprises VH comprising the amino acid sequence represented by SEQ ID NO:49, an H chain C region comprising an amino acid sequence of a human antibody IgG1 subclass, an L chain V region comprising the amino acid sequence represented by SEQ ID NO:50, and an L chain C region comprising an amino acid sequence of a human antibody κ class. Examples include anti-GD3 human chimeric antibody KM871 described in Japanese Published Unexamined Patent Application No. 304989/93.

A human CDR-grafted antibody is an antibody in which amino acid sequences of CDRs in VH and VL derived from a non-human animal antibody are grafted to appropriate positions of VH and VL of a human antibody.

The anti-CD3 CDR-grafted antibody of the present invention can be produced by constructing cDNAs encoding V regions in which CDR sequences of VH and VL of an antibody derived from a non-human animal which specifically reacts with GD3 have been grafted to CDR sequences of VH and VL of any human antibody, constructing a human CDR-grafted antibody expression vector by inserting them into an expression vector for animal cells having genes encoding human antibody CH and human antibody CL, and expressing the human CDR-grafted antibody by introducing the expression vector into an animal cell.

As the CH of a human CDR-grafted antibody, it may be any region which belongs to hIg. Those of hIgG class are preferred and any one of subclasses belonging to hIgG class such as hIgG1, hIgG2, hIgG3 and hIgG4 can also be used. Also, as the CL of a human chimeric antibody, it may be any region which belongs to hIg, and those of κ class or λ class can be used.

The anti-GD3 CDR-grafted antibody includes a human CDR-grafted antibody which comprises CDR1, CDR2 and CDR3 of VH comprising the amino acid sequences represented by SEQ ID NOs:3, 4 and 5, respectively, and CDR1, CDR2 and CDR3 of VL comprising the amino acid sequences represented by SEQ ID NOs:6, 7 and 8, respectively; specifically, a human CDR-grafted antibody which comprises VH and VL comprising the amino acid sequences represented by SEQ ID NOs:9 and 10, respectively; and more specifically, a human CDR-grafted antibody which comprises VH comprising the amino acid sequence represented by SEQ ID NO:9, an H chain C region comprising an amino acid sequence of a human antibody IgG1 subclass, an L chain V region comprising the amino acid sequence represented by SEQ ID NO:10, and an L chain C region comprising an amino acid sequence of a human antibody κ class. Examples include anti-GD3 human CDR-grafted antibody KM8871 produced by transformed cell clone KM8871 which has been internationally deposited as FERM BP-6790 on July 22, 1999, in National Institute of Bioscience and Human Technology, Agency of Industrial Science and Technology, the Ministry of International Trade and Industry (present name: International Patent Organism Depositary, National Institute of Advanced Industrial Science and Technology (AIST Tsukuba Central 6, 1-1, Higashi 1-Chome Tsukuba-shi, Ibaraki-ken, Japan).

Originally, a human antibody is an antibody naturally present in the human body. It also includes antibodies obtained from a human antibody phage library and a human antibody-producing transgenic animal which are produced based on the recent advance in genetic engineering, cell engineering and developmental engineering techniques.

With regard to the antibody present in the human body, a lymphocyte capable of producing the antibody can be cultured by isolating a human peripheral blood lymphocyte and infecting it with EB virus or the like to thereby immortalize it, followed by cloning, and the antibody can be purified from the culture mixture.

The human antibody phage library is a library in which antibody fragments such as Fab and scFv are expressed on the phage surface by inserting an antibody gene prepared from human B cell into a phage gene. From the library, a phage expressing a desired antibody fragment having an antigen binding activity can be recovered, using its activity to bind to an antigen-immobilized substrate as the marker. The antibody fragment can be further converted genetic engineeringly into a human antibody molecule having two complete H chains and two complete L chains.

A human antibody-producing transgenic non-human animal is an animal in which a human antibody gene is introducing into cells. Specifically, a human antibody-producing transgenic animal can be produced by introducing a human antibody gene into a mouse ES cell, inoculating the ES cell into an early stage embryo of other mouse and then developing it. As the preparation method of a human antibody from the human antibody-producing transgenic animal, the human antibody can be produced and accumulated in a culture by obtaining a human antibody-producing hybridoma according to a hybridoma production method usually carried out in non-human mammals and then culturing it.

The antibody fragment includes Fab, Fab', F(ab')₂, scFv, dsFv, a peptide comprising CDR and the like.

An Fab is an antibody fragment having a molecular weight of approximately 50,000 and an antigen binding activity, in which about a half of the N-terminal side of H chain and a full L chain, among fragments obtained by treating IgG with a protease, papain (cut at the amino acid residue at position 224 in the H chain), are bound together through a disulfide bond.

The Fab of the present invention can be obtained by treating an antibody which specifically reacts with GD3 with a protease, papain. Also, the Fab can be produced by inserting a DNA encoding Fab of the antibody into an expression vector, and introducing the vector into a host cell to express the Fab.

An F(ab')₂ is an antibody fragment having a molecular weight of approximately 100,000 and an antigen binding activity, which is slightly larger than the Fab bound via a disulfide bond of the hinge region, among fragments obtained by treating IgG with a protease, pepsin (cut at the amino acid residue at position 234 in the H chain).

The F(ab')₂ of the present invention can be obtained by treating an antibody which specifically reacts with GD3, with a protease, pepsin. Also, the F(ab')₂ can be produced by binding Fab' described below via a thioether bond or a disulfide bond.

An Fab' is an antibody fragment having a molecular weight of approximately 50,000 and an antigen binding activity, which is obtained by cutting a disulfide bond of the hinge region of the F(ab')₂.

The Fab' of the present invention can be obtained by treating F(ab')₂ which specifically reacts with GD3 with a reducing agent, dithiothreitol. Also, the Fab' can be produced by inserting DNA encoding an Fab' fragment of the antibody into an expression vector, and introducing the vector into a host cell to express the Fab'.

An scFv is a VH-P-VL or VL-P-VH polypeptide in which one chain VH and one chain VL are linked using an appropriate peptide linker (hereinafter referred to as "P"). The VH and VL in the scFv used in the present invention may be derived from any one of antibodies produced by a hybridoma, a humanized antibody and a human antibody.

The scFv of the present invention can be produced by obtaining cDNAs encoding the VH and VL of an antibody which specifically reacts with GD3, constructing DNA encoding scFv, inserting the DNA into an expression vector, and then introducing the expression vector into a host cell to express the scFv.

A dsFv is obtained by binding polypeptides in which one amino acid residue of each of VH and VL is substituted with a cysteine residue via a disulfide bond between the cysteine residues. The amino acid residue substituted with a cysteine residue can be selected based on a three-dimensional structure estimation of the antibody in accordance with the method shown by Reiter *et al*. [*Protein Engineering*, 7, 697 (1994)]. The VH and VL in the dsFv of the present invention may be derived from any one of antibodies produced by a hybridoma, a humanized antibody and a human antibody.

The dsFv of the present invention can be produced by obtaining cDNAs encoding the VH and VL of an antibody which specifically reacts with GD3, constructing DNA encoding dsFv, inserting the DNA into an expression vector, and then introducing the expression vector into a host cell to express the dsFv.

A peptide comprising CDR is constituted by at least one region of H chain and L chain CDRs. Plural CDRs can be bound directly or via an appropriate peptide linker.

The peptide comprising CDR of the present invention can be produced by obtaining cDNA encoding the VH and VL of an antibody which specifically reacts with GD3, inserting the cDNA into an expression vector, and then introducing the expression vector into a host cell to express the peptide comprising CDR.

Also, the peptide comprising CDR can be produced by a chemical synthesis method such as Fmoc method (fluorenylmethoxycarbonyl method) or tBoc method (t-butyloxycarbonyl method).

The substance which activates an immunocompetent cell or the substance having an antitumor activity used in the present invention includes cytokine, a chemotherapeutant, a hormonotherapeutant and the like.

Any cytokine can be used, so long as it activates an effector cell relating to ADCC activity caused by an antibody, such as NK cell, macrophage, monocyte and granulocyte. The cytokine which activates NK cell includes IL-2, IFNα, IL-12, IL-18 and the like, and IL-2 is preferred. The cytokine which activates macrophage includes M-CSF. The cytokine which activates monocyte and granulocyte includes GM-CSF.

The cytokine having an antitumor activity includes interferons, TNFα, TNFβ, IL-1α, IL-1β and the like, and IFNα is preferred.

The chemotherapeutant and hormonotherapeutant used in the present invention include dacarbazine, cisplatin, fotemustine, carmustine, nimustine, lomustine, semustine, pacritaxel, dosetaxel, vindesine, vinblastine, vincristine, carboplatin, bleomycin, doxorubicin, tamoxifen and the like.

The medicament of the present invention includes anti-GD3 human chimeric antibody KM871 or anti-GD3 CDR-grafted antibody KM8871 in combination with at least one agent selected from IL-2, IFNα, dacarbazine and cisplatin.

Although the above substance which activates an immunocompetent cell or substance having an antitumor activity has a possibility of inducing side effects when it is administered alone to the living body at a high dose, the medicament of the present invention can relieve side effects because it can be used at a lower dose.

The medicament of the present invention can be used against tumors such as melanoma, sarcoma, glioma and neuroblastoma. Particularly, the medicament is preferably used against tumors in which ganglioside GD3 is highly expressed.

Antitumor effects can be examined using an animal model.

The animal model includes a xenograft model, a syngeneic graft model and the like. A xenograft model can be prepared by inoculating a culture cell line derived from a human cancer tissue, into various regions of an immunodeficiency mouse such as a nude mouse, for example, subcutaneously, intracutaneously, intraperitoneally, intravenously, *etc.*

A syngeneic graft model can be prepared by inoculating a cultured mouse cancer cell line into various regions of a wild-type mouse having a normal immune system, for example, subcutaneously, intracutaneously, intraperitoneally, intravenously, *etc*..

When antitumor effects by the combined use with cytokine such as IL-2 capable of activating T cells are examined, evaluation is preferably carried out using a syngeneic graft model having no T cell defection.

A syngeneic graft model for evaluation of a GD3 monoclonal antibody is prepared as follows.

A syngeneic graft model for evaluation of an anti-GD3 monoclonal antibody can be prepared by inoculating a GD-3 positive mouse cell into a wild-type mouse having a normal immune system. The GD3-positive mouse can be prepared by introducing a GM3-specific α-2,8-sialyltransferase (GD3 synthase) gene into a mouse culture cell line which expresses a GD3 biosynthesis precursor, GM3, to prepare a GD3-positive transformant [*Proc. Natl. Acad Sci. U.S.A*., 91, 10455 (1994)].

A xenograft model for evaluation of a GD3 monoclonal antibody can be prepared by inoculating into a nude mouse a tumor section collected from a tumor mass of a GD3-positive human malignant myeloma cell line, such as H-187 cell or G-361.

The antitumor effects of the medicament of the present invention can be evaluated using the above animal model by comparing the effects of the medicament of the present invention with effects of the administration of an antibody alone and administration of a substance which activates an immunocompetent cell alone or a substance having an antitumor activity alone.

The medicament of the present invention can be administered alone. Generally, it is preferred to provide it in the form of a pharmaceutical formulation produced by mixing it with at least one pharmaceutically acceptable carrier in accordance with a method well known in the technical field of pharmaceutics.

It is preferred to select a route of administration which is the most effective in carrying out the intended treatment such as oral administration or parenteral administration, e.g., intraorally, tracheally, rectally, subcutaneously, intramuscularly, intravenously, *etc*. In the case of a protein formulation, intravenous administration is preferred.

The dosage form includes sprays, capsules, tablets, granules, syrups, emulsions, suppositories, injections, ointments, tapes and the like.

Formulations suitable for oral administration include emulsions, syrups, capsules, tablets, powders, granules and the like.

Liquid preparations such as emulsions and syrups, can be produced using additives, for example, water; saccharides such as sucrose, sorbitol and fructose; glycols such as polyethylene glycol and propylene glycol; oils such as sesame oil, olive oil and soybean oil; antiseptics such as p-hydroxybenzoate; flavors such as strawberry flavor and peppermint; and the like.

Capsules, tablets, powders, granules and the like can be produced using additives, for example, excipients such as lactose, glucose, sucrose and mannitol; disintegrants such as starch and sodium alginate; lubricants such as magnesium stearate; binders such as polyvinyl alcohol, hydroxypropylcellulose and gelatin; surfactants such as fatty acid ester; plasticizers such as glycerine; and the like.

Formulations suitable for parenteral administration include injections, suppositories, sprays and the like.

Injections can be prepared using a carrier such as a salt solution, a glucose solution or a mixture thereof

Suppositories can be prepared using a carrier such as cacao butter, hydrogenated fat or carboxylic acid. Also, sprays can be prepared from the medicament itself or using a carrier or the like which does not stimulate oral and airway mucous membranes of patients and can facilitate absorption of the medicament by dispersing it as minute particles.

The carrier includes lactose, glycerine and the like. Depending on the properties of the medicament and the used carrier, aerosols, dry powders and the like can be produced. The additives exemplified in the oral preparations can also be added to the parenteral preparations. The dose and frequency of administration vary depending on intended therapeutic effects, administration method, treating period, age, body weight and the like, and the dose for adult patient is generally from 0.1 to 20 mg/kg in terms of anti-GD3 antibody per one administration. The agent used in combination with the antibody is administered at a dose equal to or lower than the dose when the agent is used alone in clinic.

Processes for producing a humanized antibody against ganglioside GD3 are explained below.

### 1. Production of humanized antibody

### (1) Construction of humanized antibody expression vector

The humanized antibody expression vector is an expression vector for animal cells to which genes encoding CH and CL of a human antibody are inserted, and can be constructed by cloning the genes encoding CH and CL of a human antibody into an expression vector for animal cells. The C regions of a human antibody can be any human antibody CH and CL, and examples include a C region belonging to IgG1 subclass in an H chain of a human antibody (hereinafter referred to as "hCγ1") and a C region belonging to κ class in an L chain of a human antibody (hereinafter referred to as "hCκ"). A chromosomal DNA comprising an exon and an intron can be used as the gene encoding CH and CL of a human antibody, and cDNA can also be used.

Any expression vector for animal cells can be used, so long as a gene encoding the human antibody C region can be inserted and expressed. Examples include pAGE107 [*Cytotechnology*, 3, 133 (1990)], pAGE103 [*J. Biochem*., 101, 1307 (1987)], pHSG274 [*Gene*, 27, 223 (1984)], pKCR [*Proc. Natl. Acad Sci. U.S.A*., 78, 1527 (1981)], pSG1βd2-4 [*Cytotechnology*, 4, 173 (1990)] and the like. The promoter and the enhancer used in the expression vector for animal cell include early promoter and enhancer of SV40 [*J. Biochem*., 101, 1307 (1987)], LTR promoter and enhancer of Moloney mouse leukemia virus [*Biochem. Biophys. Res. Comun*., 149, 960 (1987)], promoter [*Cell*, 41, 479 (1985)] and enhancer [*Cell*, 33, 717 (1983)] of immunoglobulin H chain, and the like.

The humanized antibody expression vector may be either of a type in which a gene encoding an antibody H chain and a gene encoding an antibody L chain exist on separate vectors or of a type in which both genes exist on the same vector (hereinafter referred to as "tandem type"). In respect of easiness of construction of a humanized antibody expression vector, easiness of introduction into animal cells, and balance between the expression levels of antibody H and L chains in animal cells, a tandem type of the humanized antibody expression vector is more preferred [*J. Immunol. Methods*, 167, 271 (1994)]. The tandem type humanized antibody expression vector includes pKANTEX93 (WO 97/10354), pEE18 [*HYBRIDOMA*, 17, 559 (1998)] and the like.

The thus constructed humanized antibody expression vector can be used for the expression of the human chimeric antibody and human CDR-grafted antibody in animal cells.

### (2) Preparation of cDNA encoding V region of antibody derived from non-human animal

cDNAs encoding VH and VL of an antibody derived from an non-human animal such as a mouse antibody are obtained as follows.

mRNA is extracted from hybridoma cells producing a mouse antibody or the like to synthesize cDNA. The synthesized cDNA is inserted into a vector such as a phage or a plasmid to prepare a cDNA library. Each of a recombinant phage or recombinant plasmid containing cDNA encoding VH and a recombinant phage or recombinant plasmid containing cDNA encoding VL is isolated from the library using a C region part or a V region part of a mouse antibody as the DNA probe.

The full nucleotide sequences of the VH and VL of the mouse antibody of interest on the recombinant phage or recombinant plasmid are determined, and the full amino acid sequences of the VH and VL are deduced from the nucleotide sequences.

The non-human animal may be any animal such as a mouse, a rat, a hamster or a rabbit, so long as a hybridoma cell can be produced therefrom.

The method for preparing total RNA from a hybridoma cell includes a guanidine thiocyanate-cesium trifluoroacetate method [*Methods in Enzymol*., 154, 3 (1987)] and the like. The method for preparing mRNA from total RNA includes an oligo (dT) immobilized cellulose column method [*Molecular Cloning: A Laboratory Manual*, Cold Spring Harbor Lab. Press, New York (1989), hereinafter referred to as "*Molecular Cloning: A Laboratory Manual*"] and the like. Also, a kit for preparing mRNA from a hybridoma cell includes Fast Track mRNA Isolation Kit (manufactured by Invitrogen), Quick Prep mRNA Purification Kit (manufactured by Pharmacia) and the like.

The method for synthesizing cDNA and preparing a cDNA library includes known methods (*Molecular Cloning: A Laboratory Manual*; *Current Protocols in* *Molecular Biology*, Supplement 1-34, hereinafter referred to as "*Current Protocols in Molecular Biology*"); a method using a commercially available kit such as Super Script™ Plasmid System for cDNA Synthesis and Plasmid Cloning (manufactured by GIBCO BRL) or ZAP-cDNA Kit (manufactured by Stratagene); and the like.

The vector into which the cDNA synthesized using mRNA extracted from a hybridoma cell as the template is inserted for preparing a cDNA library may be any vector, so long as the cDNA can be inserted. Examples include ZAP Express [*Strategies*, 5, 58 (1992)], pBluescript II SK(+) [*Nucleic Acids Research*, 17, 9494 (1989)], λzapII (manufactured by Stratagene), λgt10 and λgt11 [*DNA Cloning: A Practical Approach*, I, 49 (1985)], Lambda BlueMid (manufactured by Clontech), λExCell and pT7T3 18U (manufactured by Pharmacia), pcD2 [*Mol. Cell. Biol*., 3, 280 (1983)], pUC18 [*Gene*, 33, 103 (1985)] and the like.

Any *E. coli* for introducing the cDNA library constructed by a phage or plasmid vector can be used, so long as the cDNA library can be introduced, expressed and maintained. Examples include XL1-Blue MRF' [*Strategies*, 5, 81 (1992)], C600 [*Genetics*, 39, 440 (1954)], Y1088 and Y1090 [*Science*, 222, 778 (1983)], NM522 [*J. Mol. Biol*., 166, 1 (1983)], K802 [*J. Mol. Biol*., 16; 118 (1966)], JM105 [*Gene*, 38, 275 (1985)] and the like.

For selecting cDNA clones encoding VH and VL of an antibody derived from a non-human animal in the cDNA library, a colony hybridization or plaque hybridization method using an isotope- or fluorescence-labeled probe can be used (*Molecular Cloning: A Laboratory Manual*). Also, the cDNAs encoding VH and VL can be prepared through polymerase chain reaction (hereinafter referred to as "PCR"; *Molecular Cloning: A Laboratory Manual; Current Protocols in Molecular Biology*) by preparing primers and using cDNA prepared from mRNA or a cDNA library as the template.

The nucleotide sequence of the cDNA can be determined by digesting the cDNA selected by the above method with appropriate restriction enzymes and the like, cloning the fragments into a plasmid such as pBluescript SK(-) (manufactured by Stratagene), carrying out the reaction according to a usually used nucleotide analyzing method such as the dideoxy method of Sanger, F. *et al*. [*Proc. Natl. Acad Sci. USA*, 74, 5463 (1977)], and then analyzing the sequence using an automatic nucleotide sequence analyzer such as A.L.F. DNA sequencer (manufactured by Pharmacia).

Whether the obtained cDNAs encode the full amino acid sequences of the VH and VL of the antibody containing a secretory signal sequence can be confirmed by estimating the full amino acid sequences of the VH and VL from the determined nucleotide sequence and comparing them with full amino acid sequences of the VH and VL of known antibodies [*Sequences of Proteins of Immunological Interest*, US Dept. Health and Human Services (1991), hereinafter referred to as "*Sequences of Proteins of Immunological Interest*"].

### (3) Analysis of amino acid sequence of V region derived from a non-human animal

Regarding the complete amino acid sequences of VH and VL of the antibody comprising a secretory signal sequence, the length of the secretory signal sequence and N-terminal amino acid sequences can be deduced and subgroups to which they belong can also be known, by comparing with full amino acid sequences of VH and VL of known antibodies (*Sequences of Proteins of Immunological Interest*). Also, regarding the amino acid sequence of each CDR of VH and VL, it can be found by comparing it with amino acid sequences of VH and VL of known antibodies (*Sequences of Proteins of Immunological Interest*).

### (4) Construction of human chimeric antibody expression vector

A human chimeric antibody expression vector can be constructed by cloning cDNAs encoding VH and VL of an antibody derived from a non-human animal in the region upstream of genes encoding CH and CL of the human antibody on the humanized antibody expression vector as described in the item 1(1). For example, each of cDNAs encoding VH and VL of an antibody derived from a non-human animal is linked with a synthesized DNA comprising nucleotide sequences at the 3' terminals of VH and VL of an antibody derived from a non-human animal and nucleotide sequences at the 5' terminals of CH and CL of a human antibody and having a recognition sequence of an appropriate restriction enzyme at both terminals, and each cDNA is cloned so that it is appropriately expressed in upstream of genes encoding the CH and CL of the humanized antibody expression vector as described in the item 1(1) to thereby construct a human chimeric antibody expression vector.

### (5) Construction of cDNA encoding V region of human CDR-grafted antibody

cDNAs encoding VH and VL of a human CDR-grafted antibody can be obtained as follows. First, amino acid sequences of FRs in VH and VL of a human antibody to which amino acid sequences of CDRs in VH and VL of an antibody derived from a non-human animal antibody are grafted are selected. Any amino acid sequences of FRs in VH and VL of a human antibody can be used, so long as they are derived from human. Examples include amino acid sequences of FRs in VH and VL of human antibodies registered in database such as Protein Data Bank, amino acid sequences common to subgroups of FRs in the VH and VL of human antibodies (*Sequences of Proteins of Immunological Interest*) and the like. In order to produce a human CDR-grafted antibody having potent activity, amino acid sequences having high homology (at least 60% or more) with amino acid sequence of FRs of VH and VL of an antibody of interest derived from a non-human animal is preferably selected. Then, amino acid sequences of CDRs of VH and VL of the antibody derived from a non-human animal are grafted to the selected amino acid sequences of FRs of VH and VL of a human antibody to design amino acid sequences of the VH and VL of a human CDR-grafted antibody. The designed amino acid sequences are converted to DNA sequences by considering the frequency of codon usage found in nucleotide sequences of genes of antibodies (*Sequence of Proteins of Immunological Interest*), and the DNA sequences encoding the amino acid sequences of the VH and VL of a human CDR-grafted antibody are designed. Several synthetic DNAs having a length of about 100 nucleotides are synthesized, and PCR is carried out using them. In this case, it is preferred in each of the H chain and the L chain that 6 synthetic DNAs are designed in view of the reaction efficiency of PCR and the lengths of DNAs which can be synthesized.

Furthermore, they can be easily cloned into the humanized antibody expression vector constructed in the item 1(1) by introducing the recognition sequence of an appropriate restriction enzyme to the 5' terminal of the synthetic DNAs present on the both terminals. After the PCR, an amplified product is cloned into a plasmid such as pBluescript SK (-) (manufactured by Stratagene), and the nucleotide sequences are determined according to the method described in the item 1(2) to obtain a plasmid having DNA sequences encoding the VH and VL of a designed human CDR-grafted antibody.

### (6) Modification of amino acid sequence of V region of human CDR-grafted antibody

It is known that when a human CDR-grafted antibody is produced by simply grafting only CDRs in VH and VL of an antibody derived from a non-human animal in FRs of the VH and VL of a human antibody, its antigen binding activity is reduced in comparison with that of the original antibody derived from a non-human animal [*BIO*/*TECHNOLOGY*, 9, 266 (1991)]. As the reason, it is considered that several amino acid residues in not only CDRs but also FRs directly or indirectly relate to the antigen binding activity in the VH and VL of the original antibody derived from a non-human animal, and that these amino acid residues are changed to different amino acid residues derived from FRs of the VH and VL of the human antibody, which leads to influence on the antigen binding activity. In order to resolve the problem, in human CDR-grafted antibodies, attempts have been made to identify, among amino acid sequences of the FR of the VH and VL of human antibodies, an amino acid residue which directly relates to binding to the antibody, an amino acid residue which is interacted with an amino acid residue of CDR or an amino acid residue which keeps three-dimensional structure of the antibody and directly relates to its binding to the antigen, and to increase the reduced antigen binding activity by changing them into amino acid residues of the original antibody derived from a non-human animal [*BIO*/*TECHNOLOGY*, 9, 266 (1991)]. In producing a human CDR-grafted antibody, it is the most important point to efficiently identify these FR amino acid residues related to the antigen binding activity, so that construction and analysis of the three-dimensional structure of antibodies have been carried out by X-ray crystallography [*J. Mol. Biol*., 112, 535 (1977)], computer-modeling [*Protein Engineering*, 7, 1501 (1994)] and the like. Although the information of the three-dimensional structure of antibodies has been useful in the production of a human CDR-grafted antibody, no method for producing a human CDR-grafted antibody which can be applied to any antibodies has been established yet. Therefore, various attempts must be currently be necessary, for example, several modified antibodies of each antibody are produced and the relationship between each of the modified antibodies and its antibody binding activity is examined.

Substitution of the amino acid residues of the FR in the VH and VL of a human antibody can be achieved by carrying out the PCR described in the item 1(5) using synthetic DNA for modification. With regard to an amplified product obtained by the PCR, the nucleotide sequence is determined according to the method described in the item 1(2) so that whether the objective modification has been carried out is confirmed.

### (7) Construction of human CDR-grafted antibody expression vector

A human CDR-grafted antibody expression vector can be constructed by cloning cDNAs encoding VH and VL of the human CDR-grafted antibody constructed in the items 1(5) and 1(6) into upstream of the genes encoding CH and CL of the human antibody in the humanized antibody expression vector as described in the item 1(1).

For example, when recognition sites for an appropriate restriction enzymes are introduced to the 5'-terminal of synthetic DNAs positioned at both terminals among synthetic DNAs used in the construction of VH and VL of the human CDR-grafted antibody in the items 1(5) and 1(6), a human CDR-grafted antibody expression vector can be constructed by carrying out cloning so that they are expressed in an appropriate form in upstream of genes encoding CH and CL of the human antibody in the humanized antibody expression vector as described in the item 1(1).

### (8) Transient expression of humanized antibody

In order to efficiently evaluate the antigen binding activity of various humanized antibodies produced, the humanized antibodies can be expressed transiently using the humanized antibody expression vector as described in the items 1(4) and 1(7) or the modified expression vector thereof. Any cell can be used as a host cell, so long as the host cell can express a humanized antibody. Generally, COS-7 cell (ATCC CRL1651) is used in view of its high expression level [*Methods in Nucleic Acids Res*., CRC Press, 283 (1991)]. The method for introducing the expression vector into COS-7 cell includes a DEAE-dextran method [*Methods in Nucleic Acids. Res*., CRC Press, 283 (1991)], a lipofection method [*Proc. Natl. Acad Sci. USA*, 84, 7413 (1987)], and the like.

After introduction of the expression vector, the expression level and antigen binding activity of the humanized antibody in the culture supernatant can be measured by enzyme-linked immunosorbent assay [hereinafter referred to as "ELISA"; *Antibodies: A Laboratory Manual*, Cold Spring Harbor Laboratory, Chapter 14 (1988), hereinafter referred to as "*Antibodies: A Laboratory Manual*", *Monoclonal Antibodies: Principles and Practice*, Academic Press Limited (1996), hereinafter referred to as "*Monoclonal Antibodies*" (1996)] and the like.

### (9) Stable expression of humanized antibody

A transformant which produces a humanized antibody stably can be obtained by introducing into an appropriate host cell the humanized antibody expression vector described in the items 1 (4) and 1 (7).

The method for introducing the expression vector into a host cell includes electroporation [Japanese Published Unexamined Patent Application No. 257891/90, *Cytotechnology*, 3, 133 (1990)] and the like.

Any cell can be used as the host cell into which the humanized antibody expression vector is to be introduced, so long as it can express a humanized antibody. Examples include mouse SP2/0-Ag14 cell (ATCC CRL1581), mouse P3X63-Ag8.653 cell (ATCC CRL1580), CHO cell in which a dihydrofolate reductase gene (hereinafter referred to as "*dhfr*") is defective [*Proc. Natl. Acad Sci. U.S.A*., 77, 4216 (1980)], rat YB2/3HL.P2.G11.16Ag.20 cell (ATCC CRL1662, hereinafter referred to as "YB2/0 cell"), and the like.

A host cell which expresses an antibody having high ADCC activity includes host cells in which activity of an enzyme protein relating to synthesis of an intracellular sugar nucleotide, GDP-fucose, an enzyme protein relating to modification of a sugar chain in which 1-position of fucose is bound to 6-position of N-acetylglucosamine in the reducing end through α-bond in a complex N-glycoside-linked sugar chain, or a protein relating to transport of an intracellular sugar nucleotide, GDP-fucose to the Golgi body or the like is decreased or deleted, preferably YB2/0 cell, and the like.

After introduction of the expression vector, a transformant which expresses a humanized antibody stably is selected in accordance with the method disclosed in Japanese Published Unexamined Patent Application No. 257891/90, by culturing it in a medium for animal cell culture containing an agent such as G418 sulfate (hereinafter referred to as "G418", manufactured by SIGMA). The medium for animal cell culture includes PRMI1640 medium (manufactured by Nissui Pharmaceutical), GIT medium (manufactured by Nihon Pharmaceutical), EX-CELL302 medium (manufactured by JRH), IMDM medium (manufactured by GIBCO BRL), Hybridoma-SFM medium (manufactured by GIBCO BRL), media obtained by adding various additives such as fetal bovine serum (hereinafter referred to as "FBS") to these media, and the like. The resulting transformant is cultured in a medium to thereby form and accumulate the humanized antibody in the culture supernatant. The expression level and antigen binding activity of the humanized antibody in the culture supernatant can be measured by ELISA or the like. Also, in the transformant, the expression level of the humanized antibody can be increased by using the *dhfr* amplification system or the like according to the method disclosed in Japanese Published Unexamined Patent Application No. 257891/90.

The humanized antibody can be purified from the culture supernatant of the transformant by using a protein A column [*Antibodies, A Laboratory Manual, Monoclonal Antibodies* (1996)]. Any other conventional methods for protein purification can be used. For example, the humanized antibody can be purified by a combination of gel filtration, ion-exchange chromatography, ultrafiltration and the like. The molecular weight of the H chain or the L chain of the purified humanized antibody or the antibody molecule as a whole is determined by polyacrylamide gel electrophoresis [hereinafter referred to as "SDS-PAGE", *Nature*, 227, 680 (1970)], Western blotting [*Antibodies: A Laboratory Mamial, Monoclonal Antibodies* (1996)], and the like.

### (10) Evaluation of activity of humanized antibody

The binding activity to an antigen and the binding activity to a cultured cancer cell line of the purified humanized antibody can be measured by ELISA, an immunofluorescent method [*Cancer Immunol. Immunother*., 36, 373 (1993)] and the like. The cytotoxic activity against an antigen positive culture cell line can be evaluated by measuring the CDC activity, the ADCC activity or the like [*Cancer Immunol. Immunother*., 36, 373 (1993)].

### BRIEF DESCRIPTION OF THE DRAWINGS

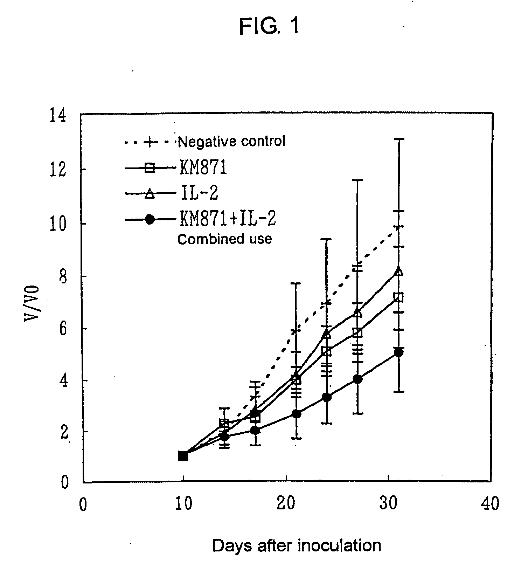

Fig. 1 shows antitumor effects by combined use of KM871 with IL-2 using a xenograft model. The abscissa and the ordinate represent the number of days after H-187 cell inoculation and V/V0, respectively. The dotted line, "□", "Δ" and "●" show a negative control group, a KM871 administration group, an IL-2 administration group, and a combined administration group of KM871 and IL-2, respectively

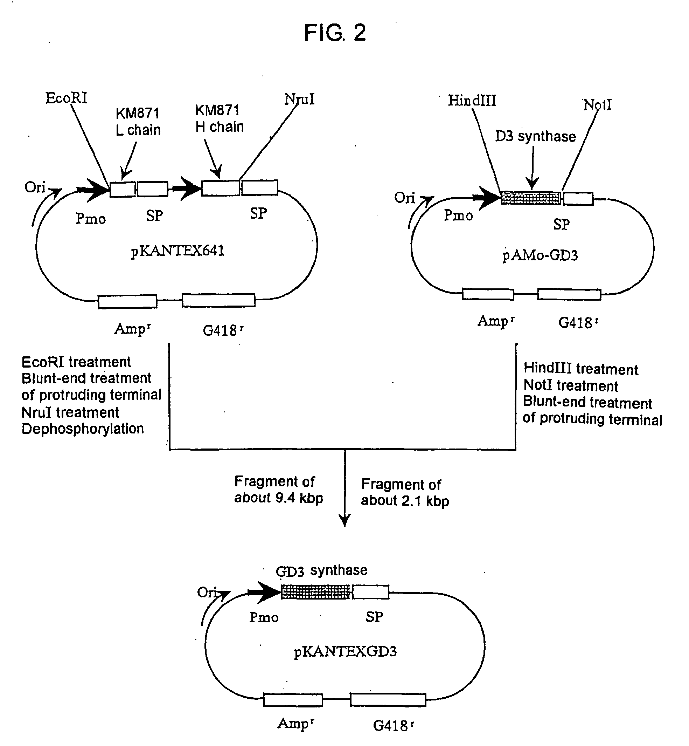

Fig. 2 shows construction steps of a GD3 synthase expression vector pKANTEXGD3.

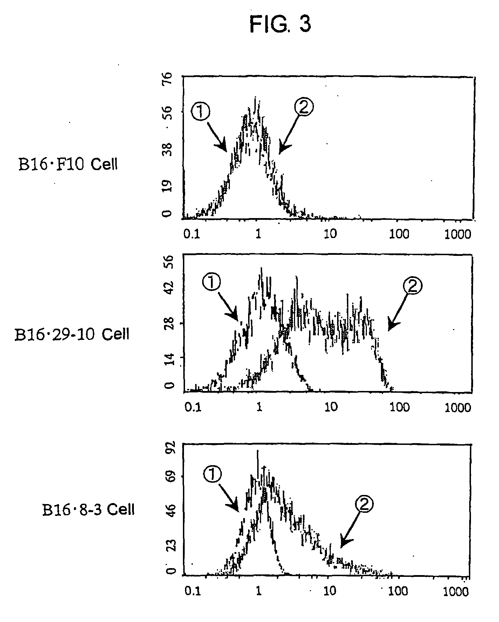

Fig. 3 shows results of analysis of GD3 expression quantity of GD3 synthase transfectants of B16·29-10 cell and B16·8-3 cell by fluorescent antibody techniques. The abscissa and the ordinate represent fluorescence intensity and the number of cells, respectively. ① and ② show staining histogram by human IgG and staining histogram by KM871, respectively.

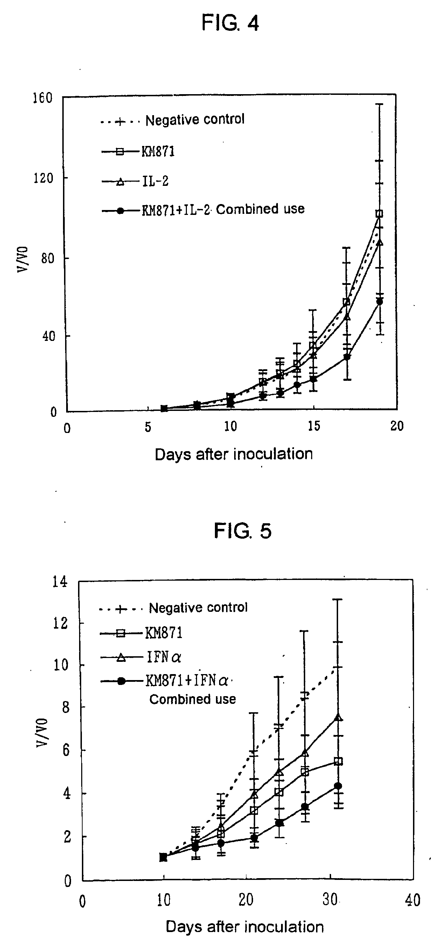

Fig. 4 shows antitumor effects by combined use of KM871 with IL-2 using a syngeneic graft model. The abscissa and the ordinate represent the number of days after B16·29-10 cell inoculation and V/V0, respectively. The dotted line, "□", "Δ" and "●" show a negative control group, a KM871 administration group, an IL-2 administration group, and a combined administration group of KM871 and IL-2, respectively.
Fig. 5 shows antitumor effects by combined use of KM871 with IFNα using a xenograft model. The abscissa and the ordinate represent the number of days after H-187 cell inoculation and V/V0, respectively. The dotted line, "□", "Δ" and "●" show a negative control group, a KM871 administration group, an IFNα administration group, and a combined administration group of KM871 and IFNα, respectively.

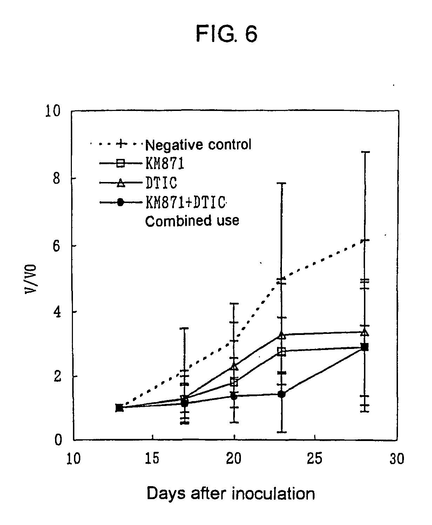

Fig. 6 shows antitumor effects by combined use of KM871 with dacarbazine using a xenograft model. The abscissa and the ordinate represent the number of days after G-361 cell inoculation and V/V0, respectively. The dotted line, "□", "Δ" and "●" show a negative control group, a KM871 administration group, a dacarbazine administration group and a combined administration group of KM871, and dacarbazine, respectively.

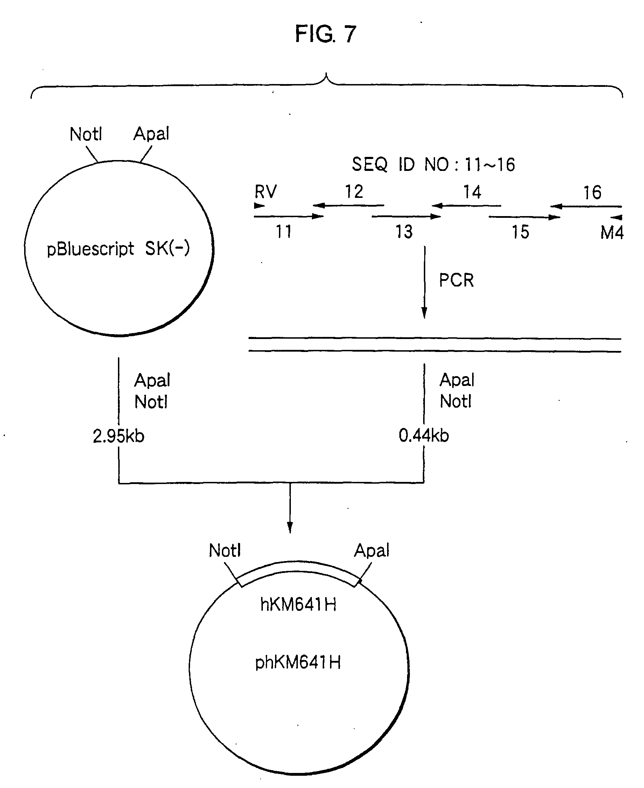

Fig. 7 shows construction steps of plasmid phKM641H.

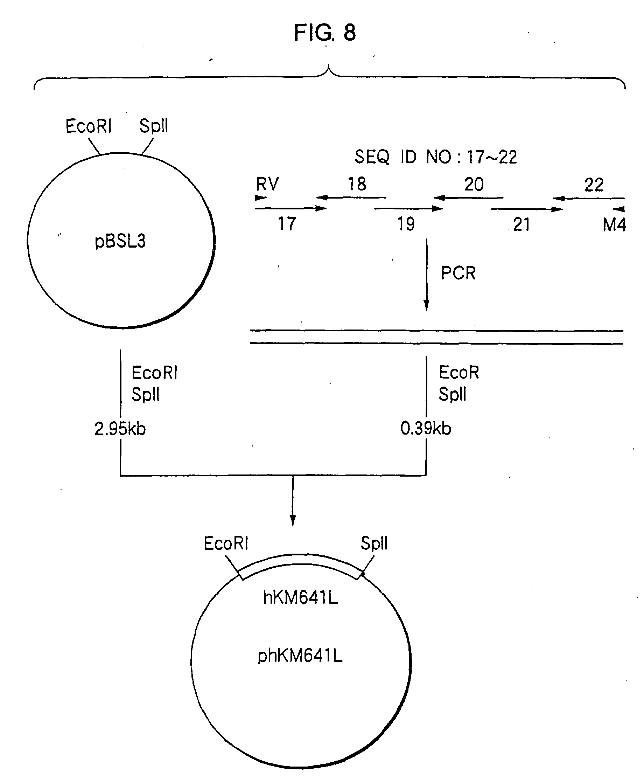

Fig. 8 shows construction steps of plasmid phKM641L.

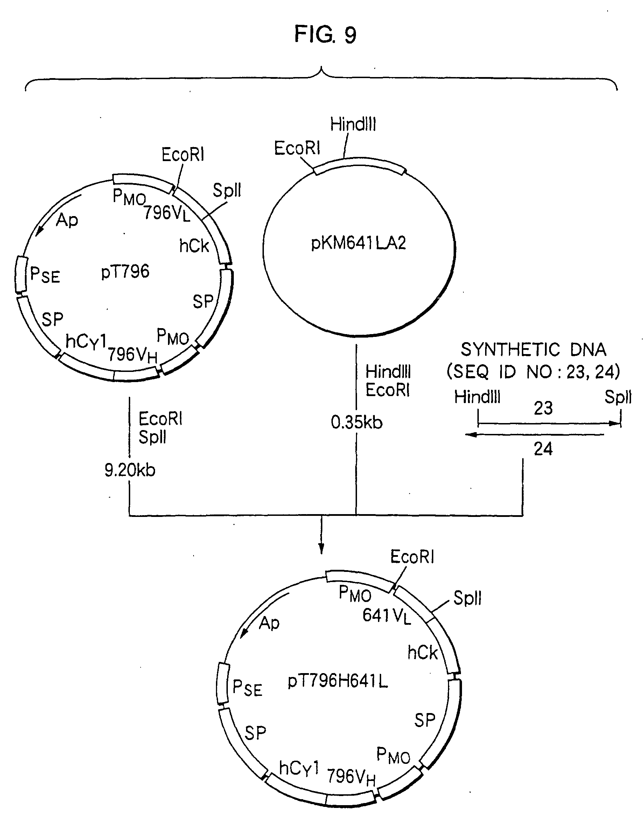

Fig. 9 shows construction steps of plasmid pT796H641L.

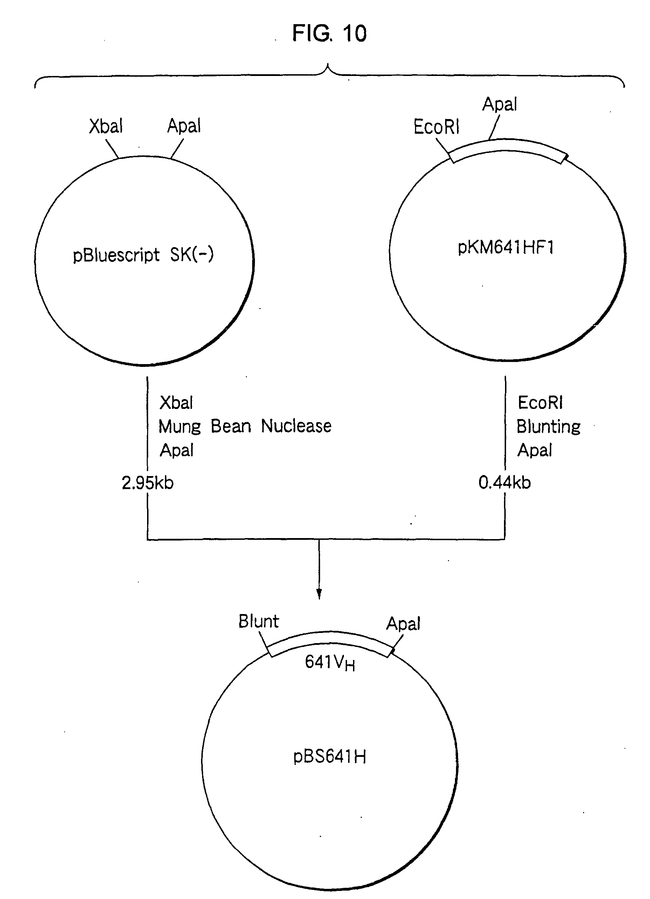

Fig. 10 shows construction steps of plasmid pBS641H.

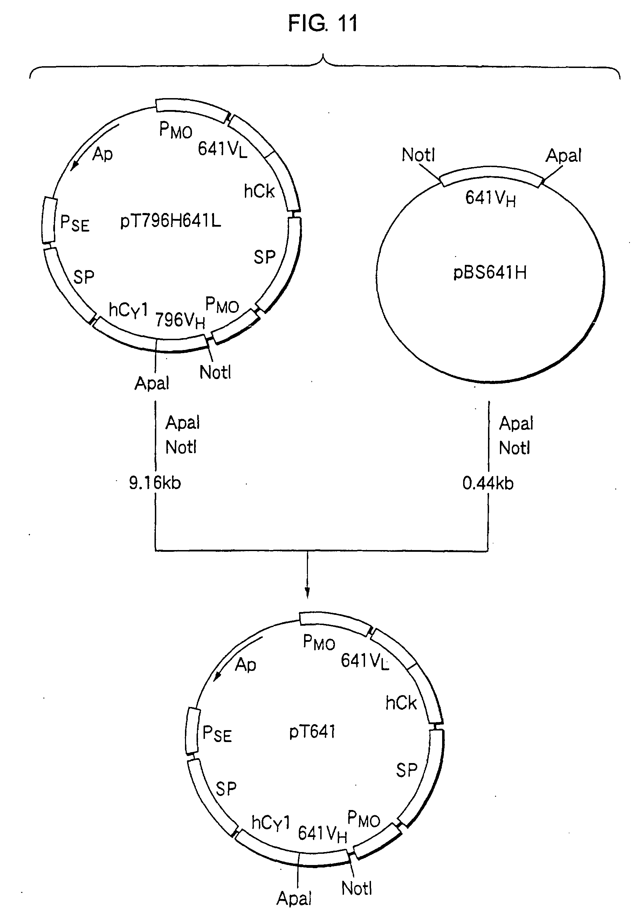

Fig. 11 shows construction steps of plasmid pT641.

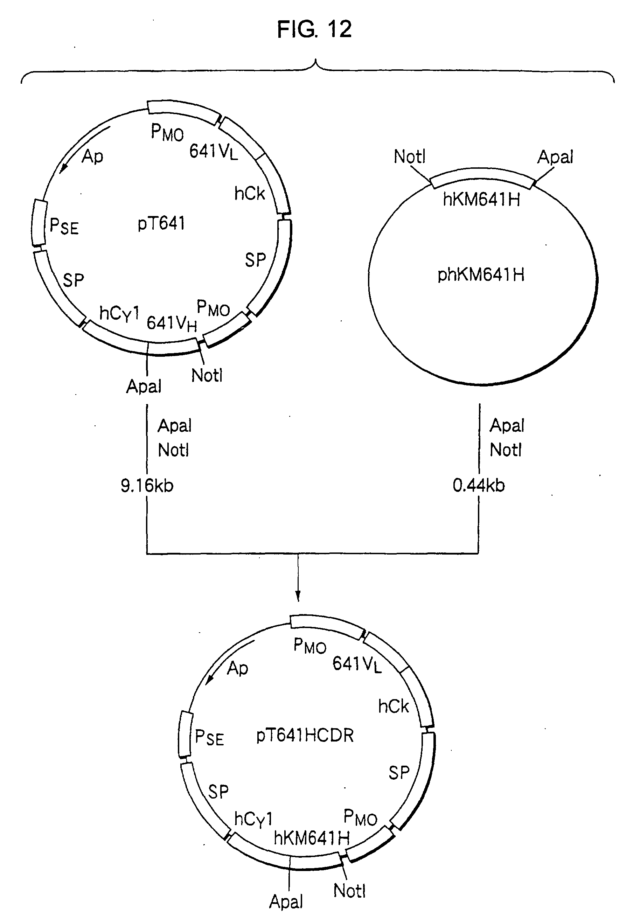

Fig. 12 shows construction steps of plasmid pT641HCDR.

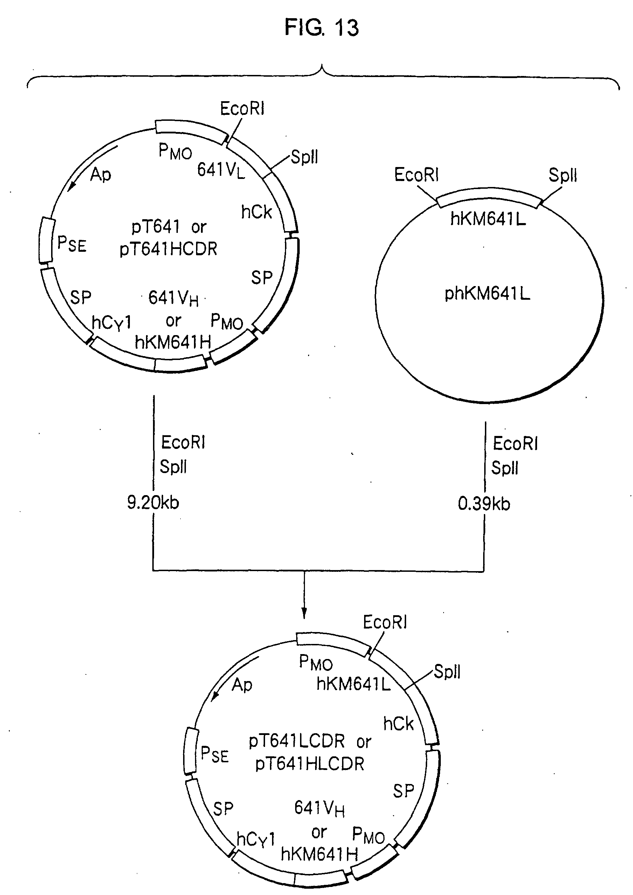

Fig. 13 shows construction steps of plasmids pT641LCDR and pT641HLCDR.

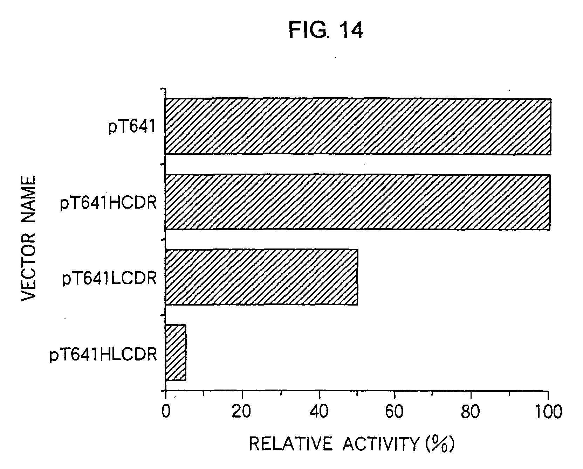

Fig. 14 shows activity evaluation of an anti-GD3 chimeric antibody and an anti-GD3 CDR-grafted antibody by their transient expression using plasmids pT641, pT641HCDR, pT641LCDR and pT641HLCDR. The ordinate and the abscissa represent the expression vector name and the relative activity (%) when the activity of anti-GD3 chimeric antibody is defined as 100, respectively.

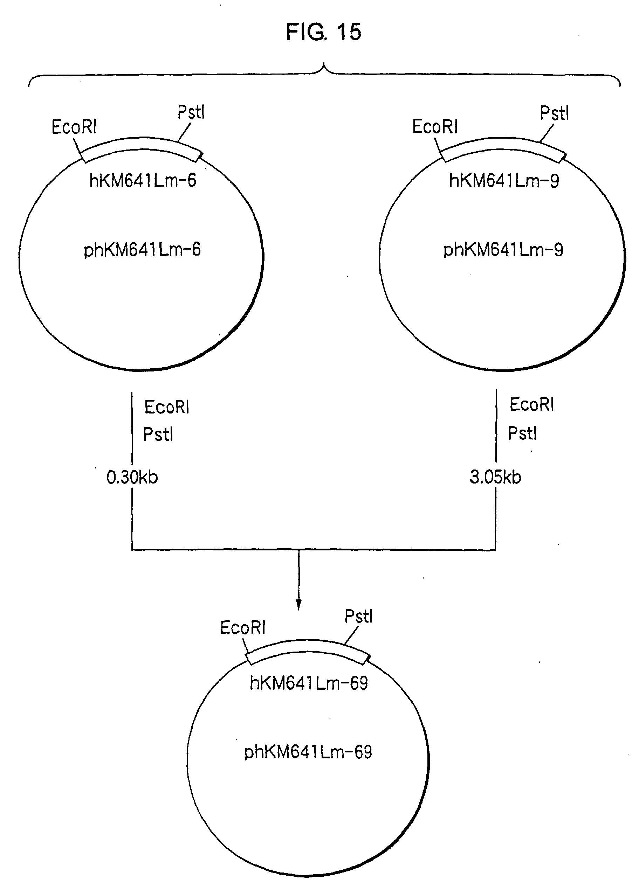

Fig. 15 shows construction steps of plasmid phKM641Lm-69.

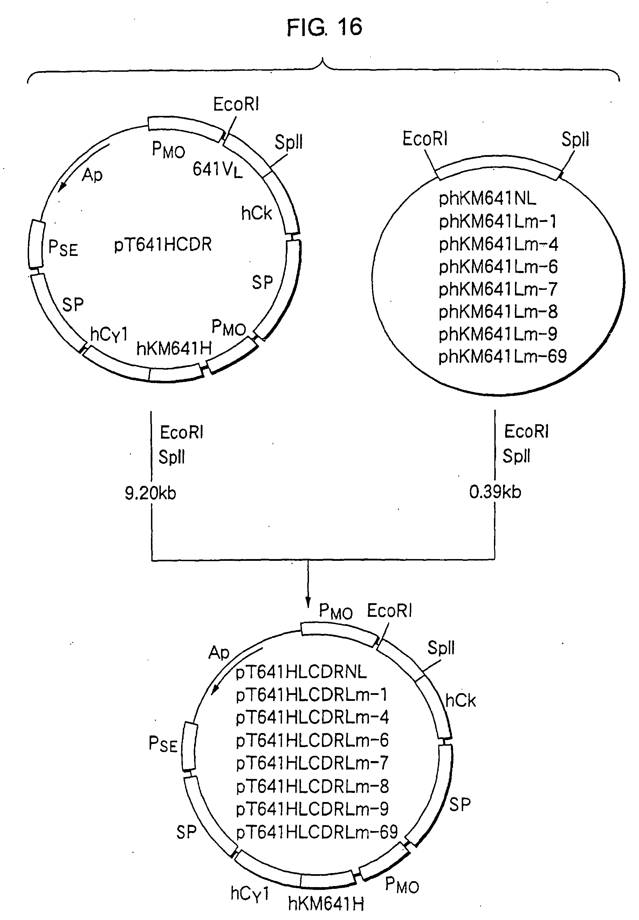

Fig. 16 shows construction steps of plasmids pT641HLCDRNL, pT641HLCDRLm-1, pT641HLCDRLm-4, pT641HLCDRLm-6, pT641HLCDRLm-7, pT641HLCDRLm-8, pT641HLCDRLm-9 and pT641HLCDRLm-69.

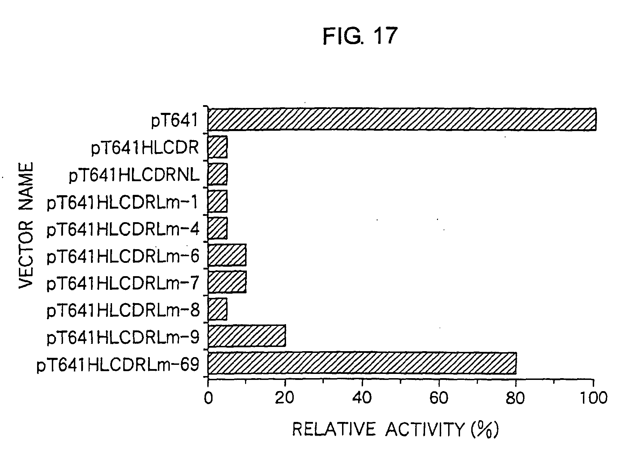

Fig. 17 shows activity evaluation of anti-GD3 chimeric antibody and anti-GD3 CDR-grafted antibody by their transient expression using plasmids pT641, pT641HLCDR, pT641HLCDRNL, pT641HLCDRLm-1, pT641HLCDRLm-4, pT641HLCDRLm-6, pT641HLCDRLm-7, pT641HLCDRLm-8, pT641HLCDRLm-9 and pT641HLCDRLm-69. The ordinate and the abscissa represent the expression vector name and the relative activity (%) when the activity of anti-GD3 chimeric antibody is defined as 100, respectively.

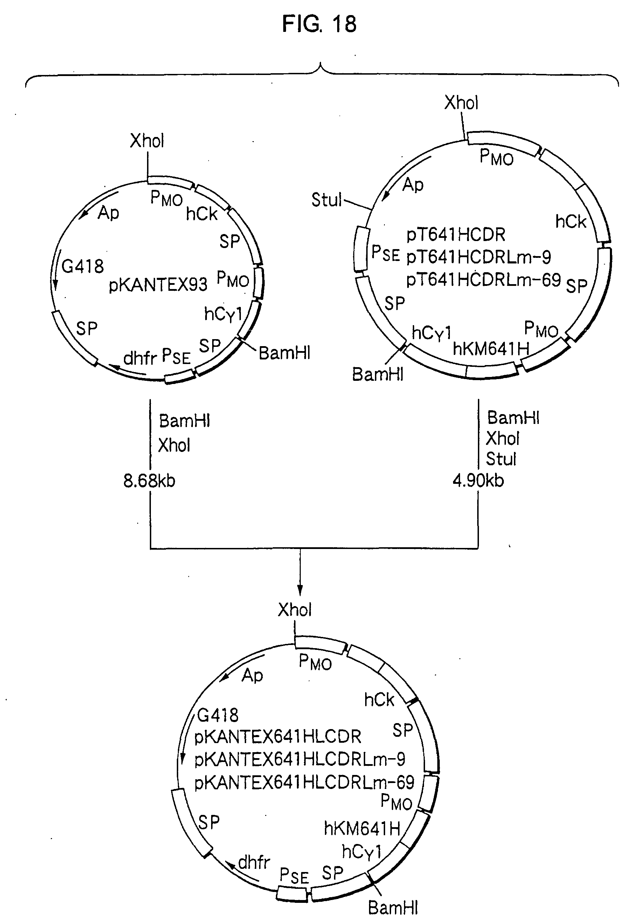

Fig. 18 shows construction steps of plasmids pKANTEX641HLCDR, pKANTEX641HLCDRLm-9 and pKANTEX641HLCDRLm-69.

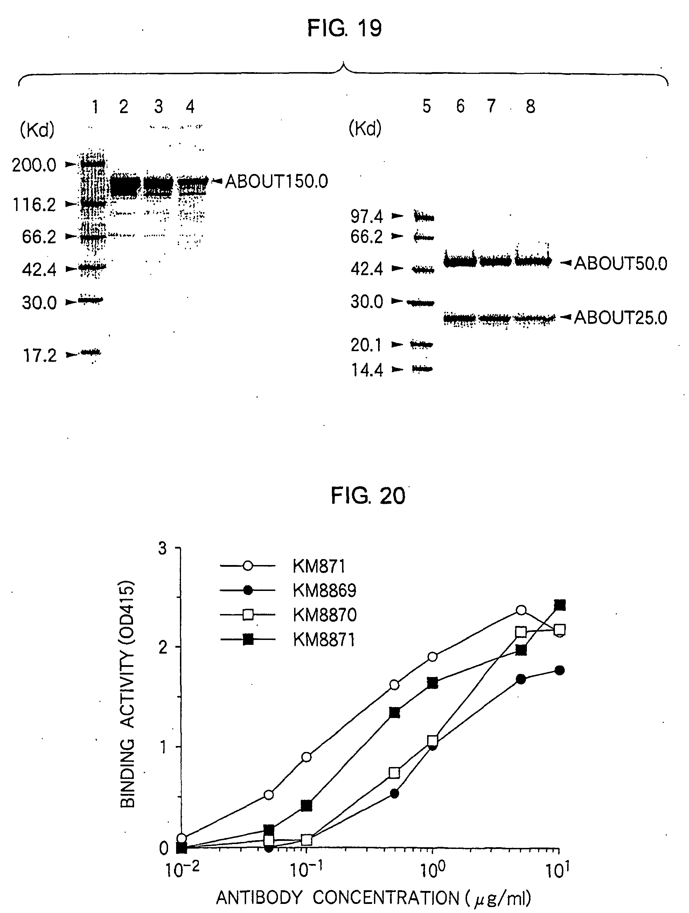

Fig. 19 shows SDS-PAGE (using a 4 to 15% gradient gel) electrophoresis patterns of purified anti-GD3 CDR-grafted antibodies KM8869, KM8870 and KM8871. The left side shows results of electrophoresis carried out under non-reducing conditions, and the right side shows those under reducing conditions. Lanes 1, 2, 3, 4, 5, 6, 7 and 8 show electrophoresis patterns of high molecular weight markers, KM8869, KM8870, KM8871, low molecular weight markers, KM8869, KM8870 and KM8871, respectively.
Fig. 20 shows binding activities of purified anti-GD3 chimeric antibody KM871 and purified anti-GD3 CDR-grafted antibodies KM8869, KM8870 and KM8871 to GD3 measured by changing the antibody concentration. The ordinate and the abscissa represent the binding activity to GD3 and the antibody concentration, respectively. "○", "●", "□" and "■" show the activities of KM871, KM8869, KM8870 and KM8871, respectively.

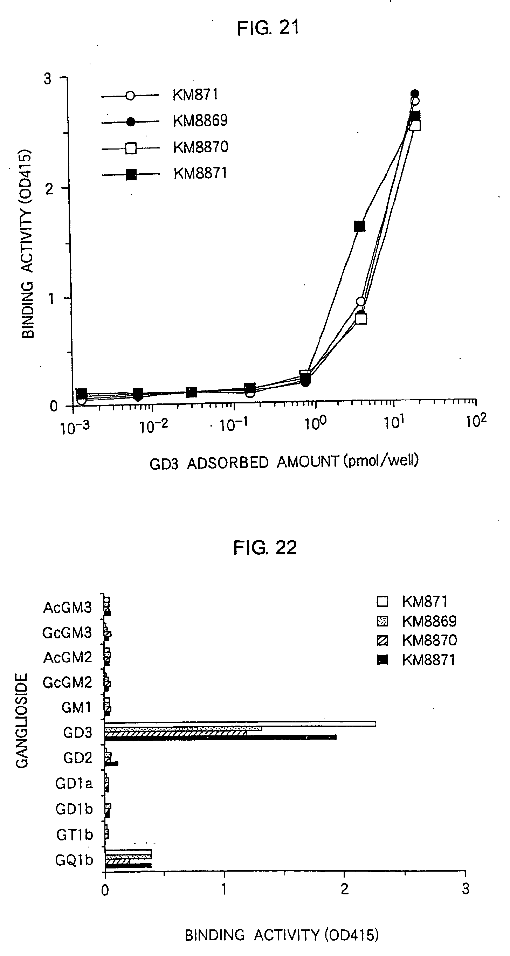

Fig. 21 shows binding activities of purified anti-GD3 chimeric antibody KM871 and purified anti-GD3 CDR-grafted antibodies KM8869, KM8870 and KM8871 to GD3 measured by changing an amount of GD3 to be adsorbed to a plate. The ordinate and the abscissa represent the binding activity to GD3 and the amount of GD3 adsorbed to the plate, respectively. "○", "●", "□" and "■" show the activities of KM871, KM8869, KM8870 and KM8871, respectively.
Fig. 22 shows reactivities of purified anti-GD3 chimeric antibody KM871 and purified anti-GD3 CDR-grafted antibodies KM8869, KM8870 and KM8871 with various gangliosides. The ordinate and the abscissa represent the kind of ganglioside and the binding activity, respectively. AcGM2, GcGM2, AcGM3 and GcGM3 show N-acetylGM2, N-glycolylGM2, N-acetylGM3 and N-glycolylGM3. "□", dotted "□", shaded "□" and "■" show the reactivities of KM871, KM8869, KM8870 and KM8871, respectively.

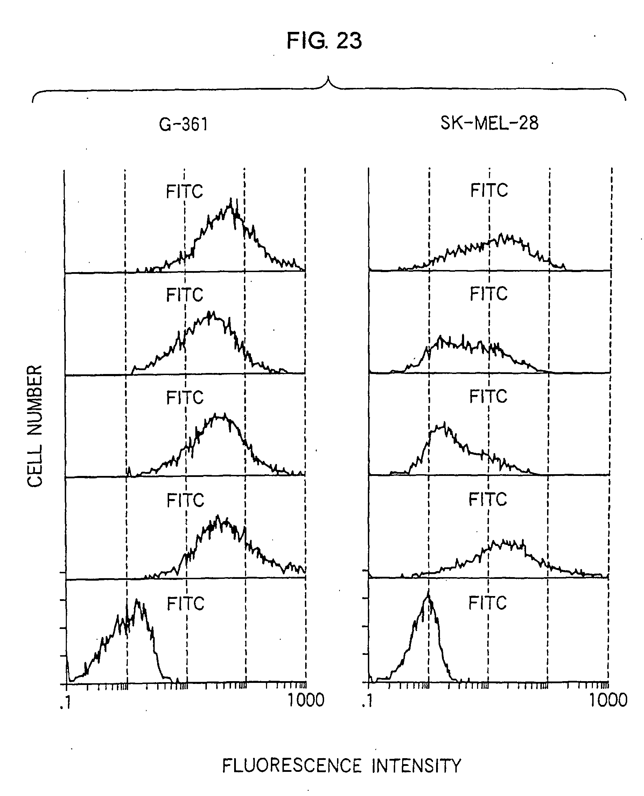

Fig. 23 shows reactivities of purified anti-GD3 chimeric antibody KM871 and purified anti-GD3 CDR-grafted antibodies KM8869, KM8870 and KM8871 with human melanoma cell lines G-361 and SK-MEL-28. The ordinate and the abscissa represent the number of cells and the fluorescence intensity, respectively. The drawings show reactivities of control, KM871, KM8869, KM8870 and KM8871, respectively, from the bottom.

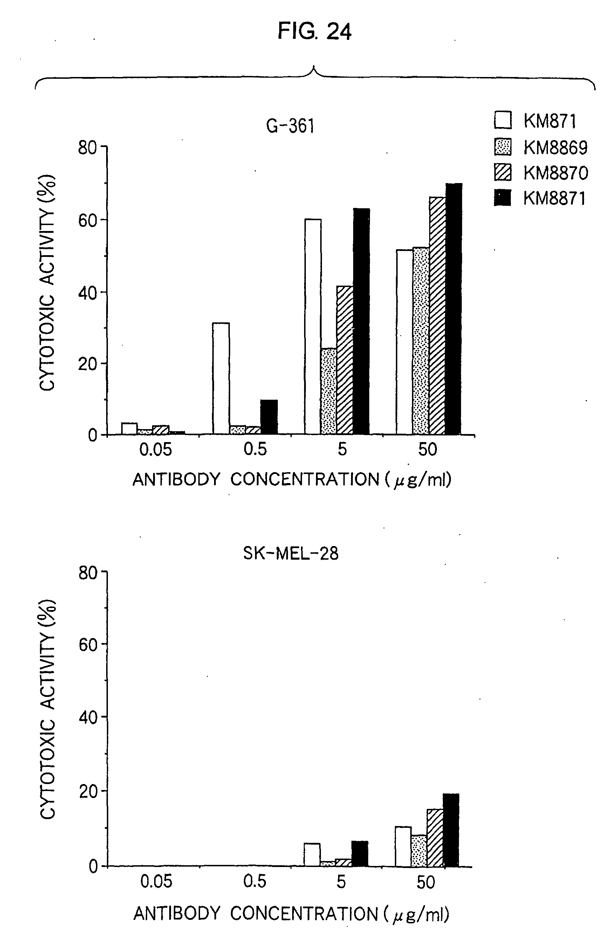

Fig. 24 shows CDC activities of purified anti-GD3 chimeric antibody KM871 and purified anti-GD3 CDR-grafted antibodies KM8869, KM8870 and KM8871 against human melanoma cell lines G-361 and SK-MEL-28. The ordinate and the abscissa represent the cytotoxic activity and the antibody concentration, respectively. "□", dotted "□", shaded "□" and "■" show reactivities of KM871, KM8869, KM8870 and KM8871, respectively.

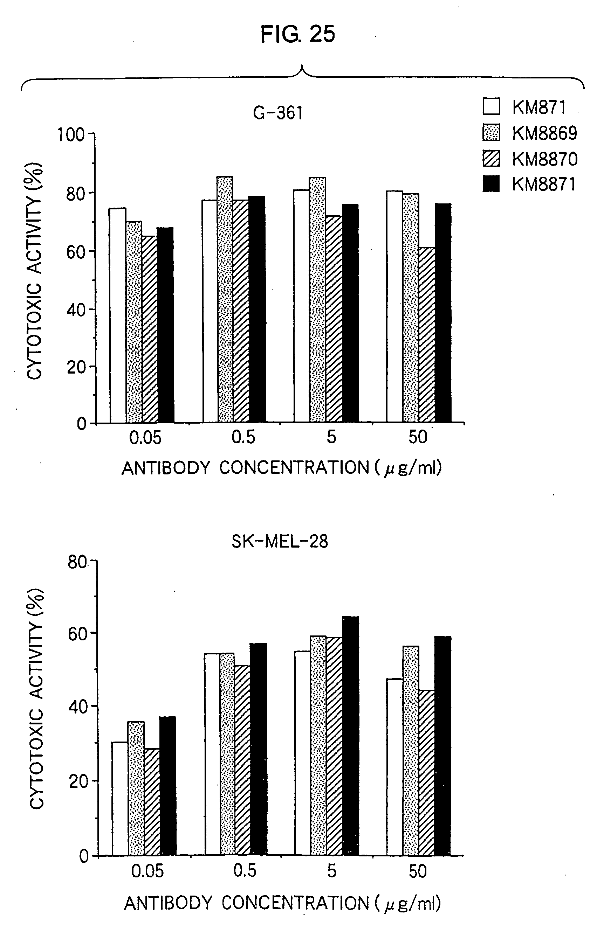

Fig. 25 shows ADCC activities of purified anti-GD3 chimeric antibody KM871 and purified anti-GD3 CDR-grafted antibodies KM8869, KM8870 and KM8871 against human melanoma cell lines G-361 and SK-MEL-28. The ordinate and the abscissa represent the cytotoxic activity and the antibody concentration, respectively. "□", dotted "□", shaded "□" and "■" show reactivities of KM871, KM8869, KM8870 and KM8871, respectively.

### BEST MODE FOR CARRYING OUT THE INVENTION

### Example 1

### Measurement of antitumor effects by combined administration of anti-GD3 human chimeric antibody KM871 with human IL-2:

### (1) Measurement of antitumor effects by combined administration of KM871 with human IL-2 using a mouse xenograft model

A 2 to 3 mm square tumor section prepared from a tumor mass of a GD3-positive human malignant myeloma cell line H-187 which had been passaged under the dorsal skin of a Balb/c nude mice (male, CLEA Japan) was inoculated using a inoculation needle under the dorsal skin of a 6-week-old male nude mouse. Ten days after the inoculation, the tumor diameter was measured using slide calipers and the tumor volume was calculated by the following equation:$\text{Tumor volume = width × length × height × 0.5}$

Individuals having a tumor volume of around 100 mm³ were selected and divided into groups in such a manner that the average tumor volume became uniform, and then the following administration groups of A to D were arranged.
A. Negative control group:
   No administration
B. KM871 alone group:
   Single administration of 800 µg/200 µl per animal on the 14th day after the inoculation
C. IL-2 (Immunase 35, manufactured by Shionogi & Co., Ltd.) alone group:
   Five days of continuous administration (on the 10th to 14th days after inoculation), once a day at a dose of 1×10⁵ JRU/400 µl/day per animal
D. KM871 + IL-2 combined administration group:
   For IL-2, five days of continuous administration (on the 10th to 14th days after inoculation), once a day at a dose of 1×10⁵ JRU/400 µl/day per animal; for KM871, single administration of 800 µg/200 µl per animal on the 14th day after the inoculation

The test was carried out using 6 animals for each group. Each agent was prepared by diluting with saline (manufactured by Otsuka Pharmaceutical) and administered from the tail vein. From the 10th day after the inoculation, the tumor volume was periodically measured, and the antitumor effects were judged by comparing the mean value of specific volume V/V0 in each group. The calculation method of V/V0 is shown in the following equation:$\text{V/V0 = (tumor volume of each individual on the measured day)} \text{/(tumor volume of each individual on the measurement starting day)}$

Results of periodical changes in the mean value of V/V0 in each group are shown in Fig. 1. As shown in Fig. 1, the combined administration of KM871 with IL-2 showed higher growth inhibitory effects than the administration of IL-2 alone or the antibody alone. Further, in order to show that the combined use of KM871 with IL-2 shows more significant effects than the individual single agents, a significance test was carried out between the value of V/V0 in the combined administration group and the values of V/V0 in other groups, and the results of obtained p values are shown in Table 1.

**Table 1**

| Day | 14 | 17 | 21 | 24 | 27 | 31 |
|---|---|---|---|---|---|---|
| Combined use vs negative control | 0.318 | **0.00233** | **0.003** | **0.00701** | **0.0118** | **0.00822** |
| Combined use vs KM871 | 0.0981 | 0.0629 | **0.0144** | **0.0107** | **0.0295** | 0.0622 |
| Combined use vs IL-2 | 0.548 | 0.0966 | **0.0179** | **0.00273** | **0.012** | **0.0181** |
| (Day: the number of days after inoculation, bold figures: p < 0.05) | | | | | | |

As shown in Table 1, the combined administration group showed significant tumor growth inhibitory effects on and after the 17th day of the inoculation, in comparison with the negative control group. In addition, significant growth inhibitory effects were found during from the 21st day to the 27th day for the antibody alone, and on and after the 21st day for the IL-2 alone. Based on the above, it was found that significantly higher tumor growth inhibitory effects can be obtained by the combined administration of KM871 with IL-2 in comparison with the individual single agents. Also, a two-tailed t-test was carried out for the judgment of significance, and p < 0.05 was used as the standard of significance judgment.

### (2) Measurement of antitumor effects by combined administration of KM871 with human IL-2 using a mouse syngeneic graft model

Among various effector cells relating to the antitumor immunity, NK cell and T cell are known as the main cell population to be activated by IL-2. Accordingly, in order to examine antitumor effects by combined administration of IL-2 with the antibody, a syngeneic graft model was constructed for inoculating a mouse-derived tumor cell into a normal mouse having no T cell defection. Since there is no available mouse-derived tumor cell line capable of expressing GD3, a cell was prepared by constructing an animal cell stable expression vector for a GD3 synthase which catalyses the reaction of synthesizing GD3 by adding sialic acid to a GD3 biosynthesis precursor GM3, and introducing the vector into a mouse melanoma B16-F10 cell capable of expressing GM3. Antitumor effects by the combined administration of KM871 with human IL-2 were measured with the syngeneic graft model constructed using the cell. Details are shown below.

### a. Construction of GD3 synthase gene stable expression vector

A schematic illustration regarding the method for constructing a stable expression vector is shown in Fig. 2. To 10 µl of a buffer comprising 10 mM Tris-HCl (pH 7.5), 50 mM sodium chloride, 10 mM magnesium chloride and 1 mM DTT, 3 µg of a vector pAMo-GD3 (WO 94/23020) for expressing GD3 synthase gene in animal cells was added, and 10 units of a restriction enzyme *Hin*dIII (manufactured by Takara Shuzo) was further added thereto to carry out the reaction at 37°C for 1 hour. The reaction solution was precipitated with ethanol and added to 10 µl of a buffer comprising 50 mM Tris-HCl (pH 7.5), 100 mM sodium chloride, 10 mM magnesium chloride, 1 mM DTT, 100 µg/ml BSA and 0.01% Triton X-100, and 10 units of a restriction enzyme *Not*I (manufactured by Takara Shuzo) was added thereto to carry out the reaction at 37°C for 1 hour. The reaction solution was precipitated with ethanol, and the 5'-protruding-end formed by the restriction enzyme digestion was changed into blunt-end using DNA Blunting Kit (manufactured by Takara Shuzo). The reaction solution was fractionated by agarose gel electrophoresis to recover about 2 µg of a blunt-ended *Hin*dIII-*Not*I fragment having about 2.1 kb using QIAquick Gel Extraction Kit (manufactured by QIAGEN) in accordance with the manufacture's instructions.

Next, 3 µg of an anti-GD3 human chimeric antibody KM871 expression vector pKANTEX641 (Japanese Published Unexamined Patent Application No. 304989/93) was added to 10 µl of a buffer comprising 50 mM Tris-HCl (pH 7.5), 100 mM sodium chloride, 10 mM magnesium chloride and 1 mM DTT, and 10 units of a restriction enzyme *Eco*RI (manufactured by Takara Shuzo) was further added thereto to carry out the reaction at 37°C for 1 hour. The reaction solution was precipitated with ethanol, and the 5'-protruding-end formed by the restriction enzyme digestion was changed into blunt-end using DNA Blunting Kit (manufactured by Takara Shuzo). The reaction solution was precipitated with ethanol and dissolved in 10 µl of a buffer comprising 50 mM Tris-HCl (pH 7.5), 100 mM sodium chloride, 10 mM magnesium chloride and 1 mM DTT, and 10 units of a restriction enzyme *Nru*I (manufactured by Takara Shuzo) was further added thereto to carry out the reaction at 37°C for 1 hour. The reaction solution was precipitated with ethanol, 10 µl of a buffer comprising 50 mM Tris-HCl (pH 7.5) and 10 mM magnesium chloride were added thereto, and 10 units of a 5'-terminal dephosphorylation enzyme, calf intestine-derived alkaline phosphatase (manufactured by Takara Shuzo), was further added thereto to carry out the reaction at 37°C for 1 hour for dephosphorylation of the 5'-terminal. The reaction solution was treated with phenol and then fractionated by agarose gel electrophoresis to recover about 2 µg of a blunt end-dephosphorylated *Eco*RI-*Nru*I fragment having about 9.4 kb using QIAquick Gel Extraction Kit (manufactured by QIAGEN) in accordance with the manufacture's instructions.

Next, 0.1 µg of the thus obtained *Eco*RI-*Nru*I fragment derived from the plasmid pKANTEX641 and 0.1 µg of the *Hin*dIII-*Not*I fragment derived from the plasmid pAMoGD3 were added to 10 µl in total volume of sterile water and ligated using DNA Ligation Kit Ver. 2 (manufactured by Takara Shuzo) in accordance with the manufacture's instructions. Using the thus obtained recombinant plasmid DNA solution, *Escherichia coli* DH5α was transformed to obtain GD3 synthase stable expression vector pKANTEXGD3 shown in Fig. 2.

### b. Introduction of GD3 synthase expression vector into B16 cell

To B16-F10 cell (ATCC CRL 1581) suspended at a density of 4×10⁶ cells/200 µl with PBS (manufactured by Gibco BRL), 4 µg of the GD3 synthase gene stable expression vector pKANTEXGD3 obtained in a. of the item (2) of Example 1 was added, and the gene was introduced by electroporation using Gene Pulsar II (manufactured by Bio Rad). The selection was carried out finally by G418 (0.5 mg/ml) to obtain transformed cell clones B16-8-3 and B16-29-10. RPMI 1640 medium (manufactured by Gibco BRL) containing 10% immobilized fetal bovine serum (manufactured by Gibco BRL) was used for culturing the cells *in vitro*.

### c. Analysis of GD3 expression of GD3 expressing cell line (fluorescent antibody technique)

Each of 1 ×10⁶ cells of the GD3 synthase gene transfectants B16-8-3 and B16-29-10 obtained in b. of the item (2) of Example 1 and the parent cell line B16-F10 cell were suspended in PBS, put into a microtube (manufactured by Treff) and centrifuged (2,000 rpm for 2 minutes) for washing the cells, 50 µl of the anti-GD3 human chimeric antibody KM871 (a solution prepared by adjusting to 5 µg/ml with 1% BSA-PBS, negative control was 1% BSA-PBS alone) was added thereto, followed by stirring, and the reaction was carried out at 4°C for 1 hour. After the reaction and subsequent washing with PBS by centrifugation three times, 100 µl of biotin-labeled goat anti-human IgG (H + L) solution (manufactured by VECTOR, used by diluting 400 folds with 1% BSA-PBS) was added thereto, followed by stirring, and the reaction was carried out at 4°C for 1 hour. After washing with PBS by centrifugation three times, 100 µl of a streptoavidin-labeled Cy5 pigment solution (Streptoavidin-RED670 manufactured by GIBCO, used by diluting 100 folds with 1% BSA-PBS) was added thereto, followed by stirring, and the reaction was carried out at 4°C for 30 minutes. After the reaction and subsequent washing with PBS by centrifugation three times, the cells were again suspended in PBS to carry out the analysis using a flow cytometer EPICS Elite (manufactured by COULTER). The results are shown in Fig. 3. As shown in Fig. 3, the anti-GD3 human chimeric antibody KM871 reacted with only B16-8-3 and B16-29-10 but did not show reactivity with the parent cell line B16-F10 cell. Since the above results showed that both of the transfectants B16-8-3 and B16-29-10 expressed GD3 on the surface, an animal model for measuring *in vivo* antitumor effects of KM871 was prepared by inoculating them into C57 BL/6 mice as the origin of B16 cell.

### d. Measurement of antitumor effects by combined administration of KM871 with human IL-2 using a homologous tumor model

The GD3 expressing B16 cell B16·29-10 obtained in c. of the item (2) of Example 1 was suspended in PBS to give a density of 1×10⁸ cells/ml, and 50 µl of the suspension was inoculated under the abdominal side skin of a C57 BL/6 mouse (male, 7-week-old, manufactured by Charles River). Six days after the tumor inoculation, the tumor diameter was measured using slide calipers to calculate the tumor volume by the following equation, and individuals having the value within the range of 10 to 80 mm³ were selected.${\text{Tumor volume = (width)}}^{\text{2}} \text{× length × 0.5}$

They were divided into groups in such a manner that the average tumor volume became uniform, and then the following administration groups of A to D were arranged to carry out continuous administration of the following agents from the tail vein once a day for 8 days (from the 6th day to 14th day).
A. Negative control group:
   No administration
B. KM871 alone group:
   50 µg/day
C. IL-2 (Human recombinant IL-2, manufactured by Peprotech) alone group:
   10 µg/day
D. KM871 + IL-2 combined administration group:
   50 µg/day + 10 µg/day

The test was carried out using 5 animals for each group. Each agent was prepared to 200 µl per animal by diluting with a citrate buffer (an aqueous solution of 10 mM citric acid and 150 mM sodium chloride, adjusted to pH 6 with sodium hydroxide). From the 6th day after the inoculation, the tumor volume was periodically measured, and the antitumor effects were judged by comparing the mean value of specific volume V/V0 in each group. Results of periodical changes in the mean value of V/V0 in each group are shown in Fig. 4. As shown in Fig. 4, the antitumor effects were not found by IL-2 alone or the antibody alone, but the combined administration of KM871 with IL-2 showed growth inhibitory effects.

Further, in order to show that the combined use of KM871 with IL-2 shows more significant effects than the individual single agents, a significance test was carried out between the value of V/V0 in the combined administration group and the values of V/V0 in other groups, and the results of obtained p values are shown in Table 2.

**Table 2**

| Day | 8 | 10 | 12 | 13 | 14 | 15 | 17 | 19 |
|---|---|---|---|---|---|---|---|---|
| Combined use vs negative control | **0.0347** | **0.0392** | **0.0293** | **0.0323** | **0.0455** | **0.0282** | **0.021** | **0.0399** |
| Combined use vs KM871 | **0.036** | **0.00107** | **0.0206** | **0.0151** | **0.0333** | 0.0507 | **0.0378** | 0.0822 |
| Combined use vs IL-2 | **0.00459** | **0.000868** | **0.000624** | **0.00199** | **0.00326** | **0.00222** | **0.00188** | **0.00355** |
| (Day: the number of days after inoculation, bold figures: p < 0.05) | | | | | | | | |

As shown in Table 2, the combined administration group showed significant tumor growth inhibitory effects on and after the 8th day of the inoculation, in comparison with the negative control group. In addition, significant growth inhibitory effects were found on the 8th, 10th, 12th, 13th, 14th and 17th days for the antibody alone, and on and after the 8th day for the IL-2 alone. Based on the above, it was found that significantly higher tumor growth inhibitory effects can be obtained by the combined administration of KM871 with IL-2, in comparison with the individual single agents, in the syngeneic graft model. Also, a two-tailed t-test was carried out for the judgment of significance, and p < 0.05 was used as the standard of significance judgment.

### Example 2

### Measurement of antitumor effects by combined administration of anti-GD3 human chimeric antibody KM871 and human IFNα:

A 2 to 3 mm square tumor section prepared from a tumor mass of a GD3-positive human malignant myeloma cell line H-187 which had been passaged under the dorsal skin of a Balb/c nude mouse (male, manufactured by CLEA Japan) was inoculated using a inoculation needle under the dorsal skin of a 6-week-old male nude mouse. Ten days after the inoculation, the tumor diameter was measured using slide calipers and the tumor volume was calculated using the above equation.

Individuals having a tumor volume of around 100 mm³ were selected and divided into groups in such a manner that the average tumor volume became uniform, and then the following administration groups of A to D were arranged.
A. Negative control group:
   No administration
B. KM871 alone group:
   Single administration of 800 µg/200 µl per animal on the 10th day after the inoculation
C. IFNα (Intron A Injection, manufactured by Schering-Plough Corp.) alone group:
   Five days of continuous administration (on the 10th to 14th days after inoculation), once a day at a dose of 2×10⁵ IU/400 µl/day per animal
D. KM871 + IFNα combined administration group:
   For IFNα, five days of continuous administration (on the 10th to 14th days after inoculation), once a day at a dose of 12×10⁵ IU/400 µl/day per animal; for KM871, 800 µg per animal on the 10th day after the inoculation

The test was carried out using 6 animals for each group. Each agent was prepared by diluting with saline (manufactured by Otsuka Pharmaceutical) and administered from the tail vein. From the 10th day after the inoculation, the tumor volume was periodically measured, and the antitumor effects were judged by comparing the mean value of specific volume V/V0 in each group.

Results of periodical changes in the mean value of V/V0 in each group are shown in Fig. 5. As shown in Fig. 5, the combined administration of KM871 with IFNα showed further higher growth inhibitory effects than the administration of IFNα alone or the antibody alone.

In addition, in order to show that the combined use of KM871 with IFNα shows more significant effects than their single administration, a two-tailed t-test was carried out between the value of V/V0 in the combined administration group and the values of V/V0 in other groups, and the results of obtained p values are shown in Table 3.

**Table 3**

| Day | 14 | 17 | 21 | 24 | 27 | 31 |
|---|---|---|---|---|---|---|
| Combined use vs. negative control | **0.0425** | **0.00033** | **0.000341** | **0.00168** | **0.00358** | **0.00243** |
| Combined use vs. KM871 | 0.583 | 0.335 | 0.071 | 0.0658 | 0.0611 | 0.232 |
| Combined use vs. IFNα | 0.529 | 0.189 | **0.0235** | **0.0294** | 0.06 | 0.0596 |
| (Day: the number of days after inoculation, bold figures: p < 0.05) | | | | | | |

As shown in Table 3, the combined administration group showed significant tumor growth inhibitory effects on and after the 14th day of the inoculation, in comparison with the negative control group. In addition, significant difference was found on the 21 st and 24th days for the IFNα alone.

Since significant difference was not found in the above results between the combined administration group and the antibody single administration group, a significance test was carried out between the negative control group and the antibody alone group or the combined administration group, with the results of obtained p values shown in Table 4.

**Table 4**

| Day | 14 | 17 | 21 | 24 | 27 | 31 |
|---|---|---|---|---|---|---|
| Negative control vs. KM871 | 0.195 | **0.0117** | **0.0151** | **0.0302** | **0.0417** | **0.0162** |
| Negative control vs. combined use | **0.0425** | **0.00033** | **0.000341** | **0.00168** | **0.00358** | **0.00243** |
| (Day: the number of days after inoculation, bold figures: p < 0.05) | | | | | | |

As shown in Table 4, the combined administration group showed significant difference in the growth inhibitory effects against the negative control in all of the measuring days on and after the 14th day, while significant difference was not obtained on the 14th day in the antibody alone group. Based on the above, it was found that significantly higher tumor growth inhibitory effects can be obtained by the combined administration of KM871 with IFNα in comparison with the individual single agents. Also, a two-tailed t-test was carried out for the judgment of significance, and p < 0.05 was used as the standard of significance judgment.

### Example 3

### Antitumor effects by the combined administration of anti-GD3 human chimeric antibody KM871 with dacarbazine:

A GD3-positive human malignant melanoma cell line G-361 (ATCC CRL-1424) which had been cultured *in vitro* using McCoy's 5A medium (manufactured by Gibco BRL) containing 10% immobilized fetal bovine serum (manufactured by Gibco BRL) was suspended in phosphate buffered saline (manufactured by Gibco BRL) to give a density of 1×10⁸ cells/ml, and 50 µl of the suspension was inoculated under the abdominal side skin of a Balb/c nude mouse (male, 7-week-old, manufactured by CLEA Japan). Thirteen days after the tumor inoculation, the tumor diameter was measured using slide calipers to calculate the tumor volume by the above equation.

Individuals having the tumor volume within the range of 5:0 to 60 mm³ were selected and divided into groups in such a manner that the average tumor volume became uniform, and then the following administration groups of A to D were arranged.
A. Negative control group:
   No administration
B. KM871 alone group:
   Single administration of 800 µg/200 µl per animal on the 13th day after the inoculation
C. Dacarbazine (Dacarbazine Injection, manufactured by Kyowa Hakko Kogyo) alone group:
   Five days of continuous administration (on the 13th to 17th days after inoculation), once a day at a dose of 50 mg/kg/day
D. KM871 + Dacarbazine combined administration group:
   For dacarbazine, five days of continuous administration (on the 13th to 17th days after inoculation), once a day at a dose of 50 mg/kg/day per animal; for KM871, 800 µg per animal on the 13th day after the inoculation

The test was carried out using 5 animals for each group. Each agent was prepared by diluting with saline (manufactured by Otsuka Pharmaceutical) and administered from the tail vein. From the 13th day after the inoculation, the tumor volume was periodically measured, and the antitumor effects were judged by comparing a mean value of specific volume V/V0 in each group.

Results of periodical changes in the mean value of V/V0 in each group are shown in Fig. 6. As shown in Fig. 6, the combined administration of KM871 with dacarbazine showed higher growth inhibitory effects than the dacarbazine alone or the antibody alone.

Further, in order to clarify the effects of the combined use of KM871 with dacarbazine, a significance test was carried out between the value of V/V0 in the combined administration group and the values of V/V0 in other groups, and the results of obtained p values are shown in Table 5.

**Table 5**

| Day | 4 | 7 | 10 | 15 |
|---|---|---|---|---|
| Combined use vs. negative control | 0.112 | **0.0114** | **0.0238** | **0.0289** |
| Combined use vs. KM871 | 0.753 | 0.422 | 0.0963 | 0.994 |
| Combined use vs. DTIC | 0.626 | 0.214 | 0.0703 | 0.681 |
| (Day: the number of days after inoculation, bold figures: p < 0.05) | | | | |

As shown in Table 5, the combined administration group showed significant tumor growth inhibitory effects on and after the 7th day of the inoculation, in comparison with the negative control group. However, since significant difference was not found between the combined administration group and the antibody alone group or dacarbazine alone group, a significance test was carried out between the negative control group and each of other groups, and the results of obtained p values are shown in Table 6.

**Table 6**

| Day | 4 | 7 | 10 | 15 |
|---|---|---|---|---|
| Negative control vs. KM871 | 0.167 | **0.0298** | 0.101 | **0.0252** |
| Negative control vs. DTIC | 0.145 | 0.198 | 0.205 | **0.0367** |
| Negative control vs. combined use | 0.112 | **0.0114** | **0.0238** | **0.0289** |
| (Day: the number of days after inoculation, bold figures: p < 0.05) | | | | |

As shown in Table 6, the combined administration group showed significant difference against the negative control in all of the measuring days on and after the 7th day, whereas significant difference was obtained only on the 7th and 15th days regarding the antibody alone group and on the 15th day regarding the dacarbazine alone group. Based on the above, it was found that significantly higher tumor growth inhibitory effects can be obtained by the combined administration of KM871 with dacarbazine in comparison with the individual single agents. Also, a two-tailed t-test was carried out for the judgment of significance, and p < 0.05 was used as the standard of significance judgment.

### Reference Example

### Production of anti-GD3 CDR-grafted antibody:

### 1. Analysis of amino acid sequence of V region of anti-GD3 mouse antibody KM641

A full amino acid sequence of VH of anti-GD3 mouse antibody KM641 disclosed in Japanese Published Unexamined Patent Application No. 304989/93 is represented by SEQ ID NO:1, and a full amino acid sequence of VL thereof is represented by SEQ ID NO:2. Based on the comparison of both sequences with analytical results of amino acid sequences of known antibodies (*Sequences of Proteins of Immunological Interest*) and N-terminal amino acid sequences of H chain and L chain of purified anti-GD3 mouse antibody KM641, it was confirmed that positions -19 to -1 in the amino acid sequence of H chain and positions -20 to -1 in the amino acid sequence of L chain are. Full amino acid sequences of the secretory VH and VL are represented by SEQ ID NOs:49 and 50, respectively. Also, it was found that CDRs 1, 2 and 3 of VH have the amino acid sequences represented by SEQ ID NOs:3, 4 and 5, and CDRs 1, 2 and 3 of VL have those represented by SEQ ID NOs:6, 7 and 8.

### 2. Measurement of binding activity of antibody to various ganglioside (ELISA)

The binding activity of antibodies to various gangliosides was measured as follows.

Each ganglioside (2 nmol) was dissolved in 2 ml of ethanol solution containing 10 µg of dipalmitoylphosphatidylcholine (manufactured by SIGMA) and 5 µg of cholesterol (manufactured by SIGMA). In each well of a 96 well plate for ELISA (manufactured by Greiner), 20 µl of the solution (becomes 20 pmol/well) or 20 µl of the solution diluted with ethanol was respectively dispensed, followed by airdrying, and then PBS containing 1% BSA (hereinafter referred to as "1% BSA-PBS") was added in 100 µl/well and allowed to react at room temperature for 1 hour to thereby block remaining active groups. After discarding 1% BSA-PBS, culture supernatants of transformants or diluted solutions of humanized antibodies were added in 50 µl/well and allowed to react at room temperature for 1 hour. After the reaction, each well was washed with PBS containing 0.05% Tween 20 (hereinafter referred to as "Tween-PBS") and then a peroxidase-labeled goat anti-human IgG(γ) antibody solution (manufactured by Kirkegaard & Perry Laboratories) diluted 1,000 folds with 1% BSA-PBS was added as a secondary antibody solution in 50 µl/well and allowed to react at room temperature for 1 hour. After the reaction and subsequent washing with Tween-PBS, an ABTS substrate solution (a solution prepared by dissolving 0.55 g of 2,2'-azinobis(3-ethylbenzothiazoline-6-sulfonic acid) in 1 L of 0.1 M citrate buffer (pH 4.2) and adding 1 µl/ml hydrogen peroxide just before the use) was added in 50 µl/well for color development and the absorbance at 415 nm (hereinafter referred to as "OD415") was measured.

### 3. Construction of cDNAs encoding VH and VL of anti-GD3 CDR-grafted antibody

### (1) Design of amino acid sequences of VH and VL of anti-GD3 CDR-grafted antibody

First, an amino acid sequence of VH of an anti-GD3 CDR-grafted antibody was designed as follows. In order to graft the CDR amino acid sequences of VH of the anti-GD3 mouse antibody KM641 identified in the item 1 of Reference Example, an FR amino acid sequence of VH of a human antibody was selected. Kabat *et al*. have classified the VH of various known human antibodies into three subgroups (HSG I to III) based on the homology of the amino acid sequences and then reported consensus sequences among respective subgroups (*Sequences of Proteins of Immunological Interest*). Since these consensus sequences have a possibility that the immunogenicity is reduced in human, it was decided to design a VH amino acid sequence of an anti-GD3 CDR-grafted antibody based on these consensus sequences. In order to produce an anti-GD3 CDR-grafted antibody having higher activity in designing it, it was decided to select an FR amino acid sequence having the highest homology with the FR amino acid sequence of VH of KM641, among FR amino acid sequences of consensus sequences of the three subgroups of VH of human antibodies. Results of the homology search are shown in Table 7. As shown in Table 7, the FR amino acid sequence of VH of KM641 showed the highest homology with the subgroup III.

**Table 7**

| Homology between FR amino acid sequence of consensus sequence of each subgroup of human antibody H chain V region and FR amino acid sequence of H chain V region of KM641 | | |
|---|---|---|
| HSG I | HSG II | HSG III |
| 62.1% | 56.3% | 78.2% |

Based on the above results, an amino acid sequence HV.0 of VH of the anti-GD3 CDR-grafted antibody was designed by grafting the CDR amino acid sequence of VH of the anti-GD3 mouse antibody KM641 to an appropriate position of the amino acid sequence of FR of the consensus sequence of subgroup III of VH of the human antibody.

Next, an amino acid sequence of VL of an anti-GD3 CDR-grafted antibody was designed as follows. In order to graft the CDR amino acid sequence of VL of the anti-GD3 mouse antibody KM641 identified in the item 1 of Reference Example, an FR amino acid sequence of VL of a human antibody was selected. Kabat *et al*. have classified the VL of various known human antibodies into four subgroups (HSG I to IV) based on the homology of the amino acid sequences and then reported consensus sequences among respective subgroups (*Sequences of Proteins of Immunological Interest*). Accordingly, similar to the H chain, an FR amino acid sequence having the highest homology with the FR amino acid sequence of VL of KM641 was selected from FR amino acid sequences of consensus sequences of the four subgroups of VL of human antibodies. Results of the homology search are shown in Table 8. As shown in Table 8, the FR amino acid sequence of VL of KM641 showed the highest homology with the subgroup I.

**Table 8**

| Homology between FR amino acid sequence of consensus sequence of each subgroup of human antibody L chain V region and FR amino acid sequence of L chain V region of KM641 | | | |
|---|---|---|---|
| HSG I | HSG II | HSG III | HSG IV |
| 76.2% | 60.0% | 62.5% | 67.5% |

Based on the above results, an amino acid sequence LV.0 of VL of the anti-GD3 CDR-grafted antibody was designed by grafting the CDR amino acid sequence of VL of the anti-GD3 mouse antibody KM641 to an appropriate position of the amino acid sequence of FR of the consensus sequence of subgroup I of VL of the human antibody.

The thus designed amino acid sequence HV.0 of VH and amino acid sequence LV.0 of VL of the anti-GD3 CDR-grafted antibody are sequences in which only the CDR amino acid sequence of the anti-GD3 mouse antibody is grafted to the FR amino acid sequence of the selected human antibody. In general, in the human CDR-grafted antibodies, the antigen binding activity is reduced in many cases by grafting of a mouse antibody CDR amino acid sequence alone. Thus, in order to avoid the reduction of the antigen binding activity of antibodies, among FR amino acid residues different between a human antibody and a mouse antibody, amino acid residues considered to have influences on the antigen binding activity are grafted together with a CDR amino acid sequence. Accordingly, FR amino acid residues considered to have influences on the activity were identified in this Reference Example.

First, a three-dimensional structure of an antibody V region comprised of the amino acid sequence HV.0 of VH and amino acid sequence LV.0 of VL of anti-GD3 CDR-grafted antibody designed in the above (hereinafter referred to as "HV0LV0") was constructed using computer-modeling. The three-dimensional structure coordinates was carried out using a software AbM (manufactured by Oxford Molecular) and the display of three-dimensional structure was carried out using a software Pro-Explore (manufactured by Oxford Molecular) in accordance with the respective manufacture's instructions. Also, a computer model of the three-dimensional structure of the V region of anti-GD3 mouse antibody KM641 was constructed in the same manner. In addition, a three-dimensional structure model of a modified HV0LV0 having an amino acid sequence in which at least one amino acid residue different from anti-GD3 mouse antibody KM641 in the FR amino acid sequences of VH and VL of HV0LV0 was substituted by the amino acid residues of positions corresponding to the anti-GD3 mouse antibody KM641 in order was also constructed. Three-dimensional structures of V regions of the anti-GD3 mouse antibody KM641, HV0LV0 and modified product were compared. As a result, amino acid residues considered to have influences on the antigen binding activity by changing three-dimensional structure of the antigen-binding region were selected from the FR amino acid residues of HV0LV0. As a result of substituting the thus selected FR amino acid residues of HV0LV0 by the amino acid residues found in the mouse antibody KM641, an amino acid sequence hKM641H of VH of the anti-GD3 CDR-grafted antibody represented by SEQ ID NO:9 and an amino acid sequence hKM641L of VL of the anti-GD3 CDR-grafted antibody represented by SEQ ID NO:10 were designed. In the hKM641H, Gly at position 10, Leu at position 11, Leu at position 20, Thr at position 28, Asn at position 84, Thr at position 91, Tyr at position 95, Ala at position 97 and Val at position 115 in the FR amino acid sequence of HV.0 were replaced by Asp, Phe, Val, Ala, Arg, Ser, Phe, Thr and Leu, respectively, as amino acid residues of positions corresponding to the VH of anti-GD3 mouse antibody KM641. In the hKM641L, Tyr at posiition 49, Ser at position 65 and Phe at position 71 in the FR amino acid sequence of LV.0 were replaced by Tyr, Ser and Phe, respectively, as amino acid residues of positions corresponding to the VL of anti-GD3 mouse antibody KM641.

### (2) Construction of cDNA encoding VH of anti-GD3 CDR-grafted antibody

A cDNA encoding the anti-GD3 CDR-grafted antibody VH amino acid sequence hKM641H designed in the item 3(1) of Reference Example was constructed as follows.

First, the designed amino acid sequence was ligated with the secretory signal sequence of H chain of anti-GD3 mouse antibody KM641 represented by SEQ ID NO:1 to produce a full antibody amino acid sequence. Next, the amino acid sequence is converted into genetic codons. When two or more genetic codons were present for one amino acid residue, corresponding genetic codon was determined by taking the frequency of codon usage found in nucleotide sequences of antibody genes into consideration (*Sequences of Proteins of Immunological Interest*). A nucleotide sequence of cDNA encoding the complete antibody V region amino acid sequence was designed by ligating the thus determined genetic codons, and nucleotide sequences of primer binding site for the PCR amplification (including restriction enzyme recognition sequences for cloning into a humanized antibody expression vector) were added to the 5'-terminal and 3'-terminal. The thus designed nucleotide sequence was divided into a total of 6 nucleotide sequences from the 5'-terminal side, each having about 100 bases (adjoining nucleotide sequences are designed such that the termini have an overlapping sequence of about 20 bases), and they were synthesized in alternating order of the sense chain and the antisense chain using an automatic DNA synthesizer (380A, manufactured by Applied Biosystems).

Specifically, 6 synthetic DNA fragments of SEQ ID NOs:11 to 16 were synthesized. Each DNA was added to 50 µl of a buffer comprising 10 mM Tris-HCl (pH 8.3), 50 mM potassium chloride, 1.5 mM magnesium chloride, 0.001% gelatin, 200 µM dNTPs, 0.5 µM M13 primer RV (manufactured by Takara Shuzo), 0.5 µM M13 primer M4 (manufactured by Takara Shuzo) and 2 units of TaKaRa Taq DNA polymerase (manufactured by Takara Shuzo) to give a final concentration of 0.1 µM, and the solution was covered with 50 µl of mineral oil and set to a DNA thermal cycler (PJ480, manufactured by PERKIN ELMER) to carry out the reaction of 30 cycles of heating at 94°C for 2 minutes, at 55°C for 2 minutes and at 72°C for 2 minutes as one cycle. The reaction solution was purified using QIAquick PCR Purification Kit (manufactured by QIAGEN) in accordance with the manufacture's instructions and made into 30 µl of a buffer comprising 10 mM Tris-HCl (pH 7.5), 10 mM magnesium chloride and 1 mM DTT, and 10 units of a restriction enzyme *Apa*I (manufactured by Takara Shuzo) was further added thereto to carry out the reaction at 37°C for 1 hour. The reaction solution was precipitated with ethanol, added to 10 µl of a buffer comprising 50 mM Tris-HCl (pH 7.5), 100 mM sodium chloride, 10 mM magnesium chloride, 1 mM DTT, 100 µg/ml BSA and 0.01% Triton X-100, and 10 units of a restriction enzyme *Not*I (manufactured by Takara Shuzo) was further added to carry out the reaction at 37°C for 1 hour. The reaction solution was fractionated by agarose gel electrophoresis to recover about 0.2 µg of an *Apa*I-*Not*I fragment having about 0.44 kb using QIAquick Gel Extraction Kit (manufactured by QIAGEN) in accordance with the manufacture's instructions.

Next, 3 µg of a plasmid pBluescript SK(-) was added to 10 µl of a buffer comprising 10 mM Tris-HCl (pH 7.5), 10 mM magnesium chloride and 1 mM DTT, and 10 units of a restriction enzyme *Apa*I (manufactured by Takara Shuzo) was further added thereto to carry out the reaction at 37°C for 1 hour. The reaction solution was precipitated with ethanol and added to 10 µl of a buffer comprising 50 mM Tris-HCl (pH 7.5), 100 mM sodium chloride, 10 mM magnesium chloride, 1 mM DTT, 100 µg/ml BSA and 0.01% Triton X-100, and 10 units of a restriction enzyme *Not*I (manufactured by Takara Shuzo) was further added thereto to carry out the reaction at 37°C for 1 hour. The reaction solution was fractionated by agarose gel electrophoresis to recover about 2 µg of an *Apa*I-*Not*I fragment having about 2.95 kb using QIAquick Gel Extraction Kit (manufactured by QIAGEN) in accordance with the manufacture's instructions.

Next, 0.1 µg of the *Apa*I-*Not*I fragment of the PCR product of VH of anti-GD3 CDR-grafted antibody and 0.1 µg of the *Apa*I-*Not*I fragment of the plasmid pBluescript SK(-), both obtained in the above, were added to 20 µl in total volume of sterile water and ligated using Ready-To-Go T4 DNA Ligase (manufactured by Pharmacia). Using the thus obtained recombinant plasmid DNA solution, an *Escherichia coli* line HB101 was transformed. Each plasmid DNA was produced from 10 clones of the transformants, allowed to react in accordance with the manufacture's instructions attached to AutoRead Sequencing Kit (manufactured by Pharmacia) and then subjected to electrophoresis using A.L.F. DNA Sequencer (manufactured by Pharmacia) to determine the nucleotide sequence, and as a result, the plasmid phKM641H shown in Fig. 7 having the nucleotide sequence of interest was obtained.

### (3) Construction of cDNA encoding VL of anti-GD3 CDR-grafted antibody

A cDNA encoding the anti-GD3 CDR-grafted antibody VL amino acid sequence hKM641L designed in the item 3(1) of Reference Example was constructed as follows using the PCR similar to the VH. In this case, the amino acid sequence of L chain of anti-GD3 mouse antibody KM641 represented by SEQ ID NO:2 was used as the secretory signal sequence.

First, 6 synthetic DNA fragments of SEQ ID NOs:17 to 22 were synthesized using an automatic DNA synthesizer (380A, manufactured by Applied Biosystems). Each of the thus synthesized DNA fragments was added to 50 µl of a buffer comprising 10 mM Tris-HCl (pH 8.3), 50 mM potassium chloride, 1.5 mM magnesium chloride, 0.001% gelatin, 200 µM dNTPs, 0.5 µM M13 primer RV (manufactured by Takara Shuzo), 0.5 µM M13 primer M4 (manufactured by Takara Shuzo) and 2 units of TaKaRa Taq DNA polymerase (manufactured by Takara Shuzo) to give a final concentration of 0.1 µM, and the solution was covered with 50 µl of mineral oil and set to a DNA thermal cycler (PJ480, manufactured by PERKIN ELMER) to carry out the reaction of 30 cycles of heating at 94°C for 2 minutes, at 55°C for 2 minutes and at 72°C for 2 minutes as one cycle. The reaction solution was purified using QIAquick PCR Purification Kit (manufactured by QIAGEN) in accordance with the manufacture's instructions and made into 30 µl of a buffer comprising 50 mM Tris-HCl (pH 7.5), 100 mM sodium chloride, 10 mM magnesium chloride, 1 mM DTT and 100 µg/ml BSA, and 10 units of a restriction enzyme *Eco*RI (manufactured by Takara Shuzo) and 10 units of a restriction enzyme *Spl*I (manufactured by Takara Shuzo) were further added thereto to carry out the reaction at 37°C for 1 hour. The reaction solution was fractionated by agarose gel electrophoresis to recover about 0.2 µg of an *Eco*RI- *Spl*I fragment having about 0.39 kb using QIAquick Gel Extraction Kit (manufactured by QIAGEN) in accordance with the manufacture's instructions.

Next, 3 µg of a plasmid pBSL3 described in Japanese Published Unexamined Patent Application No. 257893/98 was added to 10 µl of a buffer comprising 50 mM Tris-HCl (pH 7.5), 100 mM sodium chloride, 10 mM magnesium chloride, 1 mM DTT and 100 µg/ml BSA, and 10 units of a restriction enzyme *Eco*RI (manufactured by Takara Shuzo) and 10 units of a restriction enzyme *Spl*I (manufactured by Takara Shuzo) were further added thereto to carry out the reaction at 37°C for 1 hour. The reaction solution was fractionated by agarose gel electrophoresis to recover about 2 µg of an *Eco*RI-*Spl*I fragment having about 2.95 kb using QIAquick Gel Extraction Kit (manufactured by QIAGEN) in accordance with the manufacture's instructions.

Next, 0.1 µg of the *Eco*RI-*Spl*I fragment of the PCR product of VL of anti-GD3 CDR-grafted antibody and 0.1 µg of the *Eco*RI-*Spl*I fragment of the plasmid pBSL3, both obtained in the above, were added to 20 µl in total volume of sterile water and ligated using Ready-To-Go T4 DNA Ligase (manufactured by Pharmacia). Using the thus obtained recombinant plasmid DNA solution, *E. coli* HB101 was transformed. Each plasmid DNA was prepared from 10 clones of the transformants, allowed to react in accordance with the manufacture's instructions attached to AutoRead Sequencing Kit (manufactured by Pharmacia) and then subjected to electrophoresis using A.L.F. DNA Sequencer (manufactured by Pharmacia) to determine the nucleotide sequence, and as a result, the plasmid phKM641L shown in Fig. 8 having the nucleotide sequence of interest was obtained.

### 4. Activity evaluation of anti-GD3 CDR-grafted antibody by transient expression using animal cell

In order to evaluate activity of anti-GD3 CDR-grafted antibody more quickly, transient expression of anti-GD3 CDR-grafted antibody was carried out using COS-7 cell (ATCC CRL 1651) as follows.

### (1) Construction of anti-GD3 chimeric antibody KM871 transient expression vector pT641

In order to use as a positive control in the activity evaluation of anti-GD3 CDR-grafted antibody by transient expression, a transient expression vector pT641 for anti-GD3 chimeric antibody KM871 was constructed as follows.

Since the efficiency of transient expression using animal cells generally depends on the copy number of introduced expression vector, it was considered that smaller expression vector has higher expression efficiency. Accordingly, the transient expression vector pT641 for anti-GD3 chimeric antibody KM871 was constructed as follows using the transient expression vector pT796 for anti-ganglioside GM2 chimeric antibody KM966 described in Japanese Published Unexamined Patent Application No. 257893/98 and the plasmid pKM641HF1 having VH of anti-GD3 mouse antibody KM641 and plasmid pKM641LA2 having VL of anti-GD3 mouse antibody KM641 described in Japanese Published Unexamined Patent Application No. 304989/93.

First, 3 µg of the plasmid pKM641LA2 having VL of anti-GD3 mouse antibody KM641 described in Japanese Published Unexamined Patent Application No. 304989/93 was added to 10 µl of a buffer comprising 10 mM Tris-HCl (pH 7.5), 50 mM sodium chloride, 10 mM magnesium chloride and 1 mM DTT, and 10 units of a restriction enzyme *Hin*dIII (manufactured by Takara Shuzo) was further added thereto to carry out the reaction at 37°C for 1 hour. The reaction solution was precipitated with ethanol and added to 10 µl of a buffer comprising 50 mM Tris-HCl (pH 7.5), 100 mM sodium chloride, 10 mM magnesium chloride and 1 mM DTT, and 10 units of a restriction enzyme *Eco*RI (manufactured by Takara Shuzo) was further added to carry out the reaction at 37°C for 1 hour. The reaction solution was fractionated by agarose gel electrophoresis to recover about 0.2 µg of a *Hin*dIII-*Eco*RI fragment having about 0.35 kb using QIAquick Gel Extraction Kit (manufactured by QIAGEN) in accordance with the manufacture's instructions.

Next, 3 µg of the transient expression vector pT796 for anti-ganglioside GM2 chimeric antibody KM966 described in Japanese Published Unexamined Patent Application No. 257893/98 was added to 10 µl of a buffer comprising 50 mM Tris-HCl (pH 7.5), 100 mM sodium chloride, 10 mM magnesium chloride, 1 mM DTT and 100 µg/ml BSA, and 10 units of a restriction enzyme *Eco*RI (manufactured by Takara Shuzo) and 10 units of a restriction enzyme *Spl*I (manufactured by Takara Shuzo) were added thereto to carry out the reaction at 37°C for 1 hour. The reaction solution was fractionated by agarose gel electrophoresis to recover about 2 µg of an *Eco*RI-*Spl*I fragment having about 9.20 kb using QIAquick Gel Extraction Kit (manufactured by QIAGEN) in accordance with the manufacture's instructions.

Next, synthetic DNA fragments respectively having the nucleotide sequences represented by SEQ ID NOs:23 and 24 were synthesized using an automatic DNA synthesizer (380A, manufactured by Applied Biosystems). To 15 µl of sterile water, 0.3 µg of each of the thus obtained synthetic DNA fragments was added, followed by heating at 65°C for 5 minutes. After allowing the reaction solution to stand at room temperature for 30 minutes, 2 µl of a 10 × buffer (500 mM Tris-HCl (pH 7.6), 100 mM magnesium chloride, 50 mM DTT) and 2 µl of 10 mM ATP were added thereto, and 10 units of T4 Polynucleotide Kinase (manufactured by Takara Shuzo) was further added thereto to carry out the reaction at 37°C for 30 minutes to thereby phosphorylate the 5'-terminal.

Next, 0.1 µg of the *Hin*dIII-*Eco*RI fragment derived from the plasmid pKM641LA2, 0.1 µg of the *Eco*RI-*Spl*I fragment derived from the plasmid pT796 and 0.05 µg of the phosphorylated synthetic DNA, obtained in the above, were added to 20 µl in total volume of sterile water and ligated using Ready-To-Go T4 DNA Ligase (manufactured by Pharmacia). Using the thus obtained recombinant plasmid DNA solution, *E. coli* HB101 was transformed to obtain the plasmid pT796H641L shown in Fig. 9. The thus obtained plasmid (10 µg) was allowed to react in accordance with the manufacture's instructions attached to AutoRead Sequencing Kit (manufactured by Pharmacia) and then subjected to an electrophoresis using A.L.F. DNA Sequencer (manufactured by Pharmacia) to determine the nucleotide sequence, and as a result, it was confirmed that a plasmid into which the DNA of interest was cloned was obtained.

Next, 3 µg of a plasmid pBluescript SK(-) (manufactured by Stratagene) was added to 10 µl of a buffer comprising 10 mM Tris-HCl (pH 7.5), 50 mM sodium chloride, 10 mM magnesium chloride, 1 mM DTT and 100 µg/ml BSA, and 10 units of a restriction enzyme *Xba*I (manufactured by Takara Shuzo) was further added thereto to carry out the reaction at 37°C for 1 hour. The reaction solution was precipitated with ethanol, added to 30 µl of a buffer comprising 30 mM sodium acetate (pH 5.0), 100 mM sodium chloride, 1 mM zinc acetate and 5% glycerol, and 30 units of a modification enzyme Mung Bean Nuclease (manufactured by Takara Shuzo) were further added thereto to carry out the reaction at 25°C for 15 minutes. The reaction solution was precipitated with ethanol, added to 10 µl of a buffer comprising 10 mM Tris-HCl (pH 7.5), 10 mM magnesium chloride and 1 mM DTT, and 10 units of a restriction enzyme *Apa*I (manufactured by Takara Shuzo) was further added thereto to carry out the reaction at 37°C for 1 hour. The reaction solution was fractionated by agarose gel electrophoresis to recover about 2 µg of a blunt end-*Apa*I fragment having about 2.95 kb using QIAquick Gel Extraction Kit (manufactured by QIAGEN) in accordance with the manufacture's instructions.

Next, 3 µg of the plasmid pKM641HF1 was added to 10 µl of a buffer comprising 50 mM Tris-HCl (pH 7.5), 100 mM sodium chloride, 10 mM magnesium chloride and 1 mM DTT, and 10 units of a restriction enzyme *Eco*RI (manufactured by Takara Shuzo) was further added thereto to carry out the reaction at 37°C for 1 hour. The reaction solution was precipitated with ethanol, and the 5' protruding end formed by the restriction enzyme digestion was blunt-ended using DNA Blunting Kit (manufactured by Takara Shuzo). The reaction solution was precipitated with ethanol, added to 10 µl of a buffer comprising 10 mM Tris-HCl (pH 7.5), 10 mM magnesium chloride and 1 mM DTT, and 10 units of a restriction enzyme *Apa*I (manufactured by Takara Shuzo) was further added thereto to carry out the reaction at 37°C for 1 hour. The reaction solution was fractionated by agarose gel electrophoresis to recover about 0.2 µg of a blunt end-*Apa*I fragment having about 0.44 kb using QIAquick Gel Extraction Kit (manufactured by QIAGEN) in accordance with the manufacture's instructions.

Next, 0.1 µg of the blunt end-*Apa*I fragment derived from the plasmid pBluescript SK(-) and 0.1 µg of the blunt end-*Apa*I fragment derived from the plasmid pKM641HF1, obtained in the above, were added to 20 µl in total volume of sterile water and ligated using Ready-To-Go T4 DNA Ligase (manufactured by Pharmacia). Using the thus obtained recombinant plasmid DNA solution, *E. coli* HB101 was transformed to obtain the plasmid pBS641H shown in Fig. 10.

Next, 3 µg of the plasmid pT796H641L obtained in the above was added to 10 µl of a buffer comprising 10 mM Tris-HCl (pH 7.5), 10 mM magnesium chloride and 1 mM DTT, and 10 units of a restriction enzyme *Apa*I (manufactured by Takara Shuzo) was further added thereto to carry out the reaction at 37°C for 1 hour. The reaction solution was precipitated with ethanol, added to 10 µl of a buffer comprising 50 mM Tris-HCl (pH 7.5), 100 mM sodium chloride, 10 mM magnesium chloride, 1 mM DTT, 100 µg/ml BSA and 0.01% Triton X-100, and 10 units of a restriction enzyme *Not*I (manufactured by Takara Shuzo) was further added thereto to carry out the reaction at 37°C for 1 hour. The reaction solution was fractionated by agarose gel electrophoresis to recover about 0.2 µg of an *Apa*I-*Not*I fragment having about 9.16 kb using QIAquick Gel Extraction Kit (manufactured by QIAGEN) in accordance with the manufacture's instructions.

Next, 3 µg of the plasmid pBS641H obtained in the above was added to 10 µl of a buffer comprising 10 mM Tris-HCl (pH 7.5), 10 mM magnesium chloride and 1 mM DTT, and 10 units of a restriction enzyme *Apa*I (manufactured by Takara Shuzo) was further added thereto to carry out the reaction at 37°C for 1 hour. The reaction solution was precipitated with ethanol, added to 10 µl of a buffer comprising 50 mM Tris-HCl (pH 7.5), 100 mM sodium chloride, 10 mM magnesium chloride, 1 mM DTT, 100 µg/ml BSA and 0.01% Triton X-100, and 10 units of a restriction enzyme *Not*I (manufactured by Takara Shuzo) was further added thereto to carry out the reaction at 37°C for 1 hour. The reaction solution was fractionated by agarose gel electrophoresis to recover about 2 µg of an *Apa*I-*Not*I fragment having about 0.44 kb using QIAquick Gel Extraction Kit (manufactured by QIAGEN) in accordance with the manufacture's instructions.

Next, 0.1 µg of the *Apa*I-*Not*I fragment derived from the plasmid pT796H641L and 0.1 µg of the *Apa*I-*Not*I fragment derived from the plasmid pBS641H, obtained in the above, were added to 20 µl in total volume of sterile water and ligated using Ready-To-Go T4 DNA Ligase (manufactured by Pharmacia). Using the thus obtained recombinant plasmid DNA solution, *E. coli* HB101 was transformed to obtain the plasmid pT641 shown in Fig. 11.

### (2) Construction of an anti-GD3 CDR-grafted antibody transient expression vector

A transient expression vector for anti-GD3 CDR-grafted antibody was constructed as follows using the transient expression vector pT641 for anti-GD3 chimeric antibody described in the item 4(1) of Reference Example and the plasmids phKM641H and phKM641L described in the items 3(2) and(3) of Reference Example.

First, 3 µg of the plasmid phKM641H obtained in the item 3(2) of Reference Example was added to 10 µl of a buffer comprising 10 mM Tris-HCl (pH 7.5), 10 mM magnesium chloride and 1 mM DTT, and 10 units of a restriction enzyme *Apa*I (manufactured by Takara Shuzo) was further added thereto to carry out the reaction at 37°C for 1 hour. The reaction solution was precipitated with ethanol, added to 10 µl of a buffer comprising 50 mM Tris-HCl (pH 7.5), 100 mM sodium chloride, 10 mM magnesium chloride, 1 mM DTT, 100 µg/ml BSA and 0.01% Triton X-100, and 10 units of a restriction enzyme *Not*I (manufactured by Takara Shuzo) was further added thereto to carry out the reaction at 37°C for 1 hour. The reaction solution was fractionated by agarose gel electrophoresis to recover about 0.2 µg of an *Apa*I-*Not*I fragment having about 0.44 kb using QIAquick Gel Extraction Kit (manufactured by QIAGEN) in accordance with the manufacture's instructions.

Next, 3 µg of the plasmid pT641 obtained in the item 4(1) of Reference Example was added to 10 µl of a buffer comprising 10 mM Tris-HCl (pH 7.5), 10 mM magnesium chloride and 1 mM DTT, and 10 units of a restriction enzyme *Apa*I (manufactured by Takara Shuzo) was further added thereto to carry out the reaction at 37°C for 1 hour. The reaction solution was precipitated with ethanol, added to 10 µl of a buffer comprising 50 mM Tris-HCl (pH 7.5), 100 mM sodium chloride, 10 mM magnesium chloride, 1 mM DTT, 100 µg/ml BSA and 0.01% Triton X-100, and 10 units of a restriction enzyme *Not*I (manufactured by Takara Shuzo) was further added thereto to carry out the reaction at 37°C for 1 hour. The reaction solution was fractionated by agarose gel electrophoresis to recover about 2 µg of an *Apa*I-*Not*I fragment having about 9.16 kb using QIAquick Gel Extraction Kit (manufactured by QIAGEN) in accordance with the manufacture's instructions.

Next, 0.1 µg of the *Apa*I-*Not*I fragment derived from the plasmid phKM641H and 0.1 µg of the *Apa*I-*Not*I fragment derived from the plasmid pT641 were added to 20 µl in total volume of sterile water and ligated using Ready-To-Go T4 DNA Ligase (manufactured by Pharmacia). Using the thus obtained recombinant plasmid DNA solution, *E. coli* HB101 was transformed to obtain the plasmid pT641HCDR shown in Fig. 12.

Next, 3 µg of the plasmid phKM641L obtained in the item 3(3) of Reference Example was added to 10 µl of a buffer comprising 50 mM Tris-HCl (pH 7.5), 100 mM sodium chloride, 10 mM magnesium chloride, 1 mM DTT and 100 µg/ml BSA, and 10 units of a restriction enzyme *Eco*RI (manufactured by Takara Shuzo) and 10 units of a restriction enzyme *Spl*I (manufactured by Takara Shuzo) were further added thereto to carry out the reaction at 37°C for 1 hour. The reaction solution was fractionated by agarose gel electrophoresis to recover about 0.2 µg of an *Eco*RI-*Spl*I fragment having about 0.39 kb using QIAquick Gel Extraction Kit (manufactured by QIAGEN) in accordance with the manufacture's instructions.

Next, 3 µg of each of the plasmid pT641 obtained in the item 4(1) of Reference Example and the plasmid pT641HCDR obtained in the above was added to 10 µl of a buffer comprising 50 mM Tris-HCl (pH 7.5), 100 mM sodium chloride, 10 mM magnesium chloride, 1 mM DTT and 100 µg/ml BSA, and 10 units of a restriction enzyme *Eco*RI (manufactured by Takara Shuzo) and 10 units of a restriction enzyme *Spl*I (manufactured by Takara Shuzo) were further added thereto to carry out the reaction at 37°C for 1 hour. The reaction solution was fractionated by agarose gel electrophoresis to recover about 2 µg of an *Eco*RI-*Spl*I fragment having about 9.20 kb derived from each plasmid using QIAquick Gel Extraction Kit (manufactured by QIAGEN) in accordance with the manufacture's instructions.

Next, 0.1 µg of the *Eco*RI-*Spl*I fragment derived from the plasmid phKM641L and 0.1 µg of the *Eco*RI-*Spl*I fragment derived from the plasmid pT641, obtained in the above, were added to 20 µl in total volume of sterile water and ligated using Ready-To-Go T4 DNA Ligase (manufactured by Pharmacia). Also, 0.1 µg of the *Eco*RI-*Spl*I fragment derived from the plasmid phKM641L and 0.1 µg of the *Eco*RI-*Spl*I fragment derived from the plasmid pT641HCDR, obtained in the above, were added to 20 µl in total volume of sterile water and ligated using Ready-To-Go T4 DNA Ligase (manufactured by Pharmacia). Using each of the thus obtained recombinant plasmid DNA solutions, *E. coli* HB101 was transformed to obtain the plasmids pT641LCDR and pT641HLCDR shown in.Fig. 13.

### (3) Activity evaluation of anti-GD3 chimeric antibody and anti-GD3 CDR-grafted antibody by transient expression using animal cells

Transient expression of antibodies was carried out using the transient expression vector pT641 for anti-GD3 chimeric antibody, the transient expression vector pT641HLCDR for anti-GD3 CDR-grafted antibody and the transient expression vectors pT641HCDR and pT641LCDR for anti-GD3 hybrid antibodies having V regions of mouse antibody and human CDR-grafted antibody, obtained in the items 4(1) and (2) of Reference Example.

COS-7 cells (ATCC CRL 1651) were dispensed in 2 ml at a density of 1×10⁵ cells/ml into a 6 well plate (manufactured by Falcon) and cultured overnight at 37°C. By adding 2 µg of each expression vector to 100 µl of OPTI-MEM medium (manufactured by GIBCO BRL) and further adding a solution in which 10 µl of LIPOFECTAMINE Reagent (manufactured by GIBCO BRL) was added to 100 µl of the OPTI-MEM medium, the reaction was carried out at room temperature for 40 minutes for formation of a DNA-liposome complex. The overnight-cultured COS-7 cells were washed twice with 2 ml of the OPTI-MEM medium (manufactured by GIBCO BRL), a solution prepared by adding 0.8 ml of OPTI-MEM medium to the complex-containing solution was added thereto, followed by culturing at 37°C for 7 hours, the solution was discarded and then the cells were mixed with 2 ml of 10% FBS-containing DME medium (manufactured by GIBCO BRL) and cultured at 37°C. After the introduction of each expression vector, the culture supernatant was recovered 72 hours thereafter and its concentration operation was carried out as occasion demands, and then binding activity of the anti-GD3 humanized antibody for GD3 (manufactured by DIA-IATRON) in the culture supernatant was measured by the ELISA described in the item 2 of Reference Example, by measuring concentration of the anti-GD3 humanized antibody in the culture supernatant by the ELISA described in the item 4 which will be described later and calculating the activity from the measured values as relative activity (%) when the activity of the positive control anti-GD3 chimeric antibody is defined as 100. Results are shown in Fig. 14. As shown in Fig. 14, the anti-GD3 hybrid antibody derived from the transient expression vector pT641HCDR (VH is derived from a human CDR-grafted antibody and VL is derived from a chimeric antibody) showed almost the same binding activity of that of the anti-GD3 hybrid antibody derived from the transient expression vector pT641, but the anti-GD3 hybrid antibody derived from the transient expression vector pT641LCDR (VH is derived from a chimeric antibody and VL is derived from a human CDR-grafted antibody) showed a binding activity of about 50% of the anti-GD3 chimeric antibody. In addition, the anti-GD3 CDR-grafted antibody derived from the transient expression vector pT641HLCDR showed a binding activity of only about 5% of the anti-GD3 chimeric antibody, showing considerable decrease in the activity.

Based on these results, binding activity of the anti-GD3 CDR-grafted antibody having VH and VL designed in the item 3(1) of Reference Example is considerably reduced and the main cause of the activity reduction is considered to be due to VL, but since about 50% of the activity is found by its combination with the VH of anti-GD3 chimeric antibody, it was suggested that there is a problem particularly at the interaction region with VH. Accordingly, an attempt was made to increase the antibody activity by further modifying amino acid residues of the VL designed in the item 3(1) of Reference Example.

### (4) Measurement of the concentration of humanized antibody in transient expression culture supernatant by ELISA

A solution (50 µl) prepared by diluting a goat anti-human IgG (γ-chain) antibody (manufactured by Medical & Biological Laboratories) 400 folds with PBS was dispensed in each well of a 96 well plate for ELISA (manufactured by Greiner) and allowed to react at 4°C overnight. After discarding the antibody solution, 1% BSA-PBS was added in 100 µl/well and allowed to react at room temperature for 1 hour to thereby block the remaining active groups. After discarding 1% BSA-PBS, the transient expression culture supernatants obtained in the item 4(3) of Reference Example or diluted solutions of the purified anti-GD3 chimeric antibody KM871 were added in 50 µl/well and allowed to react at room temperature for 1 hour. After the reaction, each well was washed with Tween-PBS and then a solution prepared by diluting a peroxidase-labeled mouse anti-human κ L chain antibody (manufactured by Zymed) 500 folds with PBS was added in 50 µl/well and allowed to react at room temperature for 1 hour. After the reaction and subsequent washing with Tween-PBS, an ABTS substrate solution (a solution prepared by dissolving 0.55 g of 2,2'-azinobis(3-ethylbenzothiazoline-6-sulfonic acid) in 1 L of 0.1 M citrate buffer (pH 4.2) and adding 1 µl/ml hydrogen peroxide just before the use) was added in 50 µl/well for color development and OD415 was measured.

### 5. Increase of the binding activity by modifying amino acid residues of VL of anti-GD3 CDR-grafted antibody

Increase of the binding activity by modifying amino acid residues of VL of the anti-GD3 CDR-grafted antibody designed in the item 3(1) of Reference Example was carried out as follows.

### (1) Modification of amino acid residues of VL of anti-GD3 CDR-grafted antibody and construction of cDNA encoding the modified VL

First, amino acid residues which are amino acid residues positioned in the interaction region of VH and VL and amino acid residues which are considered to have influence on the three-dimensional structure of each CDR of VL and are also different from the amino acid residues of mouse antibody in the VL of human CDR-grafted antibody were identified from computer models of the three-dimensional structures of various antibodies constructed in the item 3(1) of Reference Example. As a result, in hKM641L as the amino acid sequence of VL of anti-GD3 CDR-grafted antibody represented by SEQ ID NO:10, Ser at position 7, Pro at position 8, Ser at position 12, Gly at position 41, Pro at position 44, Thr at position 72, Ser at position 77, Phe at position 83 and Tyr at position 87 were identified. By replacing these amino acid residues by amino acid residues found in the mouse antibody, 8 VLs of modified anti-GD3 CDR-grafted antibody were designed. That is, in hKM641NL, Pro at position 8, Ser at position 12, Pro at position 44 and Tyr at position 87 in the hKM641L amino sequence were replaced by Ala, Pro, Val and Phe, respectively. In hKM641Lm-1, Ser at position 7, Pro at position 8 and Ser at position 12 in the hKM641L amino sequence were replaced by Thr, Ala and Pro, respectively. In hKM641Lm-4, Tyr at position 87 in the hKM641L amino sequence was replaced by Phe. In the case of hKM641Lm-6, Gly at position 41 and Pro at position 44 in the hKM641L amino sequence were represented by Asp and Val, respectively. In hKM641Lm-7, Thr at position 72, Ser at position 77, Phe at position 83 and Tyr at position 87 in the hKM641L amino sequence were replaced by Ser, Asn, Ile and Phe, respectively. In hKM641Lm-8, Ser at position 77 in the hKM641L amino sequence was replaced by Asn. In hKM641Lm-9, Phe at position 83 and Tyr at position 87 in the hKM641L amino sequence were replaced by Ile and Phe, respectively. In hKM641Lm-69, Gly at position 41, Pro at position 44 and Phe at position 83 in the hKM641L amino sequence were replaced by Asp, Val and Ile, respectively. Among these modified VLs, cDNA encoding each of 6 modified VLs excluding hKM641NL and hKM641Lm-69 was constructed as follows by PCR-aided mutagenesis. That is, antisense chain and sense chain DNA primers were synthesized using an automatic DNA synthesizer (380A, manufactured by Applied Biosystems) for introducing mutation, and a first PCR was carried out in accordance with the method described in the item 3(2) of Reference Example using 1 ng of the plasmid phKM641L as the template and 0.5 µM in final concentration of M13 primer RV (manufactured by Takara Shuzo) and the antisense chain DNA primer and M13 primer M4 (manufactured by Takara Shuzo) and the sense chain DNA primer. Each of the reaction solutions was purified using QIAquick PCR Purification Kit (manufactured by QIAGEN) in accordance with the manufacture's instructions by eluting with 20 µl of 10 mM Tris-HCl (pH 8.0), and then a second PCR was carried out using 5 µl of each eluate in accordance with the method described in the item 3(2) of Reference Example. The reaction solution was purified using QIAquick PCR Purification Kit (manufactured by QIAGEN) in accordance with the manufacture's instructions and made into 30 µl of a buffer comprising 50 mM Tris-HCl (pH 7.5), 100 mM sodium chloride, 10 mM magnesium chloride, 1 mM DTT and 100 µg/ml BSA, and 10 units of a restriction enzyme *Eco*RI (manufactured by Takara Shuzo) and 10 units of a restriction enzyme *Spl*I (manufactured by Takara Shuzo) were further added thereto to carry out the reaction at 37°C for 1 hour. The reaction solution was fractionated by agarose gel electrophoresis to recover about 0.2 µg of an *Eco*RI-*Spl*I fragment having about 0.39 kb using QIAquick Gel Extraction Kit (manufactured by QIAGEN) in accordance with the manufacture's instructions.

Next, 0.1 µg of the *Eco*RI-*Spl*I fragment of the PCR product of VL of the modified anti-GD3 CDR-grafted antibody obtained in the above and 0.1 µg of the *Eco*RI-*Spl*I fragment of the plasmid pBSL3 obtained in the item 3(3) of Reference Example were added to 20 µl in total volume of sterile water and ligated using Ready-To-Go T4 DNA Ligase (manufactured by Pharmacia). Using the thus obtained recombinant plasmid DNA solution, *E. coli* HB101 was transformed. Each plasmid DNA was produced from 10 clones of the transformants, allowed to react in accordance with the manufacture's instructions attached to AutoRead Sequencing Kit (manufactured by Pharmacia) and then subjected to electrophoresis using A.L.F. DNA Sequencer (manufactured by Pharmacia) to determine the nucleotide sequence to thereby obtain a plasmid having cDNA to which the intended modification was applied.

Specifically, a plasmid phKM641Lm-1 having the nucleotide sequence represented by SEQ ID NO:27 was obtained by carrying out a series of the above operations using the synthetic DNA of SEQ ID NO:25 as the antisense chain DNA primer and the synthetic DNA of SEQ ID NO:26 as the sense chain DNA primer. The hKM641Lm-1 as an amino acid sequence encoded by the nucleotide sequence is also represented by SEQ ID NO:27.

A plasmid phKM641Lm-4 having the nucleotide sequence represented by SEQ ID NO:30 was obtained by carrying out a series of the above operations using the synthetic DNA of SEQ ID NO:28 as the antisense chain DNA primer and the synthetic DNA of SEQ ID NO:29 as the sense chain DNA primer. The hKM641Lm-4 as an amino acid sequence encoded by the nucleotide sequence is also represented by SEQ ID NO:30.

A plasmid phKM641Lm-6 having the nucleotide sequence represented by SEQ ID NO:33 was obtained by carrying out a series of the above operations using the synthetic DNA of SEQ ID NO:31 as the antisense chain DNA primer and the synthetic DNA of SEQ ID NO:32 as the sense chain DNA primer. The hKM641Lm-6 as an amino acid sequence encoded by the nucleotide sequence is also represented by SEQ ID NO:33.

A plasmid phKM641Lm-7 having the nucleotide sequence represented by SEQ ID NO:36 was obtained by carrying out a series of the above operations using the synthetic DNA of SEQ ID NO:34 as the antisense chain DNA primer and the synthetic DNA of SEQ ID NO:35 as the sense chain DNA primer. The hKM641Lm-7 as an amino acid sequence encoded by the nucleotide sequence is also represented by SEQ ID NO:36.

A plasmid phKM641Lm-8 having the nucleotide sequence represented by SEQ ID NO:39 was obtained by carrying out a series of the above operations using the synthetic DNA of SEQ ID NO:37 as the antisense chain DNA primer and the synthetic DNA of SEQ ID NO:38 as the sense chain DNA primer. The hKM641Lm-8 as an amino acid sequence encoded by the nucleotide sequence is also represented by SEQ ID NO:39.

A plasmid phKM641Lm-9 having the nucleotide sequence represented by SEQ ID NO:42 was obtained by carrying out a series of the above operations using the synthetic DNA of SEQ ID NO:40 as the antisense chain DNA primer and the synthetic DNA of SEQ ID NO:41 as the sense chain DNA primer. The hKM641Lm-9 as an amino acid sequence encoded by the nucleotide sequence is also represented by SEQ ID NO:42.

Among the modified VLs, cDNA encoding the hKM641NL was constructed by synthesizing 6 synthetic DNA fragments of SEQ ID NOs:17, 22 and 43 to 46 using an automatic DNA synthesizer (380A, manufactured by Applied Biosystems) and carrying out the procedure described in the item 3(3) of Reference Example using these fragments. As a result, a plasmid phKM641NL having the nucleotide sequence represented by SEQ ID NO:47 in which the modification of interest was carried out was obtained. The hKM641NL as an amino acid sequence encoded by the nucleotide sequence is also represented by SEQ ID NO:47.

Among the modified VLs, cDNA encoding the hKM641Lm-69 was constructed as follows using the plasmids phKM641Lm-6 and phKM641Lm-9 having cDNA of modified VL obtained in the above.

First, 3 µg of the plasmid phKM641Lm-6 was added to 10 µl of a buffer comprising 50 mM Tris-HCl (pH 7.5), 100 mM sodium chloride, 10 mM magnesium chloride and 1 mM DTT and 10 units of a restriction enzyme *Eco*RI (manufactured by Takara Shuzo) and 10 units of a restriction enzyme *Pst*I (manufactured by Takara Shuzo) were further added thereto to carry out the reaction at 37°C for 1 hour. The reaction solution was fractionated by agarose gel electrophoresis to recover about 0.3 µg of an *Eco*RI-*Pst*I fragment having about 0.30 kb using QIAquick Gel Extraction Kit (manufactured by QIAGEN) in accordance with the manufacture's instructions.

Next, 3 µg of the plasmid phKM641Lm-9 was added to 10 µl of a buffer comprising 50 mM Tris-HCl (pH 7.5), 100 mM sodium chloride, 10 mM magnesium chloride and 1 mM DTT and 10 units of a restriction enzyme *Eco*RI (manufactured by Takara Shuzo) and 10 units of a restriction enzyme *Pst*I (manufactured by Takara Shuzo) were further added thereto to carry out the reaction at 37°C for 1 hour. The reaction solution was fractionated by agarose gel electrophoresis to recover about 2 µg of an *Eco*RI-*Pst*I fragment having about 3.05 kb using QIAquick Gel Extraction Kit (manufactured by QIAGEN) in accordance with the manufacture's instructions.

Next, 0.1 µg of the *Eco*RI-*Pst*I fragment derived from the plasmid phKM641Lm-6 and 0.1 µg of the *Eco*RI-*Pst*I fragment derived from the plasmid phKM641Lm-9, both obtained in the above, were added to 20 µl in total volume of sterile water and ligated using Ready-To-Go T4 DNA Ligase (manufactured by Pharmacia). Using the thus obtained recombinant plasmid DNA solution, *E. coli* HB101 was transformed to obtain the plasmid phKM641Lm-69 shown in Fig. 15. The reaction was carried out using 10 µg of the thus obtained plasmid in accordance with the manufacture's instructions attached to AutoRead Sequencing Kit (manufactured by Pharmacia) and then the resulting mixture was subjected to electrophoresis using A.L.F. DNA Sequencer (manufactured by Pharmacia) to determine the nucleotide sequence, and as a result, it was confirmed that it has the nucleotide sequence represented by SEQ ID NO:48 in which the modification of interest was carried out. The hKM641Lm-69 as an amino acid sequence encoded by the nucleotide sequence is also represented by SEQ ID NO:48

### (2) Construction of transient expression vector for anti-GD3 CDR-grafted antibodies having modified VL

Transient expression vectors for anti-GD3 CDR-grafted antibodies having various modified VLs were constructed as follows using the plasmids having cDNA molecules encoding various modified VLs, obtained in the item 5(1) of Reference Example, and the transient expression vector pT641HCDR for anti-GD3 hybrid antibodies obtained in the item 4(2) of Reference Example.

First, 3 µg of each of the plasmids phKM641NL, phKM641Lm-1, phKM641Lm-4, phKM641Lm-6, phKM641Lm-7, phKM641Lm-8, phKM641Lm-9 and phKM641Lm-69 obtained in the item 5(1) of Reference Example was added to 10 µl of a buffer comprising 50 mM Tris-HCl (pH 7.5), 100 mM sodium chloride, 10 mM magnesium chloride, 1 mM DTT and 100 µg/ml BSA, and 10 units of a restriction enzyme *Eco*RI (manufactured by Takara Shuzo) and 10 units of a restriction enzyme *Spl*I (manufactured by Takara Shuzo) were further added thereto to carry out the reaction at 37°C for 1 hour. The reaction solution was fractionated by agarose gel electrophoresis to recover about 0.2 µg of an *Eco*RI-*Spl*I fragment having about 0.39 kb using QIAquick Gel Extraction Kit (manufactured by QIAGEN) in accordance with the manufacture's instructions.

Next, 0.1 µg of each of the thus obtained *Eco*RI-*Spl*I fragments of various modified VLs and 0.1 µg of the *Eco*RI-*Spl*I fragment of the transient expression vector pT641HCDR for anti-GD3 hybrid antibody obtained in the item 4(2) of Reference Example, were added to 20 µl in total volume of sterile water and ligated using Ready-To-Go T4 DNA Ligase (manufactured by Pharmacia). Using the thus obtained respective recombinant plasmid DNA solutions, *E. coli* HB101 was transformed to obtain the transient expression vectors for anti-GD3 CDR-grafted antibodies having various modified VLs, pT641HLCDRNL, pT641HLCDRLm-1, pT641HLCDRLm-4, pT641HLCDRLm-6, pT641HLCDRLm-7, pT641HLCDRLm-8, pT641HLCDRLm-9 and pT641HLCDRLm-69, shown in Fig. 16.

### (3) Evaluation of activity of anti-GD3 CDR-grafted antibody having modified VLs by transient expression using animal cell

Transient expression and evaluation of activity of each antibody were carried out in accordance with the method described in the item 4(3) of Reference Example using the transient expression vector pT641 for ant-GD3 chimeric antibody and the transient expression vector pT641HLCDR for anti-GD3 CDR-grafted antibody, both obtained in the item 4(2) of Reference Example, and the transient expression vectors, pT641HLCDRNL, pT641HLCDRLm-1, pT641HLCDRLm-4, pT641HLCDRLm-6, pT641HLCDRLm-7, pT641HLCDRLm-8, pT641HLCDRLm-9 and pT641HLCDRLm-69, for anti-GD3 CDR-grafted antibodies having various modified VLs, obtained in the item 5(2) of Reference Example. Results are shown in Fig. 17. As shown in Fig. 17, the modified anti-GD3 CDR-grafted antibodies derived from the transient expression vectors, pT641HLCDRLm-6, pT641HLCDRLm-7, pT641HLCDRLm-9 and pT641HLCDRLm-69, showed increased binding activity in comparison with the anti-GD3 CDR-grafted antibodies before modification, particularly, the modified anti-GD3 CDR-grafted antibody derived from the transient expression vector pT641HLCDRLm-69 showed its binding activity of about 80% of the anti-GD3 chimeric antibody. On the other hand, the modified anti-GD3 CDR-grafted antibodies derived from other transient expression vectors showed no increase in the binding activity in comparison with the anti-GD3 CDR-grafted antibodies before modification. Based on these results, it was found that, among the modified amino acid residues of VL identified in the item 5(1) of Reference Example, amino acid residues as positions 41, 44, 83 and 87 greatly contribute to the increase of binding activity. It was also found that simultaneous substitution of two amino acid residues at positions 41 and 44 or at positions 83 and 87 contributes to synergistic increase in the activity, and simultaneous modification of four amino acid residues at positions 41, 44, 83 and 87 also contributes to synergistic increase in the activity. Based on the computer models of three-dimensional structures of V regions of various antibodies constructed in the item 3(1) of Reference Example, it was suggested that amino acid residues at position 41 and 44 have influence on the interaction with VH, and amino acid residues at positions 83 and 87 have influence on the three-dimensional structure of CDR3 of VL, and it was considered that three-dimensional structure of an antibody as a whole is suitably maintained by modifying these amino acid residues into amino acid residues found in a mouse antibody and, as a result, its binding activity increases. This information shows that it is necessary to take the interaction regions of VH and VL also into consideration in preparing human CDR-grafted antibodies and that it is necessary to carry out various examinations on the identification of these interaction regions based on the information from three-dimensional structures of antibody V regions. With regard to these interaction regions, it can be easily analogize that they are different in individual antibodies and it is difficult to find a general law at present, so that it is necessary to carry out trial and error in response to each antibody of interest.

### 6. Stable expression of anti-GD3 CDR-grafted antibody using animal cell

Based on the results described in the item 5(3) of Reference Example, it was suggested that the anti-GD3 CDR-grafted antibodies derived from transient expression vectors pT641HLCDRLm-9 and pT641HLCDRLm-69 have about 20% and about 80% of binding activity for GD3, respectively, in comparison with anti-GD3 chimeric antibodies. Accordingly, in order to evaluate the activity of these anti-GD3 CDR-grafted antibodies more in detail, transformed cell lines capable of stably expressing anti-GD3 CDR-grafted antibodies were obtained by the following method, and anti-GD3 CDR-grafted antibodies from culture supernatants of the transformed cell lines were purified. Also, for comparison, stable expression and purification of the anti-GD3 CDR-grafted antibody derived from the transient expression vector pT641HLCDR were carried out in the same manner.

### (1) Construction of stable expression vector for anti-GD3 CDR-grafted antibody

Stable expression vectors were constructed by introducing a resistance gene against an agent G418 and the *dhfr* gene into various transient expression vectors constructed in the item 5(2) of Reference Example.

First, 3 µg of the humanized antibody expression vector pKANTEX93 described in WO 97/10354 was added to 10 µl of a buffer comprising 20 mM Tris-HCl (pH 8.5), 10 mM magnesium chloride, 1 mM DTT and 100 mM potassium chloride, and 10 units of a restriction enzyme *Bam*HI (manufactured by Takara Shuzo) and 10 units of a restriction enzyme *Xho*I (manufactured by Takara Shuzo) were further added thereto to carry out the reaction at 37°C for 1 hour. The reaction solution was fractionated by agarose gel electrophoresis to recover about 1 µg of a *Bam*HI-*Xho*I fragment having about 8.68 kb using QIAquick Gel Extraction Kit (manufactured by QIAGEN) in accordance with the manufacture's instructions.

Next, 3 µg of each of the transient expression vectors, pT641HLCDRLm-9, pT641HLCDRLm-69 and pT641HLCDR, was added to 10 µl of a buffer comprising 20 mM Tris-HCl (pH 8.5), 10 mM magnesium chloride, 1 mM DTT and 100 mM potassium chloride, and 10 units of a restriction enzyme *Bam*HI (manufactured by Takara Shuzo), 10 units of a restriction enzyme *Xho*I (manufactured by Takara Shuzo) and 10 units of a restriction enzyme *Stu*I (manufactured by Takara Shuzo) were further added thereto to carry out the reaction at 37°C for 1 hour. The reaction solution was fractionated by agarose gel electrophoresis to recover about 1 µg of a *Bam*HI-*Xho*I fragment having about 4.90 kb using QIAquick Gel Extraction Kit (manufactured by QIAGEN) in accordance with the manufacture's instructions.

Next, 0.1 µg of the *Bam*HI-*Xho*I fragment derived from the plasmid pKANTEX93 and 0.1 µg of the *Bam*HI-*Xho*I fragment derived from each of the transient expression vectors, obtained in the above, were added to 20 µl in total volume of sterile water and ligated using Ready-To-Go T4 DNA Ligase (manufactured by Pharmacia). Using each of the thus obtained recombinant plasmid DNA solutions, *E. coli* HB101 was transformed to obtain the anti-GD3 CDR-grafted antibody stable expression vectors, pKANTEX641HLCDRLm-9, pKANTEX641HLCDRLm-69 and pKANTEX641HLCDR, shown in Fig. 18.

### (2) Stable expression of anti-GD3 CDR-grafted antibody using animal cell

Using the various stable expression vectors for anti-GD3 CDR-grafted antibody obtained in the item 6(1) of Reference Example, expression of anti-GD3 CDR-grafted antibody in animal cells was carried out as follows.

Each of the anti-GD3 CDR-grafted antibody expression vectors (4 µg) was introduced into 4×10⁶ cells of a rat myeloma cell line YB2/0 cell (ATCC CRL 1662) by the electroporation method [*Cytotechnology*, 3, 133 (1990)], and the cells were suspended in 40 ml of RPMI640-FBS(10) [RPMI1640 medium containing 10% of fetal bovine serum (FBS)] and dispensed in 200 µl/well into a 96 well microtiter plate (manufactured by Sumitomo Bakelite). After culturing at 37°C for 24 hours in a 5% CO₂ incubator, G418 was added to give a concentration of 0.5 mg/ml, followed by culturing for 1 to 2 weeks. Culture supernatants were recovered from wells in which colonies of transformants showing G418-resistance were formed and became confluent, and the antigen binding activity of various anti-GD3 CDR-grafted antibodies in the supernatants was measured by the ELISA shown in the item 2 of Reference Example (a peroxidase-labeled goat anti-human IgG(γ) antibody was used as the secondary antibody).

With regard to the transformants of wells in which expression of anti-GD3 CDR-grafted antibodies was found in the culture supernatants, in order to increase antibody expression quantity by using a *dhfr* gene amplification system, they were suspended in the RPMI1640-FBS(10) medium containing 0.5 mg/ml G418 and 50 nM methotrexate (hereinafter referred to as "MTX": manufactured by SIGMA) as an inhibitor of the *dhfr* gene product dihydrofolate reductase (hereinafter referred to as "DHFR") to give a density of 1 to 2×105 cells/ml, and dispensed in 2 ml into a 24 well plate (manufactured by Greiner). By culturing at 37°C for 1 to 2 weeks in a 5% CO₂ incubator, transformants showing 50 nM MTX resistance were induced. When the transformants became confluent, the antigen binding activity of various anti-GD3 CDR-grafted antibodies in the supernatants was measured by the ELISA shown in the item 2 of Reference Example. With regard to the transformants of wells in which expression of anti-GD3 CDR-grafted antibodies was found in the culture supernatants, the MTX concentration was increased to 100 nM and then to 200 nM by the method similar to the above, and transformants capable of growing in the RPMI1640-FBS(10) medium containing 0.5 mg/ml G418 and 200 nM MTX and of highly expressing anti-GD3 CDR-grafted antibodies were finally obtained. With regard to the thus obtained transformants, single cell isolation (cloning) was carried out twice by limiting dilution analysis. The transformed cell clones obtained by introducing the stable expression vectors, pKANTEX641HLCDRLm-9, pKANTEX641HLCDRLm-69 and pKANTEX641HLCDR, were named KM8870, KM8871 and KM8869, respectively, and expression levels of anti-GD3 CDR-grafted antibody by transformed cell clones were about 5 µg/10⁶ cells/24 hr, about 10 µg/10⁶ cells/24 hr and about 30 µg/10⁶ cells/24 hr, respectively.

### (3) Purification of anti-GD3 CDR-grafted antibody from culture supernatant

Each of the transformed cell clones, KM8870, KM8871 and KM8869, obtained in the item 6(2) of Reference Example which express various anti-GD3 CDR-grafted antibodies was suspended in GIT medium (manufactured by Nippon Pharmaceutical) containing 200 nM MTX to give a density of 1 to 2×10⁵ cells/ml, and dispensed in 200 ml into 175 cm² flasks (manufactured by Greiner). The cells were cultured at 37°C for 5 to 7 days until they became confluent and then the culture supernatant was recovered. Each anti-GD3 CDR-grafted antibody was purified from the culture supernatant using Prosep-A (manufactured by Bioprocessing) column in accordance with the manufacture's instructions. About 3 mg of anti-GD3 CDR-grafted antibody KM8870 was obtained from 500 ml of the culture supernatant of KM8870, and about 25 mg of anti-GD3 CDR-grafted antibody KM8871 from 1,600 ml of the culture supernatant of KM8871 and about 65 mg of anti-GD3 CDR-grafted antibody KM8869 from 1,000 ml of the culture supernatant of KM8869. About 4 µg of each of the thus obtained anti-GD3 CDR-grafted antibodies was subjected to an electrophoresis in accordance with the known method [*Nature*, 227, 680 (1970)] to examine its molecular weight and purification degree. Results are shown in Fig. 19. As shown in Fig. 19, molecular weight of each purified anti-GD3 CDR-grafted antibody was about 150 kilodaltons (hereinafter referred to as "Kd") under non-reducing conditions, and two bands of about 50 Kd and about 25 Kd were found under reducing conditions. The molecular weights almost coincided with the molecular weights deduced from the cDNA nucleotide sequences of H chain and L chain of the antibody (H chain: about 49 Kd, L chain: about 24 Kd, whole molecule: about 146 Kd), and also coincided with the reports that the IgG type antibody has a molecular weight of about 150 Kd under non-reducing conditions and is degraded into H chains having a molecular weight of about 50 Kd and L chains having a molecular weight of about 25 Kd under reducing conditions due to cutting of the disulfide bond (hereinafter referred to as "S-S bond") in the molecule (*Antibodies: A Laboratory Manual, Monoclonal Antibodies: Principles and Practice*), so that it was confirmed that each anti-GD3 CDR-grafted antibody was expressed as an antibody molecule of a correct structure. Also, when N-terminal amino acid sequences of the H chain and L chain of each of the purified anti-GD3 CDR-grafted antibodies were analyzed by Edman degradation using a protein sequencer (470A, manufactured by Applied Biosystems), it was confirmed that they coincide with the H chain and L chain N-terminal amino acid sequences deduced from respective cDNA nucleotide sequences.

### 7. Evaluation of activity of anti-GD3 CDR-grafted antibody

### (1) Reactivity of anti-GD3 CDR-grafted antibody with GD3 (ELISA)

Reactivity of purified anti-GD3 CDR-grafted antibodies KM8869, KM8870 and KM8871 with GD3 (manufactured by DIA-IATRON) was measured by the ELISA shown in the item 2 of Reference Example. Fig. 20 shows a result of the examination of the reactivity carried out by fixing the amount of GD3 to be adsorbed to each well of a plate for ELISA to 20 pmol/well and changing the concentration of anti-GD3 chimeric antibody KM871 and anti-GD3 CDR-grafted antibodies KM8869, KM8870 and KM8871 to be added. As shown in Fig. 20, among the anti-GD3 CDR-grafted antibodies, KM8871 showed the highest binding activity which was about 1/2 of the anti-GD3 chimeric antibody KM871. Fig. 21 shows a result of the examination of the reactivity of a constant concentration (10 µg/ml) of anti-GD3 chimeric antibody KM871 and anti-GD3 CDR-grafted antibodies KM8869, KM8870 and KM8871, carried out by changing the amount of GD3 to be adsorbed to each well of a plate for ELISA. As shown in Fig. 21, among the anti-GD3 CDR-grafted antibodies, KM8871 showed the highest binding activity which was equivalent to the anti-GD3 chimeric antibody KM871. Fig. 22 shows a result of the examination of the reactivity of a constant concentration (10 µg/ml) of anti-GD3 chimeric antibody KM871 and anti-GD3 CDR-grafted antibodies KM8869, KM8870 and KM8871, carried out by changing the kind of ganglioside to be adsorbed to each well of a plate for ELISA (adsorption amount: 20 pmol/well). A total of 11 kinds of ganglioside, GM1, N-acetylGM2 (manufactured by Boehringer Mannheim, hereinafter referred to as "AcGM2"), N-glycolylGM2 (hereinafter referred to as "GcGM2"), N-acetylGM3 (hereinafter referred to as "AcGM3"), N-glycolylGM3 (hereinafter referred to as "GcGM3"), GD1a and GD1b (manufactured by DIA-IATRON), GD2 and GD3 (manufactured by DIA-IATRON), GQ1b (manufactured by DIA-IATRON) and GT1b (manufactured by Funakoshi), were used. In this case, GM1 and GD1a were purified from bovine brain, and N-glycolylGM2 and N-glycolylGM3 from mouse liver, N-acetylGM3 from canine erythrocyte and GD2 from a human neuroblastoma culture cell line IMR32 (ATCC CCL 127), respectively in accordance with the known method [*J. Biol. Chem*., 263, 10915 (1988)]. As shown in Fig. 22, each of the anti-GD3 CDR-grafted antibodies KM8869, KM8870 and KM8871 bound strongly to GD3 and weakly to GQ1b similar to the case of the anti-GD3 chimeric antibody KM871 but did not bind to other gangliosides. Thus, it was shown that they maintain the binding specificity of chimeric antibody KM871.

### (2) Reactivity of anti-GD3 CDR-grafted antibody with human cancer cell (immunofluorescent method)

Reactivity of purified anti-GD3 CDR-grafted antibodies KM8869, KM8870 and KM8871 with human cancer cells was measured as follows. That is, 1×10⁶ cells of each of human melanoma culture cell lines G-361 (ATCC CRL 1424) and SK-MEL-28 (ATCC HTB72) were suspended in PBS, put into microtubes and centrifuged (2,000 rpm for 2 minutes), and the thus washed cells were stirred after adding 50 µl of the anti-GD3 chimeric antibody KM871 or the anti-GD3 CDR-grafted antibody, KM8869, KM8870 or KM8871 (a solution adjusted to 5 µg/ml with 1% BSA-PBS) and then allowed to react at 4°C for 1 hour. After the reaction, followed by centrifugation three times with PBS for washing, 20 µl of rabbit anti-human IgG (H + L) F(ab')₂ solution (manufactured by Wako Pure chemical Industries, used by diluting 30 folds with 1% BSA-PBS) fluorescence-labeled with fluorescein isothiocyanate (hereinafter referred to as "FITC") was added thereto, followed by stirring, and then the reaction was carried out at 4°C for 1 hour. After the reaction, followed by centrifugation three times with PBS for washing, the cells were again suspended in PBS to carry out the analysis using a flow cytometer EPICS Elite (manufactured by COULTER). As a control, the analysis was carried out by the same procedure without adding antibodies. Results are shown in Fig. 23. As shown in Fig. 23, all of the anti-GD3 chimeric antibody KM871 and the anti-GD3 CDR-grafted antibodies KM8869, KM8870 and KM8871 showed the reactivity with both cell lines. Among the anti-GD3 CDR-grafted antibodies, KM8871 showed the highest reactivity. The above results show that the anti-GD3 CDR-grafted antibodies are useful in the diagnosis, treatment and the like of GD3-positive human tumors including melanoma.

### (3) In vitro cytotoxic activity of anti-GD3 CDR-grafted antibodies (CDC activity)

In order to evaluate *in vitro* cytotoxic activity of the purified anti-GD3 CDR-grafted antibodies KM8869, KM8870 and KM8871, the CDC activity was measured by the following method.

### a. Preparation of target cell suspension

Each of human melanoma culture cell lines, G-361 (ATCC CRL 1424) and SK-MEL-28 (ATCC HTB72), was cultured in RPMI1640-FBS(10) medium and adjusted to 5×10⁶ cells, and the cells were isotope-labeled by adding 3.7 MBq equivalent of a radioactive substance Na₂⁵¹CrO₄ and carrying out the reaction at 37°C for 1 hour. After the reaction, the cells were washed three times by their suspension in RPMI1640-FBS(10) medium and centrifugation, re-suspended in the medium and then incubated in ice at 4°C for 30 minutes to thereby spontaneously release the radioactive substance. After centrifugation, the cells were adjusted to 1×10⁶ cells/ml by adding 5 ml of RPMI1640-FBS(10) medium and used as the target cell suspension.

### b. Preparation of complement solution

Sera of three healthy persons were mixed and used as the human complement source. At the time of its use, it was diluted to 15% vol/vol with RPMI1640-FBS(10) medium and used as the complement solution.

### c. Measurement of CDC activity

To each well of a 96 well U bottom plate (manufactured by Falcon), 50 µl of the target cell suspension prepared in "a." was added (5×10⁴ cells/well), and then the anti-GD3 chimeric antibody KM871 or the anti-GD3 CDR-grafted antibody, KM8869, KM8870 or KM8871, was added to give a final concentration of 0.05 to 50 µg/ml and allowed to react at room temperature for 30 minutes. After the reaction, the plate was centrifuged to remove the supernatant, and 150 µl of the human complement solution prepared in "b." was added thereto and allowed to react at 37°C for 1 hour. After centrifugation, the amount of ⁵¹Cr released in the supernatant was measured by a γ-counter. The amount of the spontaneously dissociated ⁵¹Cr was calculated by carrying out the same procedure using the medium alone instead of the antibody solution and complement solution and measuring the amount of ⁵¹Cr in the supernatant. Amount of the total dissociated ⁵¹Cr was calculated by carrying out the same procedure by adding the medium alone instead of the antibody solution, and 5 N sodium hydroxide instead of the complement solution, and measuring the amount of ⁵¹Cr in the supernatant. The CDC activity was calculated by the following equation.$\text{CDC activity (%) =} \frac{{\text{}}^{\text{51}} {\text{Cr in sample supernatant - spontaneously released}}^{\text{51}} \text{Cr}}{{\text{total released}}^{\text{51}} {\text{Cr - spontaneously released}}^{\text{51}} \text{Cr}} \text{X 100}$

Results are shown in Fig. 24. As shown in Fig. 24, it was found that all of the anti-GD3 chimeric antibody KM871 and the anti-GD3 CDR-grafted antibodies KM8869, KM8870 and KM8871 show the CDC activity for both cell lines, particularly show remarkably strong cytotoxic activity for the human melanoma culture cell line G-361. Among the anti-GD3 CDR-grafted antibodies, KM8871 showed the highest cytotoxic activity which was about 1/3 of the anti-GD3 chimeric antibody KM871.

### (4) In vitro cytotoxic activity of anti-GD3 CDR-grafted antibody (ADCC activity)

In order to evaluate *in vitro* cytotoxic activity of the purified anti-GD3 CDR-grafted antibodies KM8869, KM8870 and KM8871, the ADCC activity was measured by the following method.

### a. Preparation of target cell suspension

Each of human melanoma culture cell lines, G-361 (ATCC CRL 1424) and SK-MEL-28 (ATCC HTB72), was cultured in RPMI1640-FBS(10) medium and adjusted to 1×10⁶ cells, and the cells were isotope-labeled by adding 1.85 MBq equivalent of a radioactive substance Na₂⁵¹CrO₄ and carrying out the reaction at 37°C for 1 hour. After the reaction, the cells were washed three times by their suspension in RPMI1640-FBS(10) medium and centrifugation, re-suspended in the medium and then incubated in ice at 4°C for 30 minutes to thereby spontaneously release the radioactive substance. After centrifugation, the cells were adjusted to 2×10⁵ cells/ml by adding 5 ml of RPMI1640-FBS(10) medium and used as the target cell suspension.

### b. Preparation of effector cell suspension

Healthy human vein blood (50 ml) was collected, and 0.5 ml of heparin sodium (manufactured by Takeda Pharmaceutical) was gently added thereto. The resulting mixture was centrifuged (1,500 to 1,800 × g for 30 minutes) using Polymorphprep (manufactured by Nycomed Pharma AS) in accordance with the manufacture's instructions to separate a mononuclear cell layer. The separated cells were centrifuged (1,500 to 1,800 × g for 5 minutes) three times with RPMI1640-FBS(10) medium for washing and then re-suspended in the medium to give a density of 5×10⁶ cells/ml to be used as the effector cell suspension.

### c. Measurement of ADCC activity

Into each well of a 96 well U bottom plate (manufactured by Falcon), 50 µl of the target cell suspension prepared in "a." was dispensed (1×10⁴ cells/well). Next, 100 µl of the effector cell suspension prepared in "b." was added (5×10⁵ cells/well, ratio of the effector cells to the target cells becomes 50:1). Thereafter, each of the anti-GD3 chimeric antibody KM871 and the anti-GD3 CDR-grafted antibodies KM8869, KM8870 and KM8871 was added to give a final concentration of 0.05 to 50 µg/ml and allowed to react at 37°C for 4 hours. After the reaction, the plate was centrifuged and the amount of ⁵¹Cr in the supernatant was measured by a γ-counter. The amount of the spontaneously dissociated ⁵¹Cr was calculated by carrying out the same procedure using the medium alone instead of the effector cell suspension and antibody solution and measuring the amount of ⁵¹Cr in the supernatant. The amount of the total dissociated ⁵¹Cr was calculated by carrying out the same procedure by adding the medium alone instead of the antibody solution, and 5 N sodium hydroxide instead of the effector cell suspension, and measuring the amount of ⁵¹Cr in the supernatant. The ADCC activity was calculated by the following equation.$\text{ADCCC activity (%) =} \frac{{\text{}}^{\text{51}} {\text{Cr in sample supernatant - spontaneously released}}^{\text{51}} \text{Cr}}{{\text{total released}}^{\text{51}} {\text{Cr - spontaneously released}}^{\text{51}} \text{Cr}} \text{X 100}$

Results are shown in Fig. 25. As shown in Fig. 25, it was found that all of the anti-GD3 chimeric antibody KM871 and the anti-GD3 CDR-grafted antibodies KM8869, KM8870 and KM8871, show strong ADCC activity for both cell lines. Among the anti-GD3 chimeric antibody KM871 and the anti-GD3 CDR-grafted antibodies KM8869, KM8870 and KM8871, difference in the cytotoxic activity was not found.

The above results show that the anti-GD3 CDR-grafted antibodies are useful in the diagnosis, treatment and the like of GD3-positive human tumors similar to the case of the anti-GD3 chimeric antibody KM871. In addition, it is expected that immunogenicity of the anti-GD3 CDR-grafted antibodies is reduced in human and the effects are further prolonged.

The transformed cell clone KM8871 capable of producing KM8871 which showed the highest activity among the anti-GD3 CDR-grafted antibodies has been deposited on July 22, 1999, as FERM BP-6790 in International Patent Organism Depositary, National Institute of Advanced Industrial Science and Technology (AIST Tsukuba Central 6, 1-1, Higashi 1-Chome Tsukuba-shi, Ibaraki-ken, Japan).

### INDUSTRIAL APPLICABILITY

The present invention relates to a medicament which comprises a gene recombinant antibody against ganglioside GD3 or the antibody fragment thereof in combination with at least one of a substance which activates an immunocompetent cell and a substance having an antitumor activity. The medicament of the present invention show higher therapeutic effects at a lower dose than the case where the antibody, the substance which activates an immunocompetent cell or the substance having an antitumor activity is administered alone.

## Claims

1. A medicament which comprises a gene recombinant antibody against ganglioside GD3 or the antibody fragment thereof in combination with at least one of a substance which activates an immunocompetent cell and a substance having an antitumor activity.

2. An antitumor agent which comprises a gene recombinant antibody against ganglioside GD3 or the antibody fragment thereof in combination with at least one of a substance which activates an immunocompetent cell and a substance having an antitumor activity.

3. The antitumor agent according to claim 2, wherein the tumor is melanoma.

4. The agent according to any one of claims 1 to 3, wherein the gene recombinant antibody against ganglioside GD3 is a humanized antibody against ganglioside GD3.

5. The agent according to claim 4, wherein the humanized antibody is a human chimeric antibody or a human complementarity determining region (CDR)-grafted antibody.

6. The agent according to claim 5, wherein the humanized antibody is a humanized antibody which comprises CDR1, CDR2 and CDR3 of a heavy chain (H chain) variable region (V region) comprising the amino acid sequences represented by SEQ ID NOs:3, 4 and 5, respectively, and CDR1, CDR2 and CDR3 of a light chain (L chain) variable region (V region) comprising the amino acid sequences represented by SEQ ID NOs:6, 7 and 8, respectively.

7. The agent according to claim 5, wherein the human chimeric antibody is a human chimeric antibody which comprises an H chain V region and an L chain V region comprising the amino acid sequences represented by SEQ ID NOs:49 and 50, respectively.

8. The agent according to claim 5, wherein the human CDR-grafted antibody is a human CDR-grafted antibody which comprises an H chain V region and an L chain V region of the human CDR-grafted antibody comprising the amino acid sequences represented by SEQ ID NOs:9 and 10, respectively.

9. The agent according to any one of claims 1 to 8, wherein the substance which activates an immunocompetent cell is cytokine.

10. The agent according to claim 9, wherein the cytokine is selected from the group consisting of IFNα, IFNβ, IFNγ, TNFα, TNFβ, IL-1α, IL-1β, IL-2, IL-3, IL-4, IL-6, IL-7, IL-9, IL-12, IL-15, IL-17, IL-18, GM-CSF, G-CSF, M-CSF and SCF.

11. The agent according to any one of claims 1 to 8, wherein the substance having an antitumor activity is a hormonotherapeutant agent or a chemotherapy agent.

12. The agent according to claim 11, wherein the hormonotherapeutant agent or the chemotherapy agent is an agent selected from the group consisting of dacarbazine, cisplatin, fotemustine, carmustine, nimustine, lomustine, semustine, pacritaxel, dosetaxel, vindesine, vinblastine, vincristine, carboplatin, bleomycin, doxorubicin and tamoxifen.
